# EUROPEAN PATENT APPLICATION

(11) **EP 4 814 036 A1**
(43) Date of publication of application: **30.09.2026**
(21) Application number: 25167211.9
(22) Date of filing: 28.03.2025
(51) Int. Cl.: C08G 65/331, C08G 65/333

(54) **COMPOUND FOR FORMING LIPID NANOPARTICLES**

(71) Applicant: Freie Universität Berlin, 14195 Berlin (DE); Bayer Aktiengesellschaft, 51373 Leverkusen (DE)
(72) Inventor: HAAG, Rainer, 12209 Berlin (DE); RASHMI, Rashmi, 55122 Mainz (DE); NÖLTE, Gideon, 10961 Berlin (DE); ENSMINGER, Yara, 12167 Berlin (DE); KÖBBERLING, Johannes, 41466 Neuss (DE); DIAZ-OVIEDO, Christian David, 42275 Wuppertal (DE); KARIMOV, Michael, 10179 Berlin (DE); HAFKE, Markus, 12309 Berlin (DE)
(74) Representative: Maikowski & Ninnemann Patentanwälte Partnerschaft mbB

(57) **Abstract**

The present invention relates to a compound according to general formula (L¹)(L²)(L³)ₘ(L⁴)ₙ-X-PG, wherein L¹, L², L³, L⁴ denote independently from each other a lipid moiety; n is 0 or 1; m is 0 or 1; X is a linker moiety; and PG is a polyglycerol moiety.

## Description

The present invention relates to a compound according to claim 1 and uses of such compound according to claims 14 and 15.

Polyethylene glycol (PEG) has long been a staple in the field of drug delivery due to its ability to improve the pharmacokinetics and biocompatibility of therapeutic agents. However, concerns have arisen regarding its immunogenicity, potential toxicity, and non-biodegradability, prompting the exploration of alternative polymers with similar or superior properties. Among these alternatives, polyglycerol has emerged as a promising candidate for replacing PEG in lipid nanoparticles for advanced drug delivery systems.

The role of polyethylene glycol (PEG) lipids in the recent coronavirus pandemic has been pivotal, particularly in the development of mRNA-based COVID-19 vaccines. PEGylated lipids serve as key components of lipid nanoparticles (LNPs) used, e.g., to encapsulate and deliver mRNA, in particular mRNA vaccines, such as the Pfizer-BioNTech and Moderna COVID-19 vaccines. These LNPs protect the delicate mRNA molecules, enhance their stability, and facilitate their delivery to target cells, ultimately eliciting robust immune responses against SARS-CoV-2. However, despite their crucial role in the development of nucleic acid-based therapies (such as mRNA-based therapies like mRNA-based vaccination), PEG lipids have been associated with certain disadvantages. Concerns have been raised regarding potential allergic reactions to PEG, including anaphylaxis, as well as the presence of pre-existing immunity to PEG in some individuals. Addressing these challenges and exploring alternative platforms for delivering nucleic acids will help to improve the delivery of nucleic acids to an intended site of action. This will help, e.g., in optimizing vaccine safety, efficacy, and accessibility in the ongoing battle against viral diseases and pandemics.

### LNPs as delivery systems for mRNA

Use of mRNA in vaccines has gained much attention during the COVID-19 pandemic. Their ability to encode almost any protein while their chemical properties stay almost the same, make it possible to use nearly the same delivery technology for any mRNA. mRNA is a polyanionic oligonucleotide with high molecular weight (700-7000 kDa).^{[1]} In physiological media nucleases lead to fast degradation of naked mRNA and the phosphate backbone is prone to hydrolysis, due to intramolecular attack of the 2'-hydroxyl group.^{[1],[2]}

Due to the high molecular weight as well as the high negative charge density of the polyanionic polymer, which leads to repulsing forces with the negatively charged phosphate lipids inside of cell membranes, naked mRNA cannot pass through the lipid bilayer of the cells. ^{[4]} To overcome these transfection problems, mRNA is encapsulated inside LNPs. ^{[3]} LNPs known from prior art consist of four major components: an ionizable cationic lipid, phospholipid, cholesterol and a poly(ethylene glycol)-lipid (PEG-lipid). Details on PEG-lipids have been published, e.g., by Chaudhary et al.^{[4]}

The amine groups of the ionizable lipids are positively charged at a pH value lower than the pKₐ value of the ionizable lipid (which typically ranges from pH 6.2 to 6.5) and neutral at physiological pH.^{[5],[1]} This property decreases the toxicity of ionizable lipids compared to nonionizable cationic lipids by decreasing nonspecific lipid-protein interaction,^{[6],[7],[8]} leads to a strong interaction with the negatively charged phosphate backbone of the mRNA during LNP formation, which is done at a pH of ~4,^{[9]} and facilitates fusion with the cell membrane during cell uptake via endocytosis.^{[10]} Due to the increasing acidic milieu inside the endosomes, the ionizable lipids get protonated, leading to the fusion with oppositely charged membrane lipids. Thereby the structure of the lipid bilayer is changed into an inverted-like micellar structure,^{[5]} resulting in the disruption of the membrane and release of mRNA into the cytosol. ^{[11],[12],[13]} It has also been shown that they can play some role in specific targeting of organs. ^{[14]} Helper lipids such as phospholipids and sterol lipids (cholesterol variants) improve the stability of the particles and also help in membrane fusion.^{[15]} Two examples for frequently used phospholipids are distearoylphosphatidylcholine (DSPC) and 1,2-Dioleoyl-*sn*-glycero-3-phosphoethanolamine (DOPE).

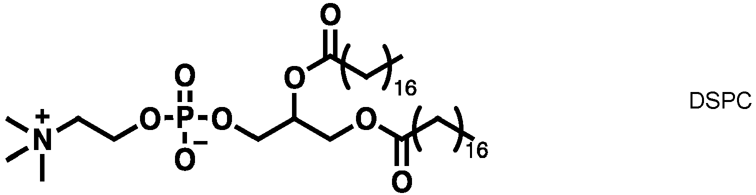

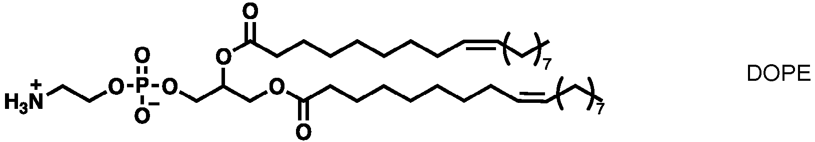

A combination of phospholipids and cholesterol helps in stabilizing not only the particle layer but also improves the encapsulation of RNA.^{[16]} PEG-lipids are an essential part in the formation of LNPs, even though they only make up for around 1-3 mol% in a typical LNP formulation. In the Covid-19 vaccine from Moderna DMG-PEG2000 is used, which consists of a poly(ethylene glycol) chain with a molecular weight of 2000 g/mol, attached to dimyristoyl glycerol via an ether linkage. 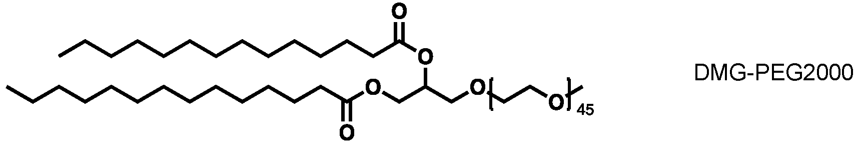 PEG is a hydrophilic polymer, which in combination with the hydrophobic lipid tail gives PEG-lipids an amphiphilic character. Good solubility in several solvents, including water, methanol, ethanol, and dichloromethane makes it useful in many products. ^{[17],[18]} It is generally regarded as an inert polymer, due to its lack of functional groups, and is essential in preventing aggregation of LNPs during particle formation and storage by steric repulsion of PEG-chains on the surface of the particle.^{[19]} This steric shielding also enables longer circulation half-life and therefore also improved distribution of the drug, due to reduced binding of plasma protein and uptake by phagocytic cells.^{[20]}

Particle size of LNPs can also be tuned by changing the amount of PEG-lipid, which is an important factor in generating particles with good pharmacokinetic properties, biodistribution, transfection potency and delivery efficiency.^{[5],[21],[22]} increasing the amount of PEGylated lipids results in a smaller particle size.^{[16]} For the efficient delivery of mRNA a size between 60 nm up to 150 nm is targeted.^{[23]} In order to have a high cell uptake and transfection efficacy the LNPs must be able to fuse with cell membranes, making a shielded particle counterproductive, also called the PEG-dilemma.^{[24]} Therefore, cleavable groups such as esters or carbamates are introduced into the structure of PEG-lipids, leading to faster PEG-shedding under physiological conditions, improving the transfection efficacy.^{[25]}

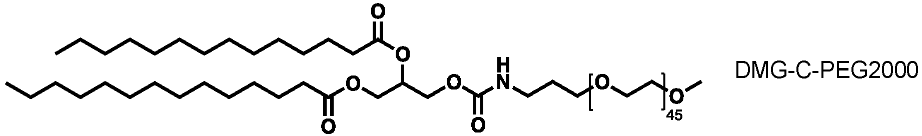

Another factor determining the desorption rate of PEG-lipids is the chain length of the lipid tail. Using shorter acyl chains (C8-C14) In comparison to longer chains between C16 and C24 leads to a higher diffusibility of PEGylated lipids. ^{[26],[22]} Although PEG is considered as a safe and non-immunogenic compound, in the last years it has been shown that the use of PEG in the biomedical applications can lead to decreased efficiency and unwanted immunogenic reactions.^{[17]}

### Immunogenicity of PEG

It has been known for a few years that repeated administration of PEGylated proteins, nanoparticles or nucleic acids, can induce anti-PEG antibodies and lead to a 2-10-fold shortened circulation half-life by accelerated blood clearance (ABC phenomenon).^{[27],[28],[29]} Especially IgM and IgG are responsible for the fast opsonization and as a consequence reduced efficacy.^{[28],[30]} Apart from reduced efficacy due to premature drug release from carriers containing PEG,^{[31]} hypersensitivity reactions induced by anti-PEG antibodies have been reported.^{[32]} Nanoparticles encapsulating poly nucleotides compared to empty NPs lead to a higher immune response and formation of more anti-PEG antibodies.^{[33]} By employing fast sheddable PEG-lipids anti-PEG antibody formation could be reduced. ^{[33]} The amount of healthy individuals in the population who possess pre-existing anti-PEG antibodies lies around 40%,^{[28]} although the reported numbers differ a lot. This number increased drastically compared to the 0.2% reported in 1984 by Richter and Akerblom^{[56]}, likely due to increased exposure to PEG in food packaging, cosmetics and pharmaceutics.^{[34],[35]} These pre-existing antibodies are assumed to be the reason for increased unwanted immunogenic reactions from pharmaceutical formulations containing PEG.^{[36]}

It was found that vaccinations with Pfizer-BioNTech and Moderna SARS-CoV-2-mRNA vaccines, lead to an increase in anti-PEG IgM and IgG antibodies by 68.5-fold and 13.1-fold for mRNA-1273 and 2.64-fold and 1.78-fold for the Pfizer-BioNTech vaccine.^{[37]} Both formulations contain 1.5 mol% of their respective PEGylated lipid, which equates to 50 µg for BNT162b2 and a calculated amount of 0.12 mg for the Moderna vaccine.^{[38]} Due to the mentioned immunogenic effect of PEG and the possible long-term problems arising from it, PEG alternatives are explored.

### Polyglycerol

Polyglycerol (PG) is a class of biocompatible, non-toxic and water-soluble polymers.^{[39],[40]} In comparison to PEG which only has two functional end groups, polyglycerols contain multiple free hydroxyl groups, which can be further functionalized to change the properties of the polymer to the desired needs or attach targeting moieties, biological agents and imaging residues.^{[39],[41],[42]} Different architectures of PG can be synthesized via polymerization of glycidol or glycidol derivatives.

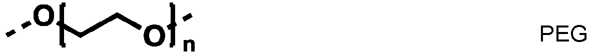

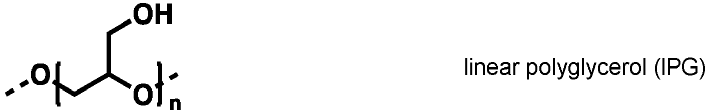

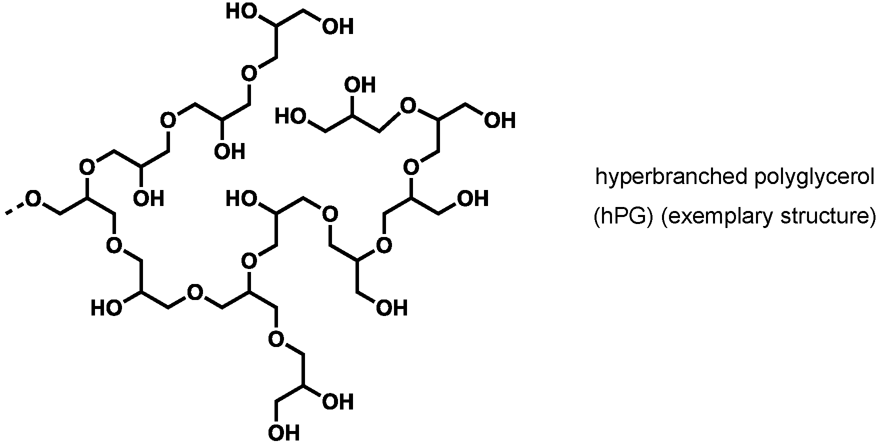

Due to the similar structure and properties of linear polyglycerol (IPG) and PEG, IPG has been used as a PEG-replacement in liposome formation. There it could be shown that use of IPG lead to longer circulation half-life compared to PEG of the same molecular weight, and even after repeated administration of IPGylated liposomes no occurrence of the ABC phenomenon was observed.[43] Attaching IPG to proteins instead of PEG could also increase circulation half-life and no binding to anti-PEG antibodies could be observed.[44],[45] IPG can be obtained via monomer activated anionic ring-opening polymerization or living anionic ring opening polymerization, giving the polymer with a narrow weight distribution.

It is an object of the present invention to provide polymer lipids that are suited for LNP formulations and do not require PEG.

This object is achieved with a compound having the claim elements of claim 1. Such a compound corresponds to general formula (I):

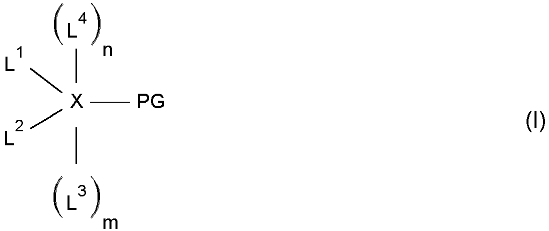

In this context, the residues have the following meaning:
- L¹, L², L³, L⁴: denote independently from each other a lipid moiety,
- n: is 0 or 1,
- m: is 0 or 1,
- X: is a linker moiety, and
- PG: is a polyglycerol moiety,

In an embodiment, the compound is claimed with the proviso that the compound does not have a structure according to formula (II):

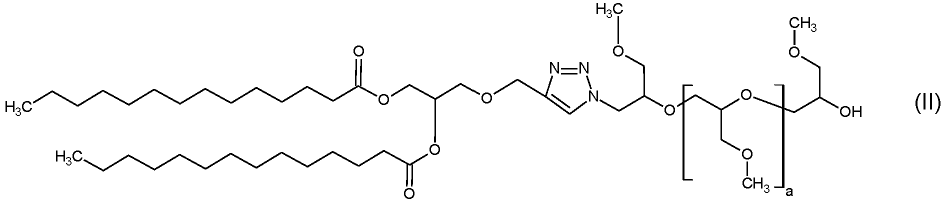

In this context,
- a: is any number from 1 to 100, in particular from 5 to 95, in particular from 10 to 90, in particular from 20 to 80, in particular from 30 to 60, in particular from 40 to 50, in particular 35 to 40, in particular 38.

In an embodiment, the compound is claimed with the proviso that the compound does not have a structure according to formula (III):

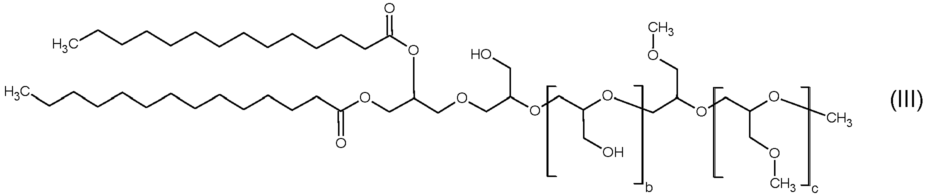

In this context,
- b, c: are independently from each other any number from 0 to 100, in particular from 5 to 95, in particular from 10 to 90, in particular from 20 to 80, in particular from 30 to 60, in particular from 40 to 50, with the proviso that a sum of b and c is a number from 10 to 200, in particular from 15 to 190, in particular from 20 to 180, in particular from 30 to 170, in particular from 40 to 160, in particular from 50 to 150, in particular from 60 to 140, in particular from 70 to 130, in particular from 80 to 120, in particular from 90 to 110.

In an embodiment, b is a number from 10 to 15, in particular 14, and c is a number from 10 to 15, in particular 13.

In an embodiment, the compound is claimed with the proviso that the compound does not have a structure according to formula (II) nor according to formula (III).

In an embodiment, L¹, L², L³, L⁴ are independently from each other a lipid moiety chosen from the group consisting of natural linear fatty acid residues, natural branched fatty acid residues, synthetic linear fatty acid residues, synthetic branched fatty acid residues, sterol lipids, and prenol lipids.

Particularly appropriate natural linear fatty acid residues are a lauric acid residue, a myristic acid residue, a palmitic acid residue, and a stearic acid residue. Thus, in an embodiment, L¹, L², L³, L⁴ are independently from each other chosen from the group consisting of a lauric acid residue, a myristic acid residue, a palmitic acid residue, and a stearic acid residue.

Particularly appropriate natural branched fatty acid residues are phytanic acid (3,7,11,15-tetramethylhexadecanoic acid), 3-hydroxyphytanic acid, 2-hydroxyphytanic acid, pristanic acid (2,6,10,14-tetramethylpentadecanoic acid), 4,8,12-trimethyltridecanoic acid, 2,6,10-trimethyldodecanoic acid, iso-fatty acids (having an iso-branching at ω-2 position), such as iso-palmitic acid (13-methyltetradecanoic acid), iso-stearic acid (15-methylhexadecanoic acid), iso-myristic acid (11-methyldodecanoic acid), and anteiso-fatty acids (having an anteiso-branching at ω-3 position), such as anteiso-palmitic acid (12-methyltetradecanoic acid), anteiso-stearic acid (14-methylhexadecanoic acid), and anteiso-myristic acid (10-methyldodecanoic acid).

Particularly appropriate synthetic linear fatty acid residues are short-chain synthetic fatty acids (e.g., nonanoic acid, undecanoic acid), medium-chain synthetic fatty acids (e.g., tridecanoic acid, pentadecanoic acid), long-chain synthetic fatty acids (e.g., heptadecanoic acid, nonadecanoic acid), very long-chain synthetic fatty acids (e.g.,heneicosanoic acid, tricosanoic acid, pentacosanoic acid, heptacosanoic acid).

Particularly appropriate synthetic branched fatty acid residues are iso-branched synthetic fatty acids (methyl at ω-2 position) (e.g., iso-nonanoic acid, iso-undecanoic acid, iso-tridecanoic acid, iso-pentadecanoic acid), anteiso-branched synthetic fatty acids (methyl at ω-3 position) (e.g., anteiso-nonanoic acid, anteiso-undecanoic acid, anteiso-tridecanoic acid, anteiso-pentadecanoic acid), and multiple methyl-branched synthetic fatty acids (e.g., 2,4-dimethyloctanoic acid, 3,5,7-trimethyldodecanoic acid, 2,6,10-trimethylpentadecanoic acid, 4,8,12-trimethylhexadecanoic acid)

Examples of particularly appropriate sterol lipids are cholesterol and derivatives, cholesteryl esters, phytosterols and derivatives, marine sterols and derivatives, fungal sterols and derivatives, steroids, C18 steroids (estrogens) and derivatives, C19 steroids (androgens) and derivatives, C21 steroids (gluco/mineralocorticoids, progestogins) and derivatives, secosteroids, vitamin D2 and derivatives, vitamin D3 and derivatives, bile acids and derivatives, C24 bile acids, alcohols, and derivatives, C26 bile acids, alcohols, and derivatives, C27 bile acids, alcohols, and derivatives, C28 bile acids, alcohols, and derivatives, steroid conjugates, glucuronides, sulfates, glycine conjugates, taurine conjugates, and hopanoids.

Examples of particularly appropriate prenol lipids are C5 isoprenoids, C10 isoprenoids (monoterpenes), C15 isoprenoids (sesquiterpenes), C20 isoprenoids (diterpenes), C25 isoprenoids (sesterterpenes), C30 isoprenoids (triterpenes), C40 isoprenoids (tetraterpenes), polyterpenes, quinones and hydroquinones, ubiquinones, vitamin **E,** vitamin K, polyprenols, bactoprenols, bactoprenol monophosphates, bactoprenol diphosphates, phytoprenols, phytoprenol monophosphates, phytoprenol diphosphates, dolichols, dolichol monophosphates, and dolichol diphosphates.

In an embodiment, the natural linear fatty acid residues, the natural branched fatty acid residues, the synthetic linear fatty acid residues, the synthetic branched fatty acid residues, the sterol lipids, and/or the prenol lipids are modified with a functional group chosen from the group consisting of amines, ethers, esters, amides, aryl substituents, and heteroaryl substituents.

In an embodiment, L¹, L², L³, L⁴ are independently from each other a C8-C24 lipid moiety, in particular a C9-C23 lipid moiety, in particular a C10-C22 lipid moiety, in particular a C11-C21 lipid moiety, in particular a C12-C20 lipid moiety, in particular a C13-C19 lipid moiety, in particular a C14-C18 lipid moiety, in particular a C15-C17 lipid moiety, in particular a C14-C16 lipid moiety.

In an embodiment, at least L¹ and L² have the same meaning.

In an embodiment, n and m are 0 so that the compound only comprises L¹ and L² as lipid moieties. This embodiment can be very well combined with preceding the explained embodiment, according to which L¹ and L² have the same meaning.

In an embodiment, L¹ and L² both are a myristic acid residue. It turned out that a compound carrying two myristic acid residues as lipid moieties has particularly appropriate properties, as will be explained below in further detail with reference to the exemplary embodiments.

In an embodiment, the linker moiety comprises a functional group chosen from the group consisting of amines, amides, esters, phosphates, aromatic carbocycles, non-aromatic carbocycles, aromatic heterocycles, and non-aromatic heterocycles. An amide, an ether, and a triazole residue are particularly appropriate examples of functional groups being present in the linker moiety.

Compound according to any of the preceding claims, **characterized** in that the linker moiety corresponds to general formula (IV) or (V):

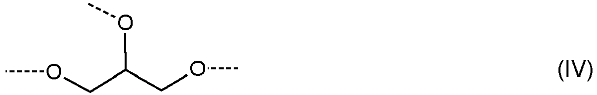

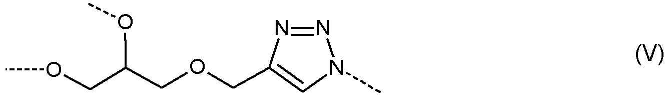

In this context, the dashed lines in general formulae (IV) and (V) indicate a covalent bond to the lipid moiety or the polyglycerol moiety bound to the linker moiety.

Generally, the polyglycerol moiety can be a hyperbranched polyglycerol (hPG). However, in an embodiment, the polyglycerol moiety is a substituted or non-substituted linear polyglycerol (IPG). It turned out that linear polyglycerols are particularly appropriate for subsequent formation of lipid nanoparticles.

In an embodiment, 0 % to 100 %, in particular 5 % to 99 %, in particular 10 % to 95 %, in particular 20 % to 90 %, in particular 30 % to 80 %, in particular 40 % to 70 %, in particular 50 % to 60 %, of the hydroxyl groups of the polyglycerol moiety are substituted with at least one substituent chosen from the group consisting of alkyl ethers, esters, amines, sulfates, sulfonates, phosphates, and phosphonates. Expressed in other words, the polyglycerol moiety has a degree of substitution lying in a range from 0 % to 100 %.

In an embodiment, the polyglycerol moiety is a substituted linear polyglycerol, wherein i) first polyglycerol units carry a first substituent and ii) second polyglycerol units carry a second substituent or being non-substituted. These first and second polyglycerol units form, in an embodiment, a statistical copolymer in which the first polyglycerol units and the second polyglycerol units are statistically distributed. In another embodiment, the first and second polyglycerol units form a block copolymer in which the first polyglycerol units and the second polyglycerol units are block-wise arranged.

In an embodiment, the compound corresponds to any of general formulae (Vla), (Vlb), (VIc), or (Vld):

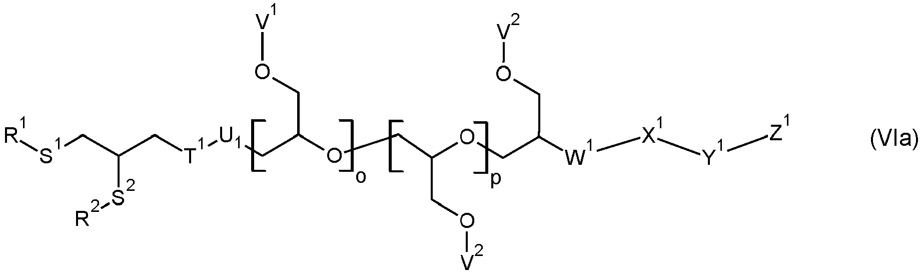

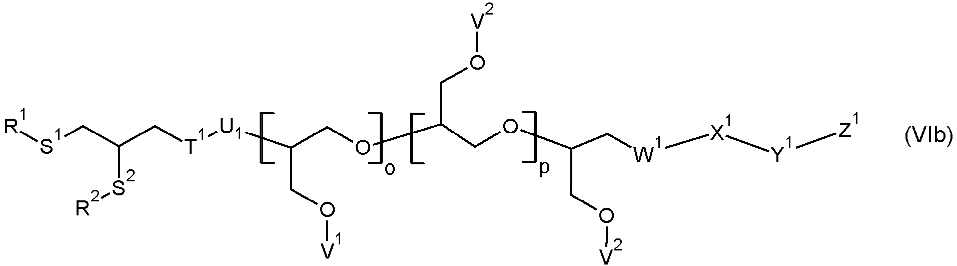

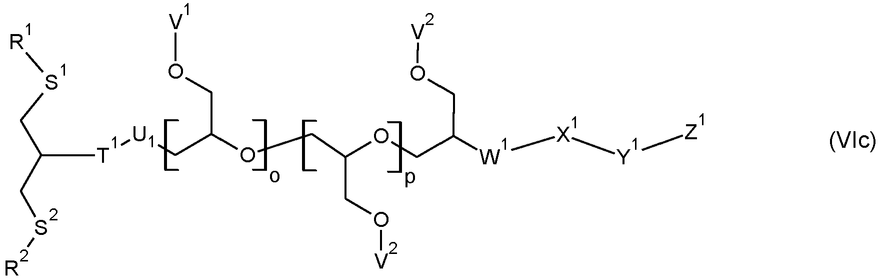

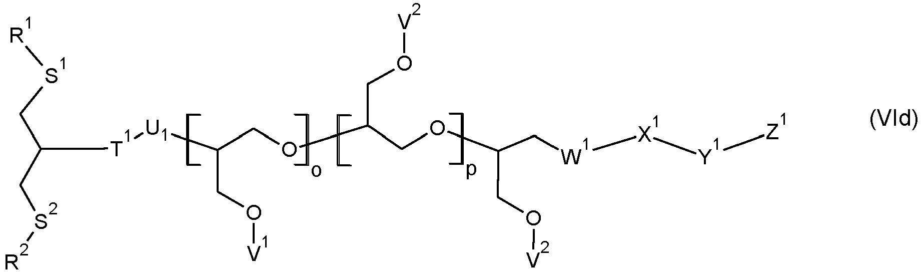

In this context, the residues have the following meanings:
- R¹, R²: denote independently from each other a C8-C22 (in particular C9-C21, in particular C10-C20, in particular C11-C19, in particular C12-C18, in particular C13-C17, in particular C14-C16, in particular C13-C15) linear or branched alkyl or alkenyl residue, a sterol lipid residue, or a prenol lipid residue, wherein the C8-C22 linear or branched alkyl or alkenyl residue, the sterol lipid residue, and/or the prenol lipid residue optionally carry a functional group chosen from the group consisting of amines, ethers, esters, amides, aryl substituents, and heteroaryl substituents,
- S¹, S²: denote independently from each other O, S, C(O)O, C(O)NH, C(O)NCH₃, NCH₃,
- T¹: denotes OCH₂, OC₂H₄, OC₃H₆, SCH₂, SC₂H₄, SC₃H₆, NHCH₂, OC(O), NHC(O), or is absent,
- U¹: denotes NH, O, or a triazole residue,
- V¹, V²: denote independently from each other H, CH₃, CH(CH₃)OCH₂CH₃, or a statistical mixture of at least two of H, CH₃, and CH(CH₃)OCH₂CH₃, wherein a percentage of an individual of H, CH₃, and CH(CH₃)OCH₂CH₃ in the statistical mixture, if present in the statistical mixture, lies within a range from 0.1 % to 99.9 %,
- o, p: are independently from each other any number from 0 to 100, in particular from 5 to 95, in particular from 10 to 90, in particular from 20 to 80, in particular from 30 to 60, in particular from 40 to 50, with the proviso that a sum of o and p is a number from 10 to 200, in particular from 15 to 190, in particular from 20 to 180, in particular from 30 to 170, in particular from 40 to 160, in particular from 50 to 150, in particular from 60 to 140, in particular from 70 to 130, in particular from 80 to 120, in particular from 90 to 110,
- W¹: denotes O, S or NH,
- X¹: denotes H, CH₃, or a C1-C4 alkylidene chain, with the proviso that Y' and Z¹ are absent if X¹ denotes H or CH₃,
- Y¹: is absent or denotes CHO, COOH, CH=CH₂, N₃, a triazole residue, O, S, NH, or NCH₃, with the proviso that Z¹ is absent if Y' denotes CHO, COOH, CH=CH₂, or N₃, and
- Z¹: is absent or denotes CH₂COOH, CH₂CHO, C2-C5alkyl-NH, C2-C5alkyl-NCH₃, CH₂C(O)NH, CH₂C(O)NCH₃, a biologically active targeting moiety, or H.

The term "alkenyl" is to be understood as encompassing alkenyls with individual double bonds as well as polyenyls such as dienyls (comprising two double bonds), trienyls (comprising three double bonds), or alkenyls with more than three double bonds.

In an embodiment, X¹ denotes H, CH₃, or a C1-C4 alkylidene chain; and Y' and Z¹ are absent.

In an embodiment, the compound corresponds to general formula (Vla) or (Vlb).

In an embodiment, the embodiments of the two preceding paragraphs are combined, i.e., the compound corresponds to general formula (Vla) or (Vlb), X¹ denotes H, CH₃, or a C1-C4 alkylidene chain, and Y' and Z¹ are absent.

In an embodiment, S¹ and S² denote independently from each other O or C(O)O.

In an embodiment, X¹ denotes H, CH₃, or a C1-C4 alkylidene chain, Y' and Z¹ are absent, and S¹ and S² denote independently from each other O or C(O)O.

In an embodiment, the compound corresponds to general formula (Vla) or (Vlb), and S¹ and S² denote independently from each other O or C(O)O.

In an embodiment, the compound corresponds to general formula (Vla) or (Vlb), X¹ denotes H, CH₃, or a C1-C4 alkylidene chain, Y' and Z¹ are absent, and S¹ and S² denote independently from each other O or C(O)O.

In an aspect, the present invention relates to a pharmaceutical composition comprising a compound according to the preceding explanations. In an embodiment, the compound serves in the pharmaceutical composition as carrier for a pharmaceutically active therapeutic substance (such as a drug), a therapeutic oligonucleotide (in particular RNA such as mRNA), or a therapeutic polynucleotide (in particular RNA such as mRNA). Expressed in other words, the compound serves, in an embodiment, as drug carrier, as oligonucleotide carrier, and/or as polynucleotide carrier.

In an embodiment, the pharmaceutical composition is a vaccine. In an embodiment, the vaccine is a vaccine against a virus causing an infection of the respiratory system of the patient, such as an influenza virus or a corona virus like SARS-CoV-2.

The term "SARS-CoV-2" or "severe acute respiratory syndrome coronavirus 2", as used herein, refers to any variant that is classified as SARS-CoV-2. In some embodiments, the SARS-CoV-2 described herein is at least one SARS-CoV-2 variant selected from the group consisting of Alpha, Beta, Gamma, Delta or Omicron variant. In some embodiments, the SARS-CoV-2 Omicron variant is at least one SARS-CoV-2 Omicron sub-lineage such as BA.1, BA.2, BA.3, BA.4, or BA.5.

In an aspect, the present invention relates to a method for preparing a vaccine from a pharmaceutical composition comprising a compound according to the preceding explanations.

In an aspect, the present invention relates to a compound according to the preceding explanations for medical use, namely, for administering a pharmaceutically active therapeutic substance (such as a drug), a therapeutic oligonucleotide (in particular RNA such as mRNA), or a therapeutic polynucleotide (in particular RNA such as mRNA) to a human or animal patient.

In an aspect, the present invention relates to a medical method comprising administering a pharmaceutically active therapeutic substance (such as a drug), a therapeutic oligonucleotide (in particular RNA such as mRNA), or a therapeutic polynucleotide (in particular RNA such as mRNA) encapsulated within a compound according to the preceding explanations to a human or animal patient in need thereof.

In an embodiment, the medical method is a method of vaccinating a human or animal patient in need thereof. The animal patient is in particular a non-human mammal such as rodents, canines, felines, or mustelids.

In an aspect, the present invention relates to the use of a compound according to the preceding explanations as carrier for a substance in an in-vitro method for delivering the substance to an intended site of action. Such use may be helpful, e.g., in transfection experiments.

In an aspect, the invention relates to a method for manufacturing a compound according to general formula (I), the method comprising the following steps:
a) reacting an alkyne-functionalized diacyl glycerol in a click coupling reaction with a mono azido poly glycidyl ether, the side chains of which are protected by a protection group, to obtain a diacyl glycerol poly glycidyl ether;
b) optionally purifying the diacyl glycerol poly glycidyl ether;
c) deprotecting the diacyl glycerol poly glycidyl ether by removing the protecting group.

The diacyl glycerol poly glycidyl ether is a bifunctional polymer comprising a lipid tail (represented by the diacyl residue) and a polyglycerol part (represented by the poly glycidyl ether).

In an embodiment, the diacyl residue is chosen from a dilauric acid residue, a dimyristic acid residue, a dipalmitic acid residue, and a distearic acid residue. A dimyristic acid residue is particularly appropriate.

In an embodiment, the click coupling reaction is a copper-catalyzed click coupling reaction. In an embodiment, copper bromide is used as copper source for the copper-catalyzed click coupling reaction. In an embodiment, N,N,N',N",N"-pentamethyldiethylenetriamine (PMDETA) is used as catalyst for the (copper-catalyzed) click coupling reaction.

In an embodiment, the protecting group is an ethoxyethyl group.

In an embodiment, the diacyl glycerol poly glycidyl ether comprises a triazole residue linking the diacyl glycerol residue and the poly glycidyl ether residue.

In an embodiment, the optional purification step is carried out by eluting the diacyl glycerol poly glycidyl ether through a purification column, in particular an aluminum oxide purification column.

All embodiments of the compound can be combined in any desired way and can be transferred either individually or in any arbitrary combination to the pharmaceutical composition, to the uses, and to the different methods. Likewise, all embodiments of the pharmaceutical composition can be combined in any desired way and can be transferred either individually or in any arbitrary combination to the compound, to the uses, and to the different methods. Likewise, all embodiments of the different uses can be combined in any desired way and can be transferred either individually or in any arbitrary combination to the compound, to the pharmaceutical composition, to the respective other uses, and to the different methods. Furthermore, all embodiments of the different methods can be combined in any desired way and can be transferred either individually or in any arbitrary combination to the compound, to the pharmaceutical composition, to the uses, and to the respective other methods.

Further details of aspects of the present invention will be explained in the following making reference to exemplary embodiments and accompanying Figures. In the Figures:
- Figure 1: is a general synthesis plan for the alkyne functionalized 1,2-dimyristoyl- glycerol (DMG) derivative 11 and subsequent coupling to mono azido-functionalized linear polymers via copper(I)-catalyzed azide-alkyne cycloaddition (CuAAC);
- Figure 2: illustrates that an esterification of myristic acid 27 with diol 22 resulted in DMG-alkyne 11;
- Figure 3: illustrates the CuAAC-reaction of α-azido-poly([(1-ethoxyethoxy)methyl]oxirane) (α-azido-PEEGE) coupled to DMG-alkyne to give DMG-T-PEEGE4500 23;
- Figure 4: shows an ¹H-NMR spectrum of DMG-T-PEEGE4500 23;
- Figure 5: illustrates the deprotection of DMG-T-PEEGE4500 **23** to give DMG-T-IPG2250 3;
- Figure 6: illustrates the synthesis of DMG-T-IPG3750 4 via coupling to PEEGE and subsequent deprotection of 24;
- Figure 7: shows an HMQC-NMR spectrum of DMG-T-IPG2250 3 in [d4]MeOD;
- Figure 8: shows a Dosy-NMR spectrum of DMG-T-IPG2250 3;
- Figure 9: illustrates the synthesis of DMG-T-MeOIPGx **8, 9** via CuAAC of **11;**
- Figure 10: illustrates the general synthesis plan for the succinimidyl carbonate activated DMG 12;
- Figure 11: illustrates the esterification of diol **26** with myristic acid **27** to give benzyl ether protected DMG 28;
- Figure 12: illustrates the deprotection of benzyl ether, resulting in DMG 29;
- Figure 13: illustrates the synthesis of succinimidyl carbonate-activated DMG **12** via coupling of DMG **29** and disuccinimidyl carbonate;
- Figure 14: illustrates the synthesis of DMG-C-PEEGE **30** via carbamate coupling of carbonate **12** to PEEGE amine 18;
- Figure 15: illustrates the deprotection of **30,** resulting in DMG-C-IPG 5;
- Figure 16A: shows an ¹³C-NMR spectrum of DMG-carbonate **12** in acetone-d₆;
- Figure 16B: shows an ¹³C-NMR spectrum of DMG-C-PEEGE4500 **30** in acetone-d₆ after coupling to 12;
- Figure 17A: shows an HMBC-NMR spectrum of **30** in acetone-d₆;
- Figure 17B: shows a COSY-NMR spectrum of **30** in acetone-d₆;
- Figure 18: illustrates the synthesis of different alkyne- and thiol-lipid tails for subsequent CuAAC or thiol-ene click reactions with an azide or alkyne functionalized polymer;
- Figure 19A: illustrates the applied general reaction scheme for the synthesis of lipid-T-PEEGE-S-COOH;
- Figure 19B: illustrates the applied general reaction scheme for the synthesis of H₂N-PEEGE-S-lipid;

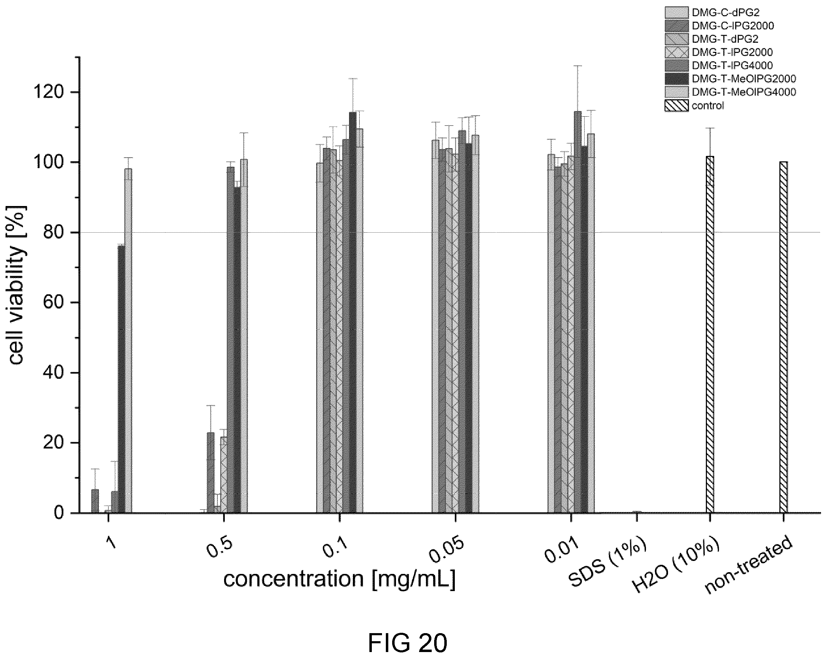

- Figure 20: shows the cytotoxicity profiles of polymer lipids **3-9** on A549 cells after 48 h treatment;
- Figure 21A: shows the stability of LNPs formulated with LP-01E/chol./DSPC/3 (50/38/9/3) and poly(A) after storage for 7 d at 4 °C or -80 °C for two different N/P ratios;
- Figure 21B: shows the encapsulation efficiency of the LNPs of Figure 21A before and after storage at 4 °C or -80 °C;
- Figure 22: illustrates the size, PDI, and encapsulation efficiency of LNPs formulated with different amounts of compound 3 compared to PEG lipid;
- Figure 23: shows the results of an eGFP-mRNA assay determining cellular uptake and biological activity for various LNP formulations;
- Figure 24: shows the results of an assessment of ATP values for the LNP-formulations of Figure 23;
- Figure 25: shows the results of an assessment of LDH for the LNP-formulations of Figure 23;
- Figure 26: illustrates the synthesis of H₂N-PEEGE-S-lipid;
- Figure 27: illustrates the thiol-ene click reaction of lipid-T-PEEGE-allyl;
- Figure 28: illustrates the synthesis of DMG alkyne;
- Figure 29: illustrates the synthesis of 3,3'-disulfanediylbis(propane-1,2-diol);
- Figure 30: illustrates the synthesis of DMG disulfide;
- Figure 31: illustrates the synthesis of DMG SH;
- Figure 32: illustrates the synthesis of 3-(tritylthio)propane-1,2-diol;
- Figure 33: illustrates the synthesis of BisC14-STrt;
- Figure 34: illustrates the synthesis of 2,3-bis(tetradecyloxy)propane-1-thiol (BisC14-SH);
- Figure 35: illustrates the synthesis of 1,2-dimyristoyl-3-O-benzyl-rac-glycerol (DMG-Bn)
- Figure 36: illustrates the synthesis of 1,2-dimyristoyl-rac-glycerol (DMG-OH);
- Figure 37: illustrates the synthesis of DMG succinimidyl carbonate (DMG SC);
- Figure 38: illustrates the synthesis of α-azido-ω-hydroxy-PEEGE4500 (N₃-PEEGE-OH_4.5kD), wherein n is 28 ± 2;

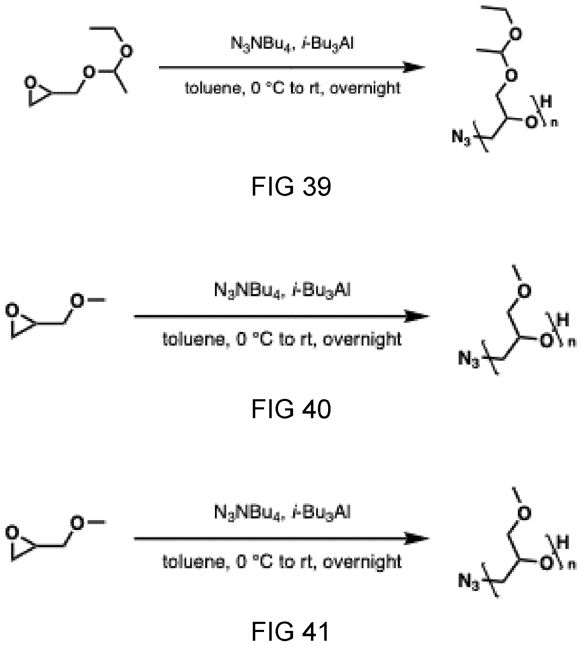

- Figure 39: illustrates the synthesis of α-azido-ω-hydroxy-PEEGE7500 (N₃-PEEGE-OH_7.5kD), wherein n is 60 ± 2;
- Figure 40: illustrates the synthesis of α-azido-ω-hydroxy-MeOIPG4000 (N₃-MeOIPG4.0kD), wherein n is 45 ± 2;
- Figure 41: illustrates the synthesis of α-azido-ω-hydroxy-MeOIPG2900 (N₃-MeOIPG2.9kD), wherein n is 22 ± 2;
- Figure 42: illustrates the synthesis of α-amino-PEEGE4500 (H₂N-PEEGE-OH_4.5kD);
- Figure 43: illustrates the synthesis of N₃-PEEGE-allyl;
- Figure 44: illustrates the synthesis of DMG-T-PEEGE4500;
- Figure 45: illustrates the synthesis of DSG-T-PEEGE5000;
- Figure 46: illustrates the synthesis of DMG-T-PEEGE7500;
- Figure 47: illustrates the synthesis of DMG-T-MeOIPG4000;
- Figure 48: illustrates the synthesis of DMG-T-MeOIPG2900;
- Figure 49: illustrates the synthesis of DMG-T-IPG2250;
- Figure 50: illustrates the synthesis of DMG-T-IPG3750;
- Figure 51: illustrates the synthesis of DMG-T-PEEGE-allyl;
- Figure 52: illustrates the synthesis of DMG-C-PEEGE4500;
- Figure 53: illustrates the synthesis of DMG-C-IPG2250;
- Figure 54: is a general synthesis scheme for IPG and MeOIPG-based polymeric lipids according to a second synthesis approach;
- Figure 55: is a general synthesis scheme for copolymeric IPG-block-MeOIPG and MeOIPG-block-IPG -based polymeric lipids;
- Figure 56A: shows the development of the size of mRNA LNP systems functionalized with variations of PEG and PG over 21 days;
- Figure 56B: shows the development of the polydispersity index (PDI) of the mRNA LNP systems of Figure 56A over 21 days;
- Figure 57: illustrates the concentration of PEG based on the anti-PEG antibody affinity as determined via competitive ELISA for a variety of PEG- and PG-functionalized LNPs;
- Figure 58: illustrates the cell viability as determined via CCK8-assay using HepG2 cells exposed to 1, 0.5, 0.1 and 0.05 µg mRNA/mL encapsulated in LNPs formulated with a variety of amphiphilic polymers derived from polyglycerol;
- Figure 59: shows the eGFP fluorescence intensity after cell lysis;
- Figure 60A: shows forward and side scatter of HepG2 cells transfected with (i) none or eGFP mRNA encapsulated in (ii) PEG- and (iii) PG-LNP systems;
- Figure 60B: shows histograms of eGFP fluorescence in each cell population of Figure 60A;
- Figure 60C: illustrates the eGFP fluorescence intensity in individual HepG2 cells after transfection with the LNP systems of Figure 60A; and
- Figure 60D: illustrates the percentage of eGFP positive cells per cell population of Figure 60A;

### Synthesis of triazole linked lipids

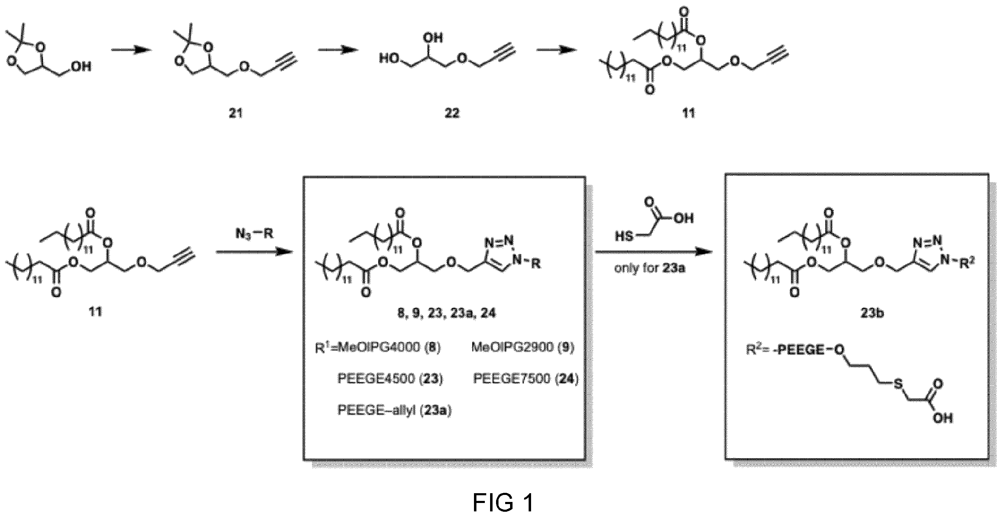

Alkyne functional dimyristoyl glycerol was obtained from solketal by following the reported method by Rashmi *et al.* ^{[46]}. After subsequent functionalization with propargyl bromide the acetal protected alkyne **21** can be obtained and after deprotection of acetal groups, diol **22** can be obtained. In a next step the fatty esters were coupled via esterification with myristic acid (27), giving the alkyne **11** (cf. Figure 1).^{[46],[47]}

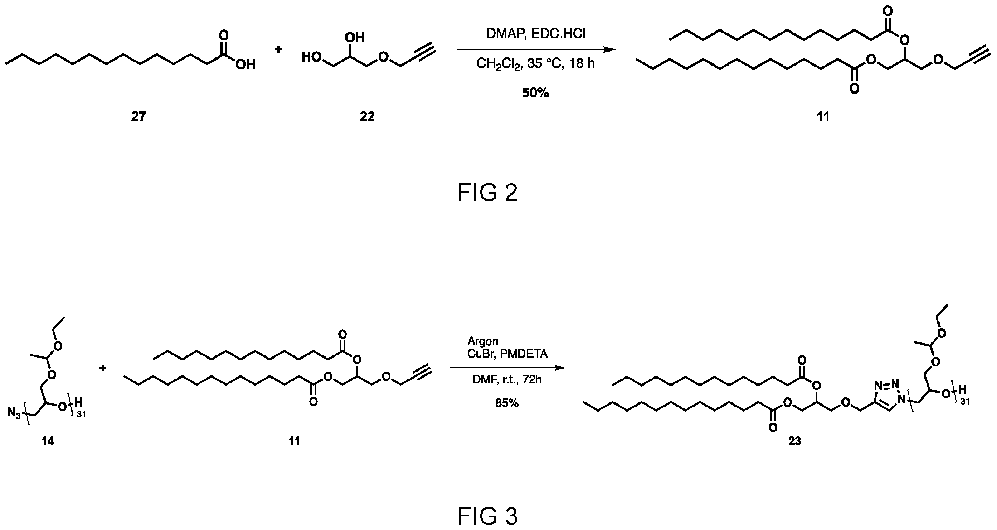

The esterification reaction of **22** with myristic acid **27** was carried out in dichloromethane (cf. Figure 2). After purification by flash column chromatography alkyne **11** was obtained in 50% yield. The product was characterized by ¹H-NMR, ¹³C-NMR and HRMS(ESI).

### Synthesis of DMG-T-IPG

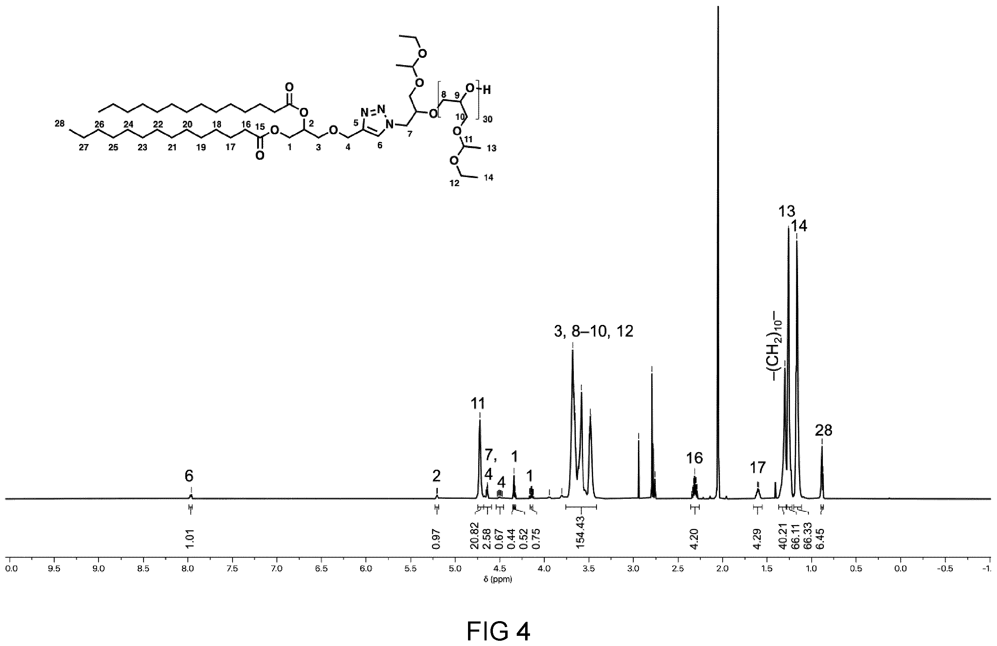

A copper-catalyzed click coupling reactions was carried out between DMG-alkyne **11** and the 4.5 kDa mono azido poly(ethoxy ethyl glycidyl ether) PEEGE **14** (cf. Figure 3), wherein the DMG-alkyne **11** was optionally dissolved in tetrahydro furan (THF). For the CuAAC reaction, CuBr was chosen as the copper(I) source together with PMDETA as a ligand, to solubilize the Cu(I) ions, and dry DMF as the solvent.^{[48],[49]} To avoid oxidation of the Cu(I) species, the reaction was carried out under inert conditions. To remove the copper species from the final product the compound was filtered over neutral aluminum oxide.^{[49]} The crude was further purified via dialysis in acetone, giving DMG-T-PEEGE4500 **23** in good yield (85%) and purity. Analysis of the compound **23** via ¹H-NMR and FTIR-spectroscopy (cf. Figure 4) proved the formation of a triazole. In the region with a chemical shift of around 8 ppm the triazole proton appears, indicating the successful coupling.

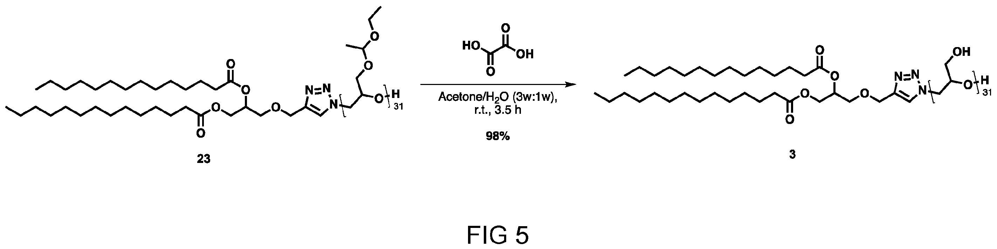

Next, acetal protecting groups of the PEEGE moiety had to be cleaved off under acidic conditions. Since the ester groups are to some degree also acid labile, even though the stability against hydrolysis increases with longer alkyl chain length, use of HCl seemed too harsh. Therefore, the strong weak acid, oxalic acid, was chosen to act as a milder cleaving agent. The deprotection of **23,** DMG-T-PEEGE4500, gave the desired product 3, DMG-T-IPG2250, after 3.5 hours in quantitative yield (cf. Figure 5). The characterization was done after dialysis in water, proving the successfully coupled product **3** via ¹H-, ¹³C-, HMQC-, COSY- and DOSY-NMR.

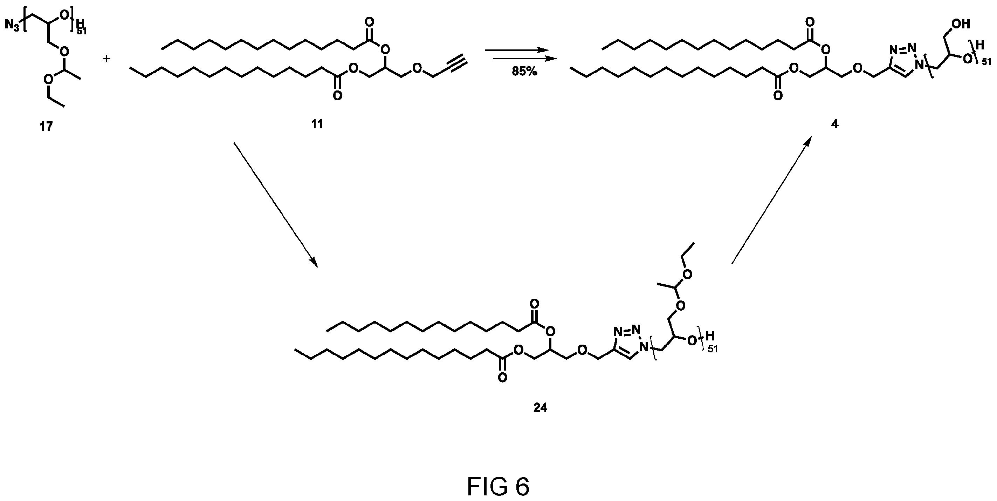

The synthesis was then repeated using the same conditions as for **3** to obtain DMG-T-IPG3750 **4** in good yield (85%) and purity (cf. Figure 6), confirmed by ¹H-NMR and ¹³C-NMR.

### Characterization of DMG-T-IPG

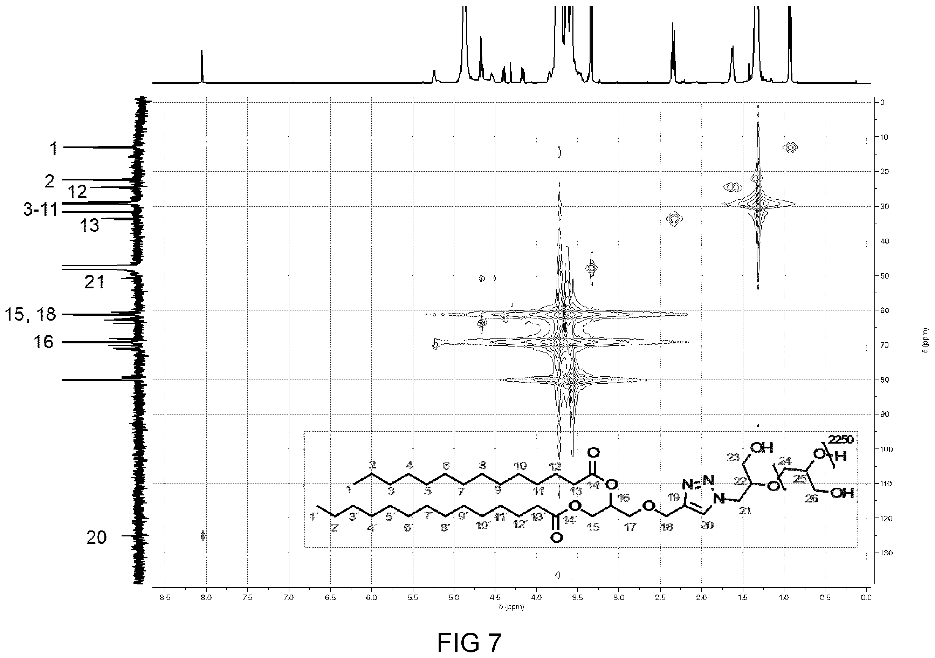

Characterization, using 2D-NMR-methods, was done in detail for DMG-T-IPG2250 3 (cf. Figure 7). Formation of the triazole was confirmed by HMQC-NMR. The peak with a chemical shift of around 8 ppm belongs to the hydrogen of the triazole unit, which shows a connectivity to a carbon with a chemical shift of around 125 ppm. Since no other carbons are expected to show any signal in this region, the formation of triazole could be confirmed.

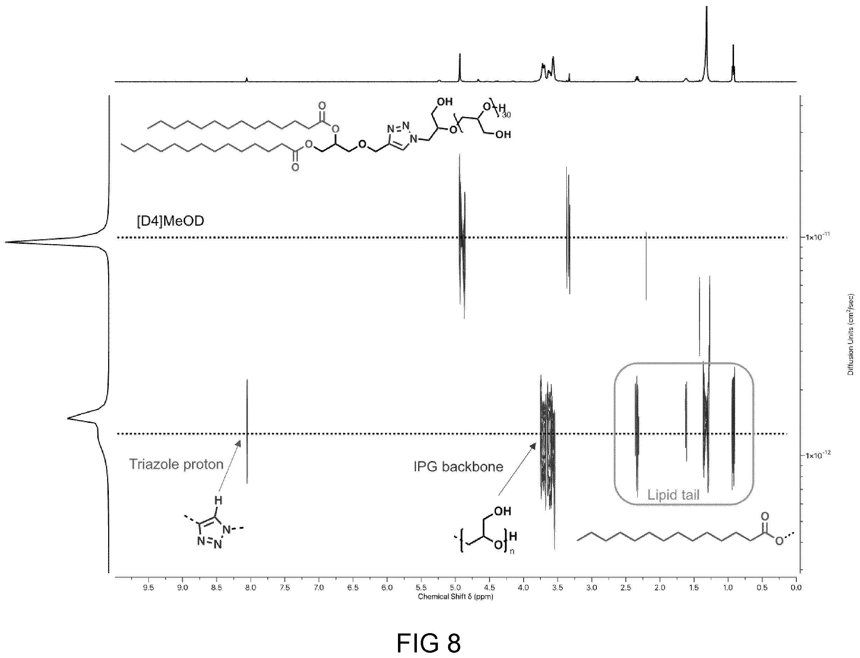

DOSY-NMR (cf. Figure 8) further proved that the fatty esters were still connected to the IPG. All the protons assigned to the coupled polymer lipid could be confirmed to belong to the same molecule, since the signals for the alkyl groups and the IPG backbone have the same diffusion units.

### Synthesis of DMG-T-MeOIPG

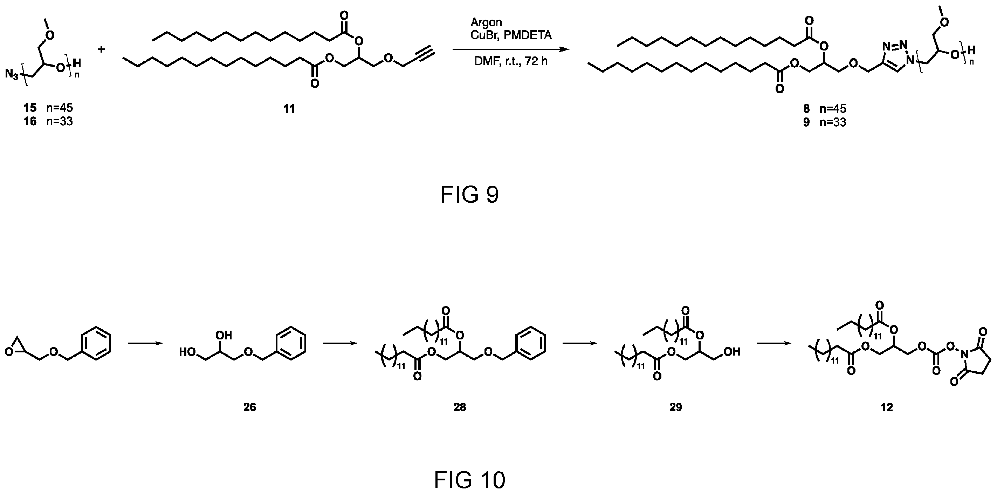

Polymer lipids coupled to poly(glycidyl methyl ether) **8, 9** were synthesized following the same procedure as for their EEGE analogues (cf. Figure 9). The yield of 49% for DMG-T-MeOIPG4000 **8** and 52% for DMG-T-MeOIPG2900 **9** was lower than for the IPG analogues **23** and **24.** The characterization was done after purification, confirming the successful synthesis of **8** via ¹H-, ¹³C-, HMQC-, COSY- and HMBC-NMR and for **9** via ¹H-, ¹³C-NMR.

### Synthesis of carbamate linked lipids

Figure 10 illustrates the general plan for the preparation of succinimidyl carbonate activated DMG **12.** The benzyl ether protected diol **26** can be obtained by simple hydrolysis of the commercially available glycidyl benzyl ether. The main reason for using benzyl ether as a protecting group, is the detectability through a UV-detector, possibly simplifying the purification via flash column chromatography. In a next step the diol can then be subjected to esterification with myristic acid **27** to form the benzyl ether protected 1,2-di-ester **28.** After deprotection of the alcohol via Pd-catalyzed hydrogenation, 1,2-dimyristoyl-glycerol **29** can be obtained. For the activation of the hydroxyl group, succinimidyl carbonate was chosen, based on the stability of the carbonate and mild reaction conditions with amines.^{[50]} The mixed carbonate is then ready to react with a primary amine to form the desired carbamates.

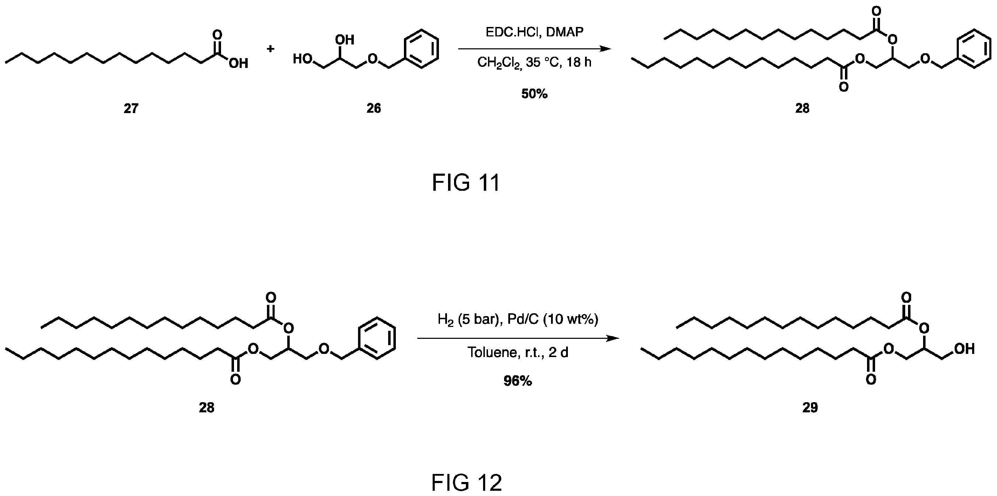

The first step was done according to a mixture of two literature known esterification procedures (cf. Figure 11).^{[46],[47]} Purification of the crude could be achieved by flash column chromatography in 100% CH₂Cl₂, giving pure benzyl ether protected DMG **28** in 50% yield.

Cleavage of the benzyl protecting group in **28** was done using a Pd/C-catalyst under hydrogen atmosphere in toluene (cf. Figure 12). The cleavage of the protecting group proceeded in good yield (96%) and the catalyst could be filtered off using Celite. No further purification was needed. The glycerol-based alcohol **29** was characterized by ¹H-NMR, ¹³C-NMR and HRMS(ESI).

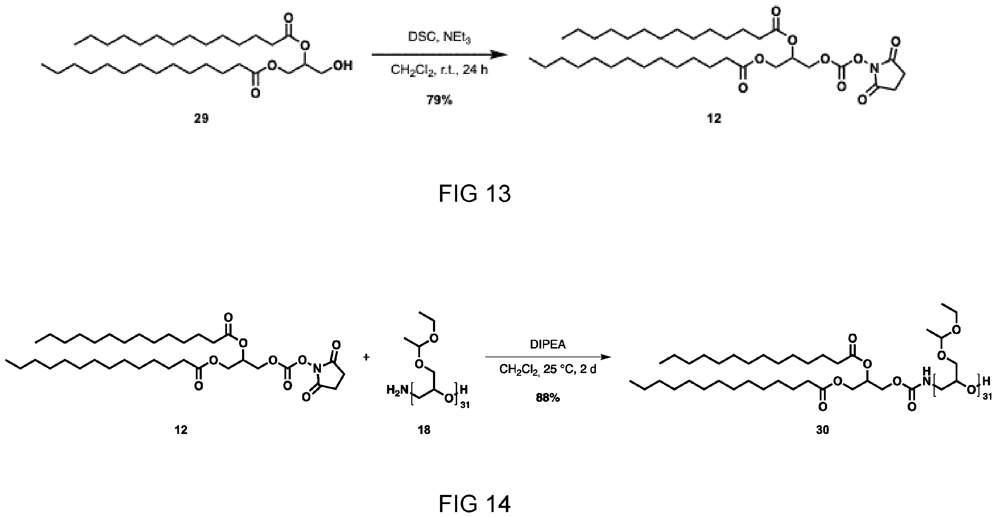

The preparation of mixed carbonates was done following the procedure described by Beloqui et al. ^{[51],[52]} (cf. Figure 13). Alcohol **29** was reacted with disuccinimidyl carbonate (DSC). After purification using flash column chromatography, the DMG-carbonate **12** could be obtained in good yield (79%), confirmed by ¹H-NMR, ¹³C-NMR and HRMS(ESI).

### Synthesis of DMG-C-IPG

Next, PEEGE amine **18** was coupled with the succinimidyl carbonate activated DMG **12** through formation of a carbamate bond, following the reaction procedure described by Beloqui et al. ^{[51]} (cf. Figure 14). Characterization via ¹H-, ¹³C-, HMQC- and HMBC-NMR confirmed the formation of the carbamate coupled DMG-C-PEEGE4500 **30.**

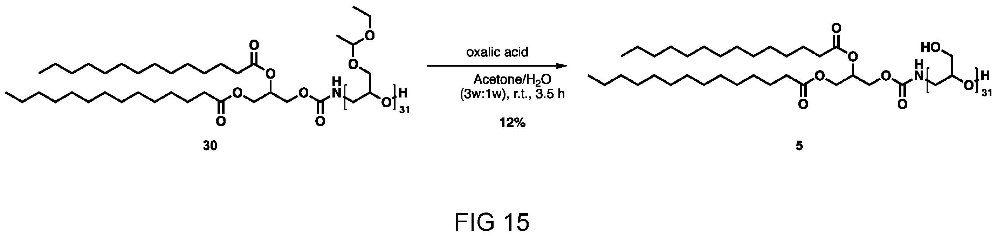

Deprotection of **30** followed the general procedure which was also used to cleave the acetal groups in DMG-T-PEEGE (cf. Figure 15). After dialysis in water, product **5** could only be obtained in 12% yield, although the cutoff used for the dialysis membranes was the same as for the triazole coupled analogue.

### Characterization of DMG-C-PEEGE

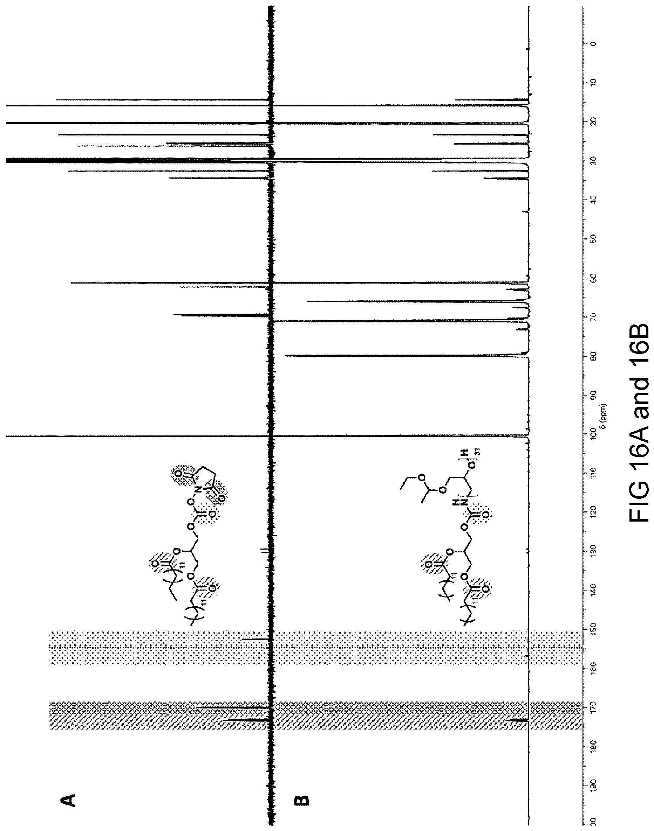

The coupling product was confirmed by ¹³C-NMR. The NMR spectrum showed that the imide-carbonyl peaks of the carbonate reactant **12** disappear after the coupling and carbon peaks belonging to the carbonate group shift to the low field, indicating that the coupling was successful. To be more precise, the ¹³C-NMR spectrum of DMG-carbonate **12** in acetone-d₆ illustrated in Figure 156A shows three signals of the ester, carbonate, and the imide-carbonyl carbon. The ¹³C-NMR spectrum of DMG-C-PEEGE4500 **30** in acetone-d₆ after coupling to **12** illustrated in Figure 16B shows no imide-carbonyl peak, and the carbamate peak was sifted to low field compared to carbonate.

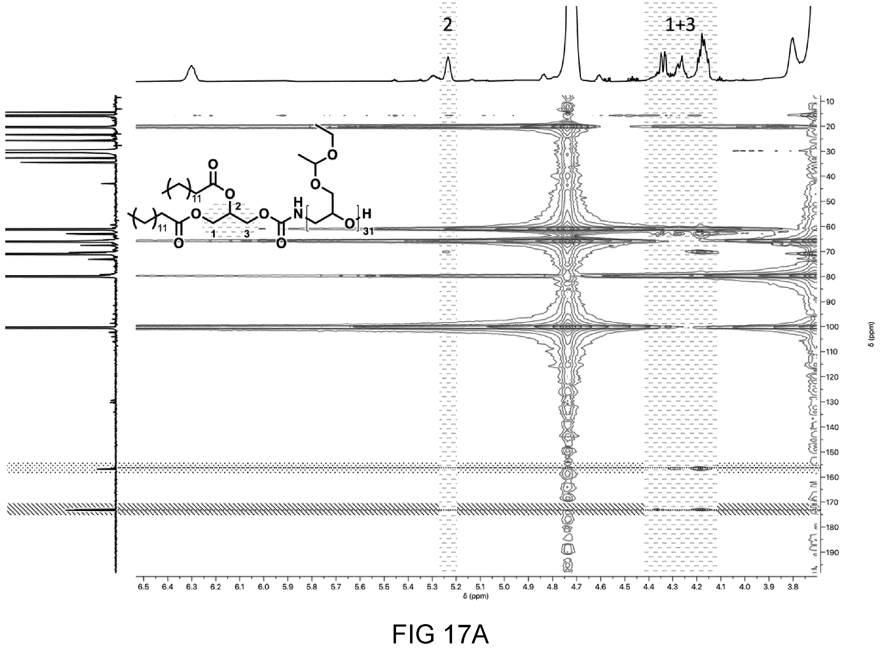

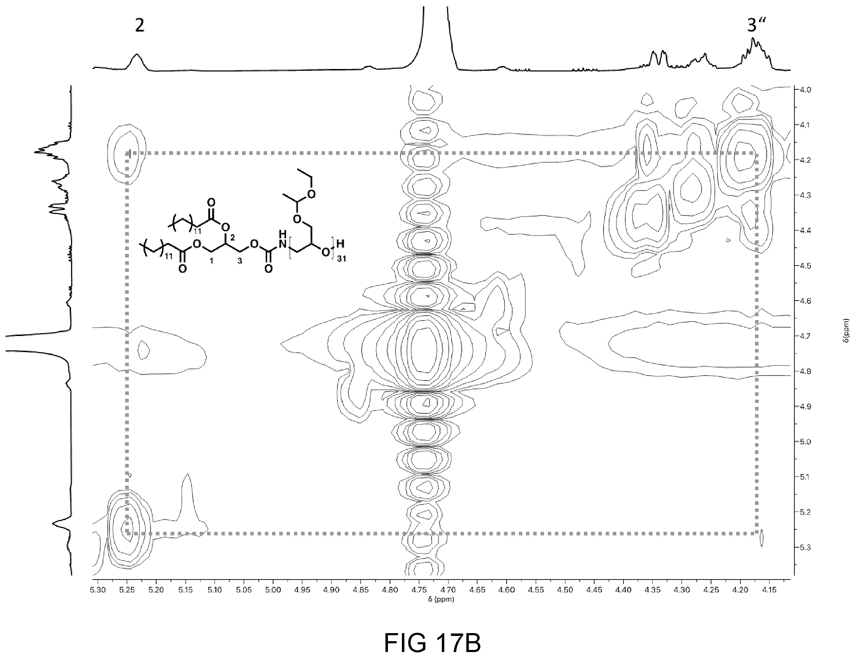

In addition, the HMBC-NMR spectrum of DMG-C-PEEGE **30** (cf. Figure 17A) showed that the carbonyl groups of the carbamate and the ester are still coupled to the glycerol spacer unit. Although the H-2 did not show connectivity to the ester carbonyl, as would be expected, it could be proven by COSY-NMR (cf. Figure 17B) that H-2 and H-3 are still coupling to each other.

### Synthesis of tailored polymer lipids

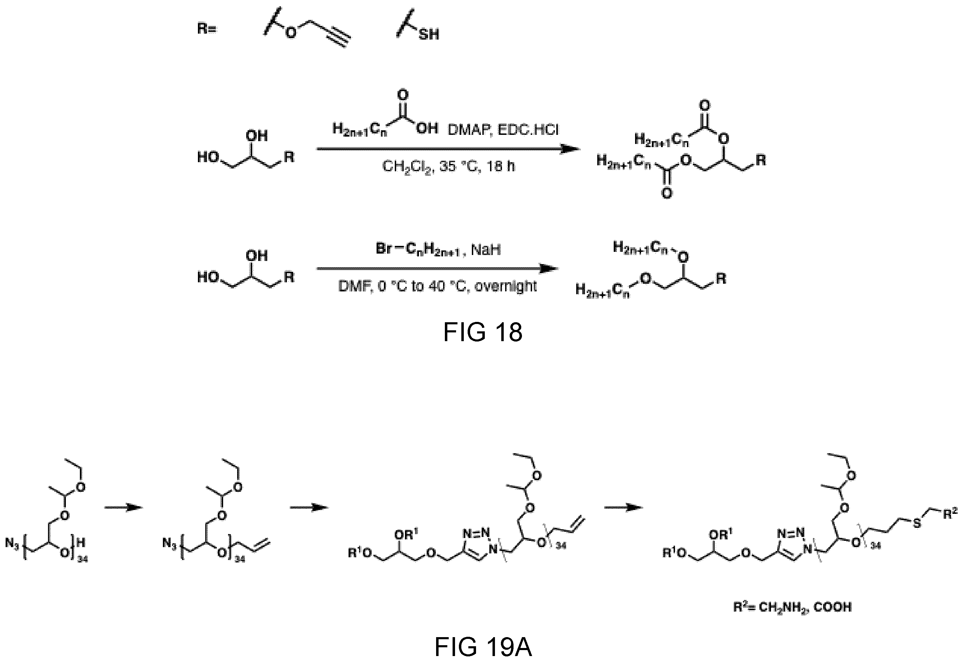

End-group functionalizable lipids can be obtained in two ways. The strategy shown in Figure 18 starts with the synthesis of the hetero-bifunctional polymer N₃-PEEGE-allyl. The polymer is then coupled to an alkyne functional lipid such as DMG-alkyne via CuAAC. Next, a thio-functional compound such as thioglycolic acid or cysteamine can be reacted with the terminal alkene, giving a polymer-lipid with a new terminal functionality such as carboxyl or amine connected through a newly formed thio-ether bond (cf. Figure 19A). The used ester was an DXG-alkyne ester, wherein "X" denotes a variable chain length of the used fatty acid. With n = 11 (lauric acid), DLG resulted; with n = 13 (myristic acid), DMG resulted; with n = 15 (palmitic acid), DPG resulted; and with n = 17 (stearic acid), DSG resulted (cf. Figure 18). Thus, when using DMG as lipid tail, DMG-T-PEEGE-S-COOH or DMG-T-IPG-S-COOH resulted. The chosen ether was a BisCY alkyne ether, wherein n = Y = 14 or 18.

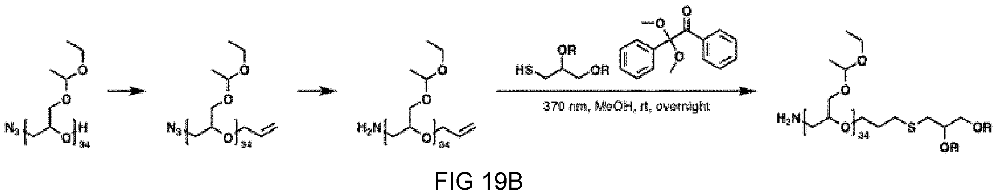

In a different approach, the azide moiety of N₃-PEEGE-allyl is first reduced to an amino group, using tris(2-carboxyethyl)phosphine) (TCEP). H₂N-PEEGE-allyl is then reacted with a thiol-functional lipid such as DMG-SH or BisC14-SH, giving H₂N-PEEGE-S-lipid (cf. Figure 19B).

### Cell viability of polymer lipids

*In vitro* cytotoxicity of dissolved polymer lipids 3-9 was investigated on A549 cells. For each compound a set of 5 concentrations between 1 mg/mL and 0.01 mg/mL in ultrapure water was tested after incubation for 48 h.

The two IPG-lipids with a shorter IPG chain length 3, 5 showed 100% cell viability up to 0.1 mg/mL and only 20% cell viability at 0.5 mg/mL. The IPG-lipid 4 with a higher M_{w} IPG attached, showed 100% cell viability up to 0.5 mg/mL but 0% at 1 mg/mL. Polyglycerol dendron lipids showed 100% cell viability up to 0.1 mg/mL but 0% cell viability at 0.5 mg/mL. Methoxylated IPG lipids **8, 9** showed no cytotoxicity up to 1 mg/mL. The MeOIPG-lipid **9** with lower M_{w} showed 80% cell viability at 1 mg/mL, while the higher M_{w} polymer-lipid **8** showed 100% cell viability up to 1 mg/mL. The general trend seems to be a decreasing cytotoxicity with increasing M_{w} of the hydrophilic unit.

### LNP Formulation with DMG-T-IPG2250

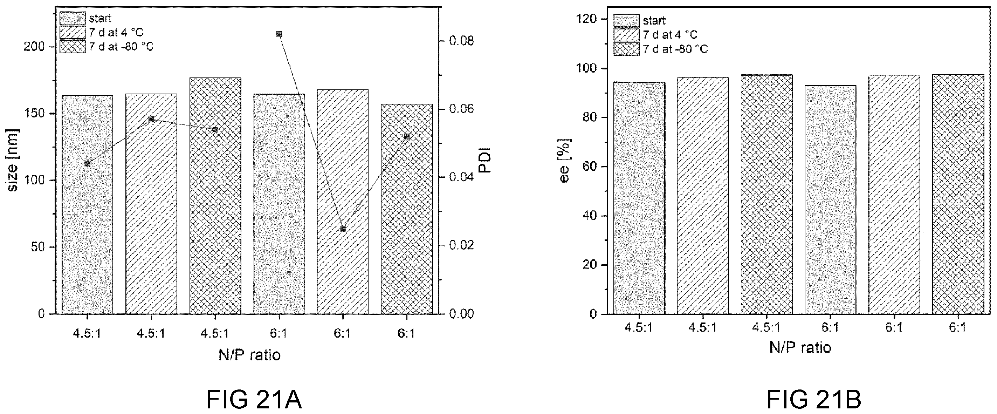

To test whether IPGylated lipids can replace PEGylated lipids to form stable mRNA encapsulating LNPs, a formulation consisting of LP-01E, cholesterol, DSPC and DMG-T-IPG2250 3 in a molar ratio of 50/38/9/3 was tested on particle size, PDI and encapsulation efficiency (EE%) for two different storage conditions (-80 °C and 4 °C) and N/P ratios (cf. Figures 21A and 21B). The N/P ratio is the molar ratio between the nitrogen of the ionizable lipid to phosphate on the RNA.^{[53]} The encapsulation efficiency is calculated according to equation 1 using a Ribogreen assay. Encapsulated RNA does not bind to Ribogreen; therefore, measuring the fluorescence gives the amount of unencapsulated RNA. Triton X-100 is used to disrupt the LNPs and release the mRNA, giving the total amount of RNA. $EE \left[%\right] = \frac{{RNA}_{total} - {RNA}_{unencapsulated}}{{RNA}_{total}} \times 100 %$

Poly(A) was chosen as a mRNA mimic for this experiment, giving particles with a size of 164 nm for both N/P-ratios. The size does not change significantly after storing at 4 °C or at -80 °C for 7 days and no leakage of poly(A) could be observed.

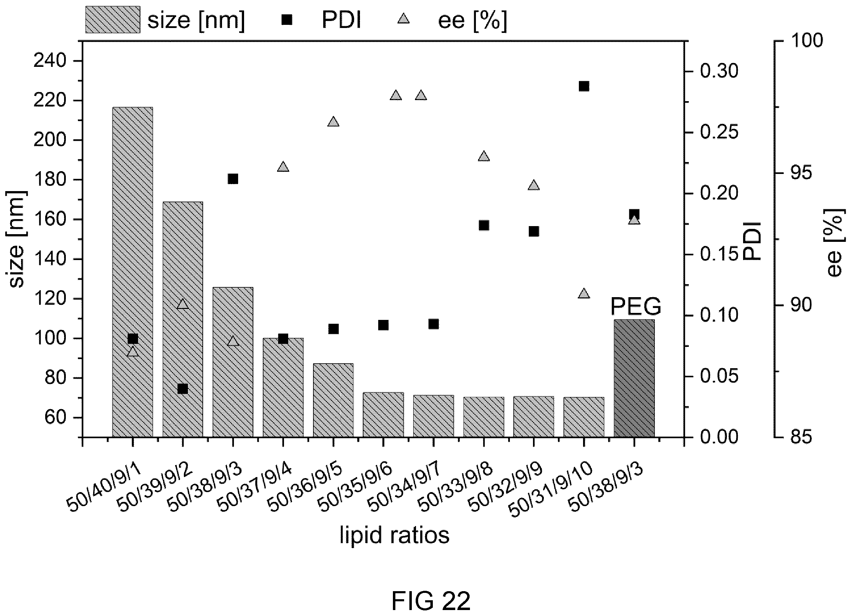

Next, characteristics of LNPs formulated with different degrees of IPGylated lipid as part of the same formulation, consisting of LP-01E, cholesterol, DSPC and lipid **3,** were tested in terms of size, PDI and encapsulation efficiency. The amount of ionizable lipid and DSPC were held constant, while the amount of IPG lipid was increased in steps of 1 mol% up to 10 mol%. As a control, DMG-PEG2000 **1** was used in a 50/38/9/3 formulation (3 mol% PEG-lipid). mRNA encoding eGFP, a green fluorescent protein, was used as a functional mRNA. With increasing amount of IPG-lipid, up to 6%, the particle size decreased from 217 nm to 71 nm. Comparing the particle properties of LNPs formulated with IPG-lipid to LNPs formulated with PEG-lipid using the same lipid ratios, that size and PDI were bigger compared to PEG-lipid formulated LNPs and the encapsulation efficiency was 5% lower. A formulation of 4% IPG-lipid showed the properties closest to the control, with a size of 100 nm and an encapsulation efficiency of 95%. (Table 1, Figure 22).

**Table 1: Size, PDI and encapsulation efficiency of LNPs formulated with different ratios of the respective functional lipid. Lipid ratios are noted as molar ratios in the order: LP-01E/cholesterol/DSPC/functional lipid.**

| No. | Functional lipid | Formulation | Size [nm] | PDI | EE [%] |
|---|---|---|---|---|---|
| 1 | 3 | 50/40/9/1 | 216.5 | 0.081 | 88.2 |
| 2 | 3 | 50/39/9/2 | 168.8 | 0.040 | 90.0 |
| 3 | 3 | 50/38/9/3 | 125.7 | 0.212 | 88.6 |
| 4 | 3 | 50/37/9/4 | 100.1 | 0.081 | 95.2 |
| 5 | 3 | 50/36/9/5 | 87.18 | 0.089 | 96.9 |
| 6 | 3 | 50/35/9/6 | 72.66 | 0.092 | 97.9 |
| 7 | 3 | 50/34/9/7 | 71.23 | 0.093 | 97.9 |
| 8 | 3 | 50/33/9/8 | 70.40 | 0.174 | 95.6 |
| 9 | 3 | 50/32/9/9 | 70.67 | 0.169 | 94.5 |
| 10 | 3 | 50/31/9/10 | 70.23 | 0.288 | 90.4 |
| 11 | DMG-PEG2000 | 50/38/9/3 | 109.5 | 0.183 | 93.2 |

### Cell tests of LNPs

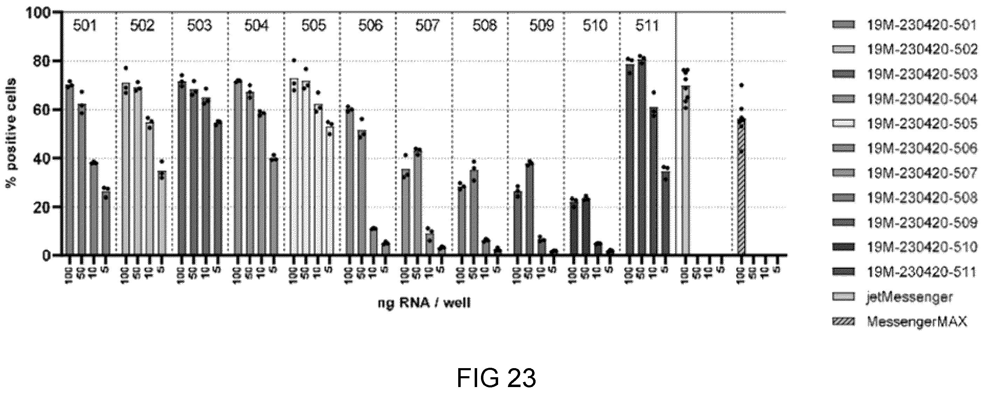

The LNP formulations encapsulating eGFP-mRNA were tested on HEP-G2 cells. After successful cellular uptake and expression of eGFP, the positive cell showed green fluorescence. The percentage of cells showing fluorescence (i.e., positive cells) is depicted in Figure 23. In this context, Figure 23 shows the results of experiments conducted with LNP formulations using 1 mol% IPG-lipid 3 (formulation 501) up to 10 mol% (formulation 510) (i.e., formulation 502 has a concentration of 2 mol%, formulation 503 has a concentration of 3 mol% etc.). LNPs with 3 mol% DMG-PEG (511) was used as a reference. Formulations with up to 5 mol% IPG-lipid (formulation 505, in Figure 23) showed no decrease of expressed protein compared to the PEG standard formulation (formulation 511, Figure 23), indicating that IPG had no influence on the cellular uptake and biological activity.

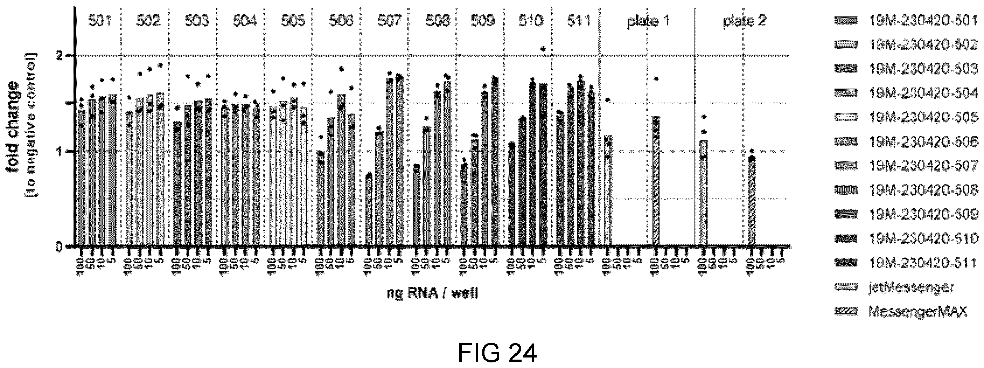

To examine the cell viability after treatment with RNA encapsulating LNPs, ATP and LDH values were determined for the different formulations also used for the experiments the results of which are depicted in Figure 23 (cf. Figure 24). ATP levels were assessed in vitro via a bioluminescent ATP assay. Higher ATP values indicate a higher number of living cells. Formulations with up to 5 mol% IPG-lipid show no decrease in cell viability compared to the PEG-lipid employing control.

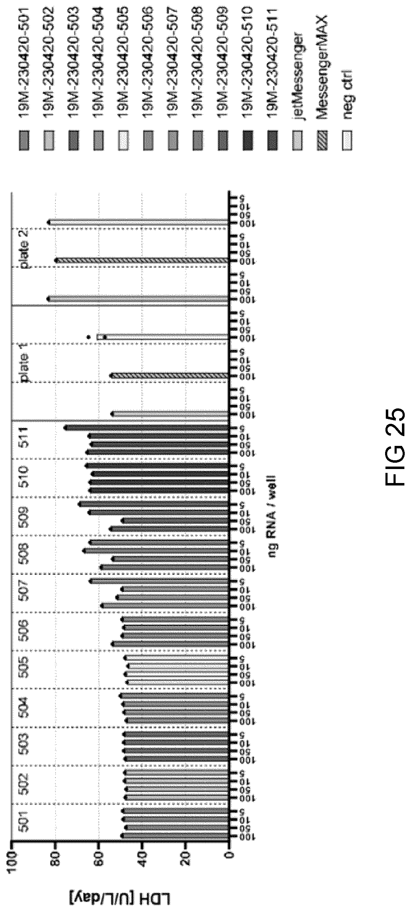

Cell damage was assessed using an lactate dehydrogenase (LDH) assay (cf. Figure 25). The amount of released LDH, a cytosolic enzyme, is an indicator for the number of lysed cells.

Released LDH reduces tetrazolium salt (INT) to tetrazan, which is a red dye. The amount of red dye is proportional to LDH. Formulations with more than 5 mol% IPG-lipid showed increased LDH levels, indicating cytotoxicity above this amount of polymer lipid.

### Materials and Methods

**Nuclear Magnetic Resonance (NMR):** ¹H NMR and ¹³C NMR spectra were recorded either on a Bruker AVANCE III 500 (Bruker Corporation), or a Jeol ECZ600 S (JEOL GmbH), or a Bruker AVANCE III 700 (Bruker Corporation). Chemical shifts are reported in δ (ppm) and referenced to the respective deuterated solvents.

**Dynamic Light Scattering (DLS):** DLS studies were done with a Malvern Zetasizer Ultra (Malvern Instruments Limited, U.K.) equipped with a 10 mW He-Ne laser operating at a wavelength of 632.8 nm. The scattered light was detected using the back-scattering setting at an angle of 173° (NIBS, noninvasive backscatter). The measurements were carried out in 12 mm square glass cuvettes (Hellma Analytics) at 25 °C and 37 °C. All samples were calibrated for 2 minutes at the respective temperature before measuring. A series of three measurements were performed to check the reproducibility of the experiments.

**Dialysis:** All polymers were purified by dialysis using pre-treated RC Tubing (MWCO 1 kDa or 3.5 kDa) Spectra/Por 7 ^{®} dialysis membranes.

**Gel Permeation Chromatography (GPC):** Molecular weight and polydispersity of the polymers were determined by a Waters 1515 gel permeation chromatography (GPC) instrument equipped with two linear PLgel columns (Mixed-C) following a guard column and a differential refractive index detector. The measurements were performed using THF for hydrophobic compounds or water for hydrophilic compounds as the eluent at a flow rate of 1.0 mL/min at 30 °C and a series of narrow polystyrene standards (THF), Pullulan (water) for the calibration of the columns.

**Cell Viability Tests (CCK-8 Assay, Dulbecco's Modified Eagle's Medium (DMEM), 48 h) of lipids:** Cells were seeded in a transparent 96-well plate with a density of 10 000 cells per well and cultured for 24 h. The medium (DMEM) was removed and replaced with a medium containing the respective compound, followed by 48 h of incubation. Subsequently, 10 µL of premixed Cell Counting Kit-8 (CCK-8) solution (Dojindo Molecular Technologies, Inc., Rockville), containing the proprietary WST-8 tetrazolium salt, was added to each well. Viable cells reduce this salt to a formazan dye whose absorbance can be measured in the medium.

The absorbance was measured at 450 nm using a Tecan Infinite 200 Pro microplate reader after 2 h. Three independent experimental runs with triplicates were performed (n = 3).

**LNP formulation and properties:** LNPs were formulated by using a microfluidic mixing method. LNP size and PDI was determined by DLS studies, conducted at 25 °C.

**Determination of encapsulation efficiency (ee%):** The encapsulation efficiency is calculated by determining the amount of RNA, using a Ribogreen assay from ThermoFisher (RediPlate 96 RiboGreen Quantitation Kit). Encapsulated RNA doesn't bind to Ribogreen, therefore measuring the fluorescence gives the amount of unencapsulated RNA. Triton X-100 is used to disrupt the LNPs and release the mRNA, giving the total amount of RNA.

### Cytotoxicity of LNPs was tested using CytoTox 96^{®} Non-Radioactive Cytotoxicity Assay

### General procedure: Synthesis of α-azido-ω-hydroxy-poly([(1-Ethoxyethoxy)methyl]oxirane)

In a flame dried Schlenk flask NBu₄N₃ (1 eq, 2.58 - 3.87 mmol) was heated up to 100 °C *in vacuo.* The molten salt was flushed with argon three times and then kept stirring for 2 h. The salt was dissolved in dry toluene (2 M concentration of the glycidyl monomer) using an ultrasonic bath. The solution was cooled down to 0°C and then the monomer [(1-Ethoxyethoxy)methyl]oxirane (EEGE) (n eq, n= 10-200) was added slowly. After letting the solution cool down to 0 °C again, triisobutylaluminium (5 - 10 eq) was added at once under vigorous stirring. The reaction was let to come to r.t. and kept stirring overnight. The reaction was quenched at 0 °C by adding H₂O (25 - 50 eq). The organic phase was separated from the aqueous phase with a separation funnel and dried over Na₂SO₄, the solvent was removed under reduced pressure and the colorless viscous liquid was dialyzed in MeOH (1 kDa MWCO).

### General procedure: Synthesis of α-azido-ω-hydroxy-poly(glycidyl methyl ether)

In a flame dried Schlenk flask tetrabutylammonium azide (1 eq, 2.58 mmol) was heated at 100 °C under vacuum for 3 h, and then purged with argon three times. The molten salt was dissolved in dry toluene (2 M concentration of glycidyl methyl ether) by ultrasonication. The solution was cooled down to 0 °C and glycidyl methyl ether (n eq, n= 10-200) was added slowly. After letting the solution cool down to 0 °C again triisobutylaluminium in toluene (5 - 10 eq) was added at once under vigorous stirring at the cooled temperature. The reaction mixture was allowed to warm up to room temperature overnight. The reaction was quenched at 0 °C by addition of water (25 - 50 eq), and the solvent was then removed under reduced pressure. The resulting residue was subsequently dissolved in methanol and dialyzed against methanol for one day using a 1 kDa MWCO RC tubing.

### General procedure: Deprotection of poly(ethoxy ethyl glycidyl ether)

To the PEEGE based compound a solution of oxalic acid (2 eq per repeating unit n of EEGE, 0.15 g/mL) in a mixture of acetone and H₂O (3:1 w/w) was added dropwise under fast stirring at room temperature. After 3.5 hours the reaction was diluted with deionized H₂O and dialyzed against deionized H₂O (1 kDa MWCO, RC tubing) until a pH of 6 to 7 was reached. The solvent was removed under reduced pressure, affording the respective product.

### General procedure: Azide reduction with (tris(2-carboxyethyl)phosphine) (TCEP) (for allyl containing compounds)

Tris(2-carboxyethyl)phosphine (TCEP) (1.6 eq) was dissolved in a 4:1 mixture of THF/H₂O in a round-bottom flask. Subsequently the respective azide functional polymer (1.0 eq) was added dropwise under stirring. The reaction was stirred at room temperature overnight. The reaction mixture was then dialyzed against MeOH/H₂O (1:1, 1kDa MWCO, RC tubing) for 2 days. The solvent was removed in vacuo, giving the product.

### General Procedure: Synthesis of H₂N-PEEGE-S-lipid

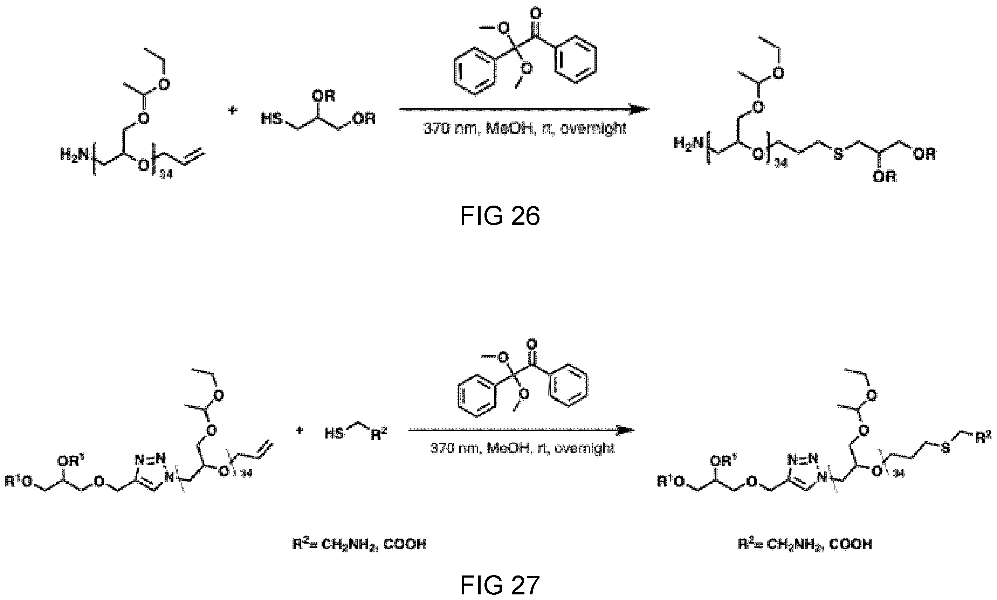

In a 50 mL Schlenk flask H₂N-PEEGE-allyl (200 mg, 1.0 eq) was dissolved in 20 mL MeOH and purged with argon for 10 min. Subsequently the respective thiol (3.0 eq) was added and the solution was purged with argon for another 10 min. Next, catalytic amounts of DMPA were added and the reaction was stirred at room temperature and UV irradiation (370 nm, 100% Intensity) overnight (cf. Figure 26). The reaction was dialyzed in MeOH (1 kDa RC tubing) for 2 days. The solvent was removed in vacuo, giving the product as a viscous liquid.

### General Procedure: thiol-ene click reaction of lipid-T-PEEGE-allyl

In a 50 mL Schlenk flask lipid-T-PEEGE-allyl (1.0 eq) was dissolved in 20 mL MeOH and purged with argon for 10 min. Subsequently the respective thiol such as thioglycolic acid or cysteamine (3.0 eq) was added and the solution was purged with argon for another 10 min. Next, catalytic amounts of DMPA were added and the reaction was stirred at room temperature and UV irradiation (370 nm, 100% Intensity) overnight (cf. Figure 27). The reaction was dialyzed in MeOH (1 kDa RC tubing) for 2 days. The solvent was removed in vacuo, giving the product as a viscous liquid.

### Synthesis of DMG-alkyne

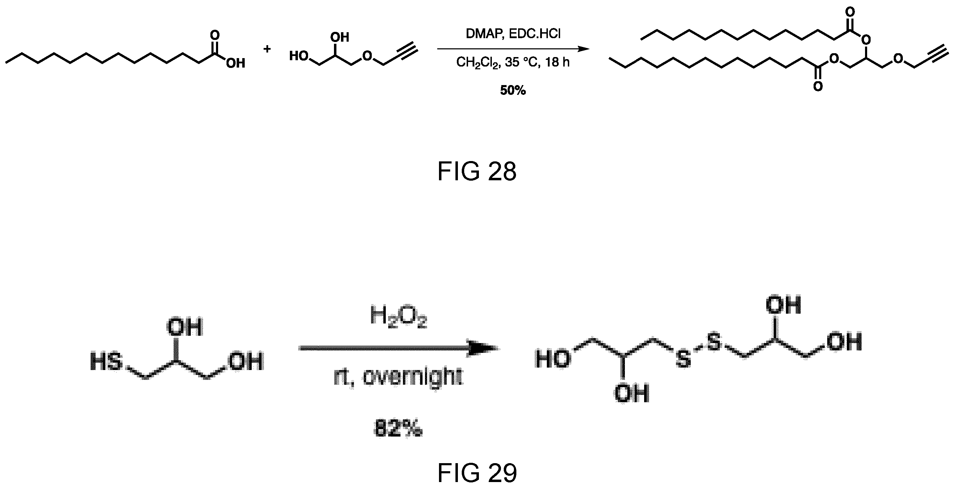

To an ice-cold and stirred solution of 3-(prop-2-yn-1-yloxy)propane-1,2-diol (1.00 g, 7.68 mmol) in CH₂Cl₂ (20 mL), EDC.HCl (3.09g, 16.1 mmol) and DMAP (1.42 g, 11.6 mmol) were added. The reaction mixture was then stirred at 30 °C for 30 min, followed by the addition of n-tetradecanoic acid (myristic acid) (4.44 g, 19.4 mmol) (cf. Figure 28). The resultant solution was stirred at 35°C for 18 h. On completion of the reaction, monitored by thin layer chromatography (TLC) (10%EtOAc in cyHex; R_{f}=0.42) the solvent was evaporated under reduced pressure. The solid was taken up in CH₂Cl₂ 200 mL) and then washed first with a mixture of 50 mL brine and 50 mL of a 0.5 M aq. HCl solution. The organic phase was washed two more time with the HCl solution (2x50 mL). Afterwards it was washed with a sat. aq. NaHCO₃ solution (3x50 mL). The organic layer was dried with MgSO₄ and the solvent was removed by rotary evaporation. The brown crude product was purified by flash column chromatography (SiO₂, 100% CH₂Cl₂, R_{f}=0.25) to give DMG-alkyne (2.12 g; 3.85 mmol; 50%) as a colorless solid.

NMR and mass spectrometric analyses gave the following results:
**¹H-NMR** (600 MHz, CDCl₃, 25 °C): *δ*=5.25-5.18 (m, 1H; CH₂C*H*), 4.38 (dd, *J=* 12.0, 3.4 Hz, 1H; (C=O)OCHH CH), 4.19 (d, *J =* 2.4 Hz, 2H; C*H*₂C≡CH), 4.15 (dd, *J =* 12.0, 6.7 Hz, 1H; (C=O)OCH*H'*CH), 3.72-3.65 (m, 2H; OC*H*₂CH), 2.88 (t, *J =* 2.4 Hz, 1H; C≡CH), 2.36-2.31 (m, 4H; (C=O)CH₂), 1.66-1.57 (m, 4H; (C=O)CH₂C*H*₂), 1.38-1.26 (m, 40H; (C=O)CH₂CH₂(C*H*₂)₁₀CH₃), 0.91 (t, *J =* 7.1 Hz, 6H; CH₃) ppm.

¹³C NMR (151 MHz, CDCl₃, 25 °C) *δ*=173.6, 173.3 (C=O), 79.2(C=CH), 75.1 (*C*≡CH), 69.9 (propargyloxy-CH₂*C*HCH₂), 68.1 (propargyloxy-*C*H₂CHCH₂), 62.7 (propargyloxy-CH₂CH*C*H₂), 58.7 (*C*H₂C≡*C*H), 34.5, 34.3 ((C=O)CH₂), 32.1 (CH₃CH₂*C*H₂), 29.8, 29.6, 29.5, 29.4, 29.3, 29.2 ((C=O)CH₂CH₂(*C*H₂)₉CH₂CH₃), 25.1, 25.0 ((C=O)CH₂*C*H₂), 22.8 (CH₂), 14.3 (CH₃) ppm.

**HRMS** (ESI) *m*/*z* calcd for C₃₄H₆₂O₅+Na⁺: 573.4495 [*M*+Na]⁺; found: 573.4476 [*M*+Na]⁺.

### Synthesis of 3,3'-disulfanediylbis(propane-1,2-diol)

In a 25 mL round-bottom flask 3-Mercapto-1,2-propanediol (5.5 mL, 64 mmol, 1.0 eq) was placed and subsequently hydrogen peroxide (30wt%, 4.0 mL, 39 mmol, 0.6 eq) was added. The reaction was the stirred at rt overnight (cf. Figure 29). After completion of the reaction as indicated by TLC (5% MeOH in DCM, staining with KMnO₄) 5 mL water and 5 mL sat. aq. NaHCO₃ solution was added to quench unreacted peroxides. The solvent was removed with a rotary evaporator, giving 7.3 g of the viscous oily colorless crude. The crude was purified using flash chromatography (5%MeOH in DCM), giving the product (5.64 g, 26.3 mmol) in 83% yield.

NMR and mass spectrometric analyses gave the following results:
**¹H-NMR** (600 MHz, [D₄]MeOD, 25 °C) δ 3.87 (dq, J = 7.4, 5.1 Hz, 2H, H-1'), 3.62 - 3.53 (m, 4H, H-1", H-2-), 2.95 (dd, J = 13.6, 5.1 Hz, 2H, H-3'), 2.77 (ddd, J = 13.6, 7.4, 4.2 Hz, 2H, H-3") ppm.

**¹³C NMR** (151 MHz, [D₄]MeOD, 25 °C) *δ*= 71.9, 65.9, 43.5 ppm.

**HRMS** (ESI) *m*/*z* calcd for C₆H₁₄NaO₄S₂+Na⁺: 237.0231 [*M*+Na]⁺; found: 237.0203 [*M*+Na]⁺.

### Synthesis of DMG-disulfide

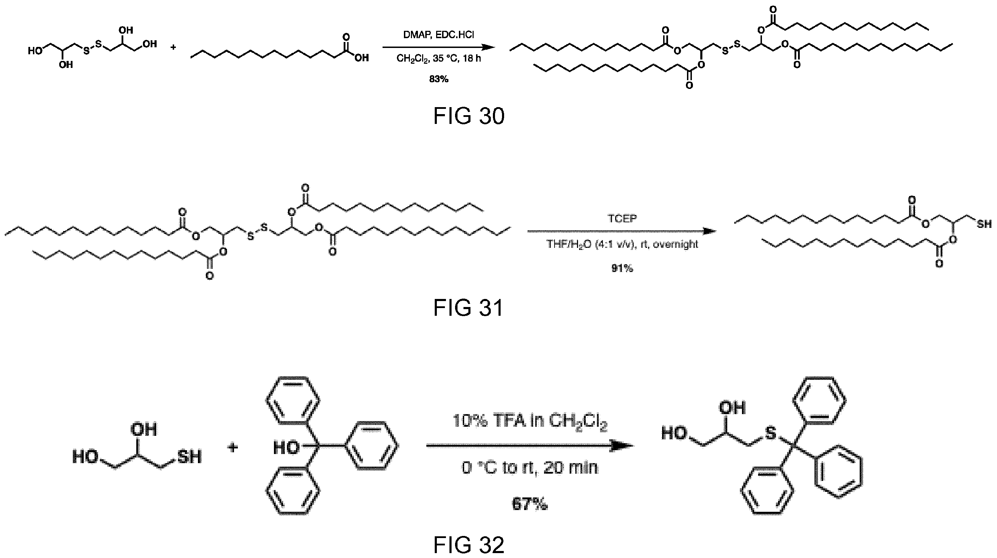

To an ice-cold and stirred solution of 3,3'-disulfanediylbis(propane-1,2-diol) (0.506 g, 2.36 mmol, 1.0 eq) in 50 mL CH₂Cl₂, EDC.HCl (1.99 g, 10.4 mmol, 4.4 eq) and DMAP (721 mg, 5.90 mmol, 2.5 eq) were added. The reaction mixture was then stirred at 30 °C for 30 min, followed by the addition of n-tetradecanoic acid (2.37 g, 10.4 mmol, 4.4 eq). The resultant solution was stirred under reflux at 35 °C overnight (cf. Figure 30).

After completion of the reaction as indicated by TLC the reaction was diluted with CH₂Cl₂ and washed first with a mixture of brine (3x20mL) and 0.5 M aq. HCl solution (3x15 mL). Afterwards it was washed with saturated aq. NaHCO₃-solution (3x20mL). The solvent was removed under reduced pressure. The crude product was placed in a 50 mL round-bottom flask and dispersed in 10 mL MeOH. The mixture was stirred under reflux at 80 °C for 5 min and then allowed to cool down. The supernatant was removed and the solid (solid at rt= 16°C) was washed with MeOH. Next, the crude was further purified via flash chromatography (CH₂Cl₂ 100%→CH₂Cl₂/MeOH 95:5), giving the pure product (2.07 g, 1.96 mmol) in 83% yield.

NMR analysis gave the following results:
**¹H-NMR** (600 MHz, CDCl₃, 25 °C): *δ*= 5.33 - 5.25 (m, 2H, H-2), 4.37 (dt, *J =* 12.0, 3.6 Hz, 2H, H-1'), 4.16 (dt, *J* = 11.7, 5.6 Hz, 2H, H-1"), 2.98 - 2.87 (m, 4H, H-3), 2.34 - 2.28 (m, 8H, (C=O)CH₂), 1.61 (h, *J =* 6.6 Hz, 8H, (C=O)CH₂C*H*₂), 1.25 (s, 80H, (C=O)CH₂CH₂(C*H*₂)₁₀CH₃), 0.88 (t, *J* = 7.0 Hz, 12H, CH₃) ppm.

**¹³C** NMR (151 MHz, CDCl₃, 25 °C) *δ*= 173.5, 173.1 (C=O), 69.8, 69.7, 63.7, 63.6, 39.4 (C-3), 34.4, 34.2 (C=O)*C*H₂, 32.1 (CH₃CH₂*C*H₂), 29.9, 29.8, 29.8, 29.7, 29.5, 29.3 ((C=O)CH₂CH₂(*C*H₂)₉CH₂CH₃), 25.0 ((C=O)CH₂*C*H₂), 22.8 (CH₂), 14.3 (CH₃) ppm.

### Synthesis of DMG-SH

In a 25 mL round-bottom flask TCEP (82 mg, 0.28 mmol, 1.6 eq) was dissolved in 10 mL of a 4:1 mixture of THF/H₂O. Subsequently DMG-disulfide (190 mg, 0.180 mmol, 1.0 eq) was added, the flask was sealed with a septum and the solution was left to stir overnight at room temperature (cf. Figure 31). THF was removed under reduced pressure and to the solution 10 mL H₂O wase added and the solution was extracted with CH₂Cl₂ (4x35 mL). The organic phase was dried over MgSO₄, and the solvent was removed with a rotary evaporator, giving the product (173 mg, 0.327 mmol) as colorless viscous liquid in 91% yield.

NMR analysis gave the following results:
**¹H-NMR** (600 MHz, CDCl₃,25 °C): *δ*= 5.08 (qd, *J* = 6.0*,* 3.9 Hz, 1H, H-2), 4.34 (dd, *J* = 11.9, 3.9 Hz, 1H, H-1'), 4.23 (dd, *J =* 11.9, 5.7 Hz, 1H, H-1"), 2.79 - 2.68 (m, 2H, H-3), 2.37 - 2.28 (m, 4H, (C=O)CH₂), 1.62 (dp, *J* = 9.6, 7.4 Hz, 4H, (C=O)CH₂ C*H*₂), 1.25 (s, 40H, (C=O)CH₂CH₂(C*H*₂)₁₀CH₃), 0.88 (t, *J =* 7.0 Hz, 6H, CH₃) ppm.

**¹³C NMR** (151 MHz, CDCl₃, 25 °C) *δ*= 173.5, 173.1 (C=O), 72.2, 63.1, 34.4, 34.3 (C=O)*C*H₂, 32.1 (CH₃CH₂*C*H₂), 29.8, 29.8, 29.8, 29.6, 29.5, 29.4, 29.3 ((C=O)CH₂CH₂(*C*H₂)₉CH₂CH₃, C-3), 25.1, 25.0 ((C=O)CH₂*C*H₂), 22.8 (CH₂), 14.3 (CH₃) ppm.

### Synthesis of 3-(tritylthio)propane-1,2-diol

To a solution of 3-mercaptopropane-1,2-diol (4.0 mL, 46 mmol, 2.0 eq) in 50 mL of a 10% TFA (4.5 mL, 58 mmol, 2.5 eq) solution in CH₂Cl₂, triphenylmethanol (6.1 g, 23 mmol, 1.0 eq) was added at 0 °C. The mixture was stirred at room temperature for 20 min until the color changed to yellow (cf. Figure 32). Subsequently, the solvent and TFA were removed under high vacuum using a cooling trap. Purification by flash chromatography (CyH/EA, 1:0→1:1) gave the pure product (5.50 g, 15.7 mmol, 67% yield) as a colorless solid.

NMR and mass spectrometric analyses gave the following results:
**¹H-NMR** (500 MHz, [D₄]-MeOD,25 °C): *δ*= 7.43 - 7.39 (m, 6H, CH_{Ar}), 7.31 - 7.26 (m, 6H, CH_{Ar}), 7.23 - 7.18 (m, 3H, CH_{Ar}), 3.45 - 3.39 (m, 1H, CH), 3.38 - 3.32 (m, 2H, CH₂), 2.31 (dd, *J* = 6.4, 3.0 Hz, 2H, CH₂S) ppm.

**¹³C NMR** (151 MHz, CDCl₃, 25 °C) *δ*= 146.3, 130.8, 128.9, 127.8, 72.3, 67.7, 66.4, 36.6 ppm.

**HRMS** (ESI) *m*/*z* calcd for C₂₂H₂₂O₂S+Na⁺: 373.1238 [*M*+Na]⁺; 389.0978 [*M*+K]⁺; found: 373.1285 [*M*+Na]⁺, 389.1025 [*M*+K]⁺.

### Synthesis of BisC14-STrt

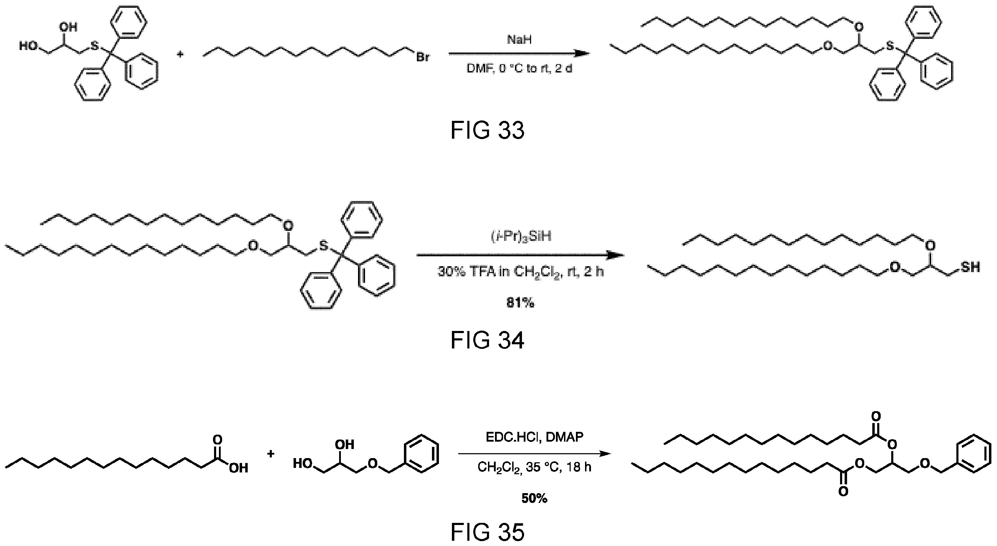

The synthesis of BisC14-STrt followed a procedure adapted from Rao and Liu ^{[54]}. 3-(tritylthio)propane-1,2-diol (100 mg, 0.285 mmol, 1.0 eq) was dissolved in 8 mL dry DMF. The flask was cooled with an ice bath and NaH (60wt%, 68 mg, 2.9 mmol, 10 eq) was slowly added. The mixture was allowed to warm up to room temperature and subsequently tetradecyl bromide (0.53 mL, 1.7 mmol, 6.0 eq) was added dropwise. The reaction was stirred at 40 °C for 2 days (cf. Figure 33). The reaction was slowly quenched with H₂O while cooling in an ice bath. The reaction was then diluted with CH₂Cl₂ and brine and extracted with CH₂Cl₂ (3x30 mL). The organic solvent was removed in vacuo. The crude was purified by flash chromatography (CyH 100% → 40% CH₂Cl₂ in CyH, NP40g), giving the pure product (100 mg, 0.135 mmol) as a colorless liquid/wax in 47% yield.

NMR analysis gave the following results:
**¹H-NMR** (600 MHz, CDCl₃, 25 °C): *δ*= 7.43 - 7.39 (m, 6H), 7.26 (dd, *J =* 8.5, 7.0 Hz, 6H), 7.22 -7.17 (m, 3H), 3.37 - 3.30 (m, 4H), 3.26 (t, *J =* 6.8 Hz, 2H), 3.19 - 3.12 (m, 1H), 1.46 (dq, *J* = 25.1, 6.7 Hz, 4H), 1.25 (d, *J* = 6.8 Hz, 44H), 0.87 (t, *J* = 7.0 Hz, 6H) ppm.

**¹³C NMR** (151 MHz, CDCl₃, 25 °C) *δ*= 145.0, 130.0, 129.9, 129.8, 128.3, 128.0, 127.7, 127.5, 127.1, 126.7, 126.4, 77.8, 72.3, 71.7, 70.3, 66.8, 33.5, 32.1, 30.1, 29.9, 29.8, 29.7, 29.5, 26.2, 26.2, 22.8, 14.3 ppm.

### Synthesis of 2,3-bis(tetradecyloxy)propane-1-thiol (BisC14-SH)

The synthesis of BisC14-SH also followed a procedure adapted from Rao and Liu ^{[54]}. In a 50 mL round-bottom flask, BisC14-STrt (880mg, 1.18 mmol, 1.0 eq) was dissolved in 30% TFA in CH₂Cl₂ (17 mL). Triisopropylsilane (55 µL, 0.27 mmol, 2.3 eq) was added and the mixture was stirred at rt for 2 hours (cf. Figure 34). The solvent was removed and then diluted again with CH₂Cl₂ and 0.5 M aq HCl solution and washed with 0.5 M aq. HCl (3x40 mL). The organic phase was dried over MgSO₄ and the solvent was removed with a rotary evaporator. The crude was purified through flash chromatography (CyH 100% → 40% CH₂Cl₂ in CyH, NP40g), giving the product (482 mg, 0.962 mmol) in 81% yield.

NMR and mass spectrometric analyses gave the following results:
**¹H-NMR** (600 MHz, CDCl₃, 25 °C): *δ*= 3.58 - 3.46 (m, 5H), 3.44 (t, *J* = 6.6 Hz, 2H), 2.78 - 2.69 (m, 1H), 2.64 (ddd, J = 13.7, 7.8, 5.9 Hz, 1H), 1.63 - 1.55 (m, 4H), 1.26 (s, 44H), 0.88 (t, J = 7.0 Hz, 6H) ppm.

**¹³C NMR** (151 MHz, CDCl₃, 25 °C) *δ*= 79.5, 77.7, 71.9, 71.2, 70.6, 41.3, 32.1, 30.2, 29.9, 29.8, 29.7, 29.6, 29.5, 26.3, 22.8, 14.3 ppm.

**HRMS** (ESI) *m*/*z* calcd for C₃₁H₆₄O₂S+Na⁺: 523.4525 [*M*+Na]⁺; 539.4264 [*M*+K]⁺; found: 523.4499 [*M*+Na]⁺, 539.4239 [*M*+K]⁺.

### Synthesis of 1,2-dimyristoyl-3-O-benzyl-rac-glycerol (DMG-Bn)

To an ice cold and stirring solution of 3-benzyloxy-1,2-propanediol (1.50 g, 8.23 mmol) in 20 mL CH₂Cl₂, EDC.HCl (3.35 g, 17.5 mmol) and DMAP (1.52 g, 12.4 mmol) were added. The reaction mixture was then stirred at 30 °C for 30 min, followed by the addition of n-tetradecanoic acid (4.76 g, 20.8 mmol). The resultant solution was stirred at 35°C (cf. Figure 35). After 18 h the reaction was left to cool down to ambient temperature and was subsequently diluted with CH₂Cl₂ (50 mL). The organic phase was first washed with a 1:1 mixture of Brine and 0.5 M aq. HCl solution (3x30 mL) and then with sat. aq. NaHCO₃ solution (3x15mL). The organic phase was dried over MgSO₄ and the solvent was removed under reduced pressure. The crude product was purified by flash column chromatography (SiO₂, 100% CH₂Cl₂) giving DMG-Bn (2.50 g, 4.15 mmol, 50%) as a colorless solid.

NMR and mass spectrometric analyses gave the following results:
**¹H-NMR** (600 MHz, CDCl₃, 25°C): *δ*=7.38-7.26 (m, 5H; CH (ar)), 5.29-5.19 (m, 1H; H-2), 4.54 (q, *J =* 12 Hz, 2H; CH₂ (bzl)), 4.34 (dd, *J* = 11.9, 3.8 Hz, 1H; H'-1), 4.19 (dd, *J =* 11.9, 6.5 Hz, 1H; H"-1), 3.59 (dd, *J* = 5.2, 2.5 Hz, 2H; H-3), 2.34-2.25 (m, 4H; (C=O)C*H*₂), 1.67-1.53 (m, 4H; (C=O)CH₂C*H*₂), 1.33-1.23 (m, 40H; (C=O)CH₂CH₂(C*H*₂)₁₀CH₃), 0.88 (t, *J* = 7.1 Hz, 5H; CH₃) ppm.
**¹³C NMR** (151 MHz, CDCl₃, 25°C) *δ*=173.6, 173.3 (C=O), 137.9, 128.6, 127.9, 127.8 (Cₐᵣ), 73.5, 70.2, 68.4, 62.8 (C-1, C-2, C-3, CH₂ (bzl)), 34.5, 34.3 ((C=O)CH₂), 32.1 (CH₃CH₂*C*H₂), 29.8, 29.8, 29.8, 29.6, 29.5, 29.4, 29.3, 29.2 ((C=O)CH₂CH₂(*C*H₂)₉CH₂CH₃), 25.1, 25.0 ((C=O)CH₂*C*H₂), 22.8 (CH₂), 14.3 (CH₃) ppm.

**HRMS** (ESI) *m*/*z* calcd for C₃₈H₆₆O₅+Na⁺ and C₃₈H₆₆O₅+K⁺: 625.4808 [*M*+Na]⁺, 641.5547 [*M*+K]⁺; found: 625.4917 [*M*+Na]⁺ and 641.4668 [*M*+K]⁺.

### Synthesis of 1,2-dimyristoyl-rac-glycerol (DMG-OH)

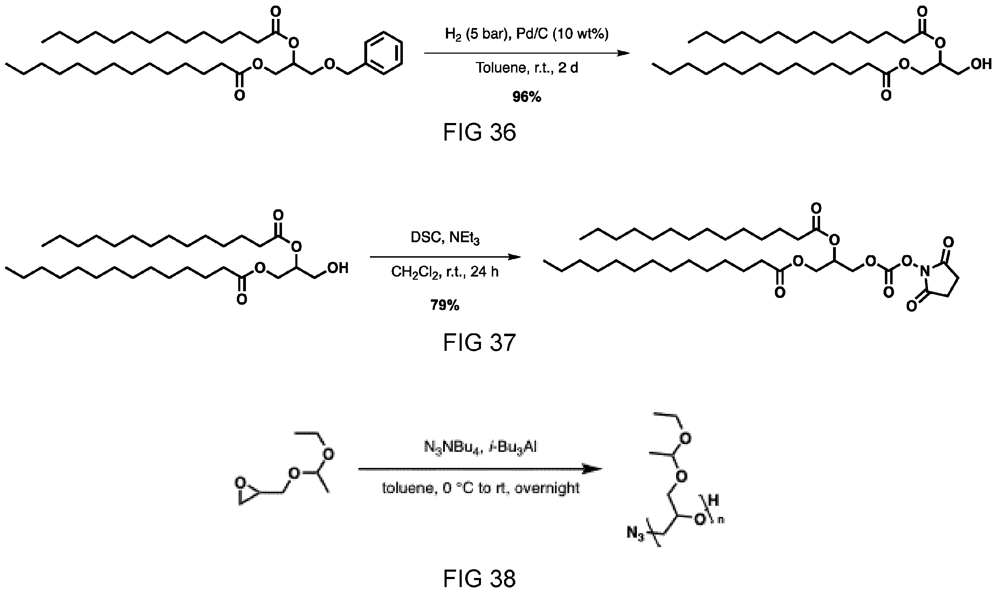

In a 5 mL glass vial DMG-Bn (1.00 g, 1.66 mmol) was dissolved in 1 mL toluene and 10wt% Pd/C cat. (0.1g) were added. The reaction was stirred under a hydrogen atmosphere in a H₂-reactor at 5-6 bar for 48 h (cf. Figure 36). The catalyst was filtered off with a filter paper and the solvent removed under reduced pressure, providing 1,2-Dimyristoyl-*rac*-glycerol (0.813 g, 1.59 mmol, 96%) as a colorless solid. The product was not further purified.

NMR and mass spectrometric analyses gave the following results:
**¹H-NMR** (700 MHz, CDCl₃, 27°C): *δ*=5.08 (p, *J* = 4.9 Hz, 1H; H-2), 4.32 (dd, *J =* 12.0, 4.5 Hz, 1H; H'-1), 4.24 (dd, *J* = 11.9, 5.7 Hz, 1H, H"-1), 3.75-3.71 (m, 2H; H-3), 2.37-2.29 (m, 4H; (C=O)C*H*₂), 1.66 - 1.58 (m, 4H; (C=O)CH₂C*H*₂), 1.35 - 1.23 (m, 40H; (C=O)CH₂CH₂(C*H*₂)₁₀CH₃), 0.88 (t, *J* = 7.1 Hz, 6H; CH₃) ppm.

**¹³C-NMR** (176 MHz, CDCl₃, 27°C) *δ*=173.9, 173.6 (C=O), 72.3, 62.1, 61.7 (C-1, C-2, C-3), 34.4, 34.3 ((C=O)CH₂), 32.1 (CH₃CH₂*C*H₂), 29.8, 29.8, 29.8, 29.6, 29.5, 29.4, 29.3, 29.2 ((C=O)CH₂CH₂(*C*H₂)₉CH₂CH₃), 25.1, 25.0 ((C=O)CH₂*C*H₂), 22.8 (CH₂), 14.3 (CH₃) ppm.

**HRMS** (ESI) *m*/*z* calcd for C₃₁H₆₀O₅+Na⁺ and C₃₁H₆₀O₅+K⁺: 535.4338 [*M*+Na]⁺, 551.4078 [*M*+K]⁺; found: 535.4332 [*M*+Na]⁺ and 551.4074 [*M*+K]⁺.

### Synthesis of DMG-succinimidyl carbonate (DMG-SC)

The synthesis procedure was slightly adapted from a literature report ^{[74]}.

To a solution of 1,2-myristoyl-rac-glycerol (200 mg, 0.39 mmol) in CH₂Cl₂ (60 mL), disuccinimidyl carbonate (820 mg, 0.140 mmol, 98%) and triethylamine (1.0 mL, 7.8 mmol) were added at 0 °C. The solution was then stirred at ambient temperature 24 h (cf. Figure 37). The organic layer was washed with deionized H₂O (3x20 mL), dried over MgSO₄, and the solvent was removed under reduced pressure. The crude product was further purified by flash column chromatography (SiO₂, 100% EtOAc) giving DMG-succinimidyl-carbonate (201 mg, 0.307 mmol, 79%) as a colorless solid.

NMR and mass spectrometric analyses gave the following results:
**¹H-NMR** (600 MHz, (CD₃)₂CO, 25°C) *δ*=5.38 (tt, *J =* 6.1, 4.0 Hz, 1H; H-2), 4.63 (dd, *J =* 11.7, 3.5 Hz, 1H; H'-1), 4.59 (dd, *J* = 11.7, 6.2 Hz, 1H; H"-1), 4.41 (dd, *J* = 12.0, 4.2 Hz, 1H; H'-3), 4.23 (dd, *J* = 12.0, 6.2 Hz, 1H; H"-3), 2.89 (s, 4H; CH_{2,Succin.}), 2.40-2.30 (m, 4H; (C=O)CH₂), 1.66-1.56 (h, *J* = 7.4 Hz, 4H; (C=O)CH₂C*H*₂), 1.29 (s, 40H; (C=O)CH₂CH₂(C*H*₂)₁₀CH₃), 0.92-0.85 (t, *J* = 7.0 Hz, 6H; CH₃) ppm.

**¹³C-NMR** (151 MHz, (CD₃)₂CO, 25°C) *δ*= 173.4, 173.1 ((C=O)ₑₛₜₑᵣ), 170.0 (C=O)_{imide}, 152.5 (C=O)_{carbonate}, 130.3129.5, 69.7, 69.3, 62.3, 34.5, 34.4 ((C=O)CH₂), 32.7 (CH₃CH₂C*H*₂), 30.4, 30.4, 30.3, 30.3, 29.8 ((C=O)CH₂CH₂(*C*H₂)₉CH₂CH₃) , 26.2, 25.6, 25.5 ((C=O)CH₂*C*H₂), 23.3 (CH₃*C*H₂), 14.4 (CH₃) ppm.

**HRMS** (ESI) *m*/*z* calcd for C₃₆H₆₃NO₉+Na⁺ and C₃₆H₆₃NO₉+K⁺: 676.4401 [*M*+Na]⁺, 692.4140 [*M*+K]⁺; found: 676.4379 [*M*+Na]⁺ and 692.4133 [*M*+K]⁺.

### Synthesis of α-azido-ω-hydroxy-PEEGE4500 (N₃-PEEGE-OH_4.5kD)

α-azido-ω-hydroxy-poly(EEGE) was prepared according to the general procedure explained above in the section entitled "Synthesis of *α*-azido-*ω*-hydroxy-poly([(1-Ethoxyethoxy)methyl]oxirane)". The educts were used in the following amounts/equivalents: tetrabutylammonium azide: 1.10 g, 3.87 mmol, 1.0 eq; [(1-Ethoxyethoxy)methyl]oxirane (EEGE): 16.0 mL, 108 mmol, 28 eq; triisobutylaluminium: 17.6 mL, 19.3 mmol, 5.0 eq.

The mono azido functional poly glycidyl α-azido-ω-hydroxy-PEEGE4500 was afforded in 50% yield.

NMR, chromatographic, and infrared (IR) analyses gave the following results:
**¹H-NMR** (700 MHz, (CD₃)₂CO, 27°C): *δ*=4.76-4.67 (m, 1H; CH₃-C*H*), 3.75-3.41 (m, 7H), 1.26 (d, *J* = 5.3 Hz, 3H; C*H*₃-CH), 1.17 (t, *J* = 7.0 Hz, 3H; CH₃) ppm.

**¹³C NMR** (176 MHz, (CD₃)₂CO, 27°C) *δ*=100.6, 100.5, 80.0, 80.0, 79.9, 79.8, 71.1, 70.9, 70.8, 66.0, 61.3, 61.2, 61.2, 20.4, 15.9 ppm.

**GPC:** Mₙ=4.5 kDa, PDI=1.4
**IR:** singlet at 2100 wavenumbers (specific for azide)

### Synthesis of α-azido-ω-hydroxy-PEEGE7500 (N₃-PEEGE-OH_7.5kD)

α-azido-ω-hydroxy-poly(EEGE) was prepared according to the general procedure explained above in the section entitled "Synthesis of *α*-azido-*ω*-hydroxy-poly([(1-Ethoxyethoxy)methyl]oxirane)". The educts were used in the following amounts/equivalents: tetrabutylammonium azide: 734 mg, 2.58 mmol, 1.0 eq; [(1-Ethoxyethoxy)methyl]oxirane (EEGE): 23.0 mL, 155 mmol, 60 eq; triisobutylaluminium: 11.7 mL, 12.9 mmol, 5.0 eq.

The azido functional poly ether α-azido-w-hydroxy-PEEGE7500 (13.91 g, 1.58 mmol) was afforded in 61% yield.

NMR and chromatographic analyses gave the following results:
**¹H-NMR** (600 MHz, (CD₃)₂CO, 25°C) *δ*=4.73 (q, *J* = 5.2 Hz, 1H; C*H*CH₃), 3.74-3.44 (m, 7H; CH₂ and CH), 1.26 (d, *J =* 5.6 Hz, 3H; CHC*H*₃), 1.17 (t, *J* = 7.0 Hz, 3H; CH₃) ppm.

**¹³C-NMR** (151 MHz, (CD₃)₂CO, 25°C) *δ*=100.6, 100.5, 80.0, 79.9, 71.0, 70.9, 66.0, 61.3, 61.2, 61.2, 52.7, 30.3, 20.4, 15.8 ppm.

GPC: Mₙ=7.5kDa; PDI:1.4

### Synthesis of α-azido-ω-hydroxy-MeOIPG4000 (N₃-MeOIPG4.0kD)

α-azido-ω-hydroxy-MeOIPG4000 was prepared according to the general procedure explained above in the section entitled "Synthesis of *α*-azido-*ω*-hydroxy-poly(glycidyl methyl ether". The educts were used in the following amounts/equivalents: tetrabutylammonium azide: 0.734 g, 2.58 mmol, 1.0 eq; glycidyl methyl ether: 10.3 mL, 116 mmol, 45 eq; triisobutylaluminium: 11.7 mL, 12.9 mmol, 5.0 eq.

The mono azido functional poly glycidyl α-azido-ω-hydroxy-MeOIPG4000 was afforded in 50% yield.

NMR and chromatographic analyses gave the following results:
**¹H-NMR** (700 MHz, [D₄]MeOD, 27°C) *δ*=3.72-3.57 (m, 3H), 3.55-3.42 (m, 2H), 3.36 (s, 3H; CH₃) ppm.

**¹³C-NMR** (176 MHz, [D₄]MeOD, 27°C) *δ*=80.1, 80.0, 79.9, 79.8, 79.8, 75.1, 73.9, 73.8, 73.2, 73.0, 71.2, 71.2, 71.1, 71.1, 71.0, 70.8, 70.7, 59.5, 53.0, 52.9, 49.9 ppm.

**GPC:** Mₙ= 4.0 kD; PDI: 1.2

### Synthesis of α-azido-ω-hydroxy-MeOIPG2900 (N₃-MeOIPG2.9kD)

α-azido-ω-hydroxy-MeOIPG2900 was prepared according to the general procedure explained above in the section entitled "Synthesis of *α*-azido-*ω*-hydroxy-poly(glycidyl methyl ether". The educts were used in the following amounts/equivalents: tetrabutylammonium azide: 0.734 g, 2.58 mmol, 1.0 eq; glycidyl methyl ether: 5.0 mL, 56 mmol, 22 eq; triisobutylaluminium: 11.7 mL, 12.9 mmol, 5.0 eq.

The azido functional poly ether α-azido-ω-hydroxy-MeOIPG2900 (3.04 g, 1.53 mmol) was afforded in 59% yield.

NMR and chromatographic analyses gave the following results:
**¹H-NMR** (700 MHz, [D₄]MeOD, 27°C) *δ*=3.72-3.57 (m, 3H), 3.55-3.42 (m, 2H), 3.36 (s, 3H; CH₃) ppm.

**¹³C-NMR** (176 MHz, [D₄]MeOD, 27°C) *δ*=80.1, 80.0, 79.9, 79.8, 79.8, 75.1, 73.9, 73.8, 73.2, 73.0, 71.2, 71.2, 71.1, 71.1, 71.0, 70.8, 70.7, 59.5, 53.0, 52.9, 49.9 ppm.

**GPC:** Mₙ= 2.9kDa; PDI:1 .3

### Synthesis of α-amino-PEEGE4500 (H₂N-PEEGE-OH_4.5kD)

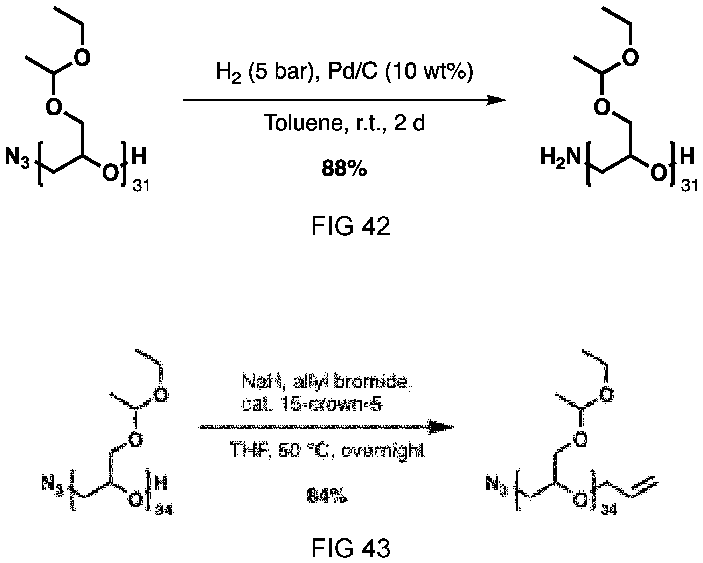

In a 10 mL glass vial the mono azido functional polymer N₃-PEEGE-OH_4.5kD (293 mg, 0.064 mmol) was dissolved in 2 mL toluene and 10 wt% of Pd/C cat. (0.029 g) were quickly added. The reaction was stirred at room temperature under a H₂ atmosphere at 5 to 6 bar for 2 days (cf. Figure 42). The catalyst was filtered off with a filter paper and the solvent was removed under reduced pressure, providing ***α***-amino-PEEGE4500 (257 mg, 0,057 mmol, 88%) as a colorless viscous liquid. FTIR showed the complete disappearance of the peak at 2100 wavenumbers, which is characteristic for the azido functionality.

NMR analysis gave the following results:
**¹H-NMR** (700 MHz, (CD₃)₂CO, 27°C) *δ*=4.73 (d, *J* = 4.8 Hz, 1H), 3.84-3.41 (m, 7H), 1.26 (d, *J* = 4.6 Hz, 3H; CH-C*H₃*), 1.17 (t, *J =* 6.9 Hz, 3H; CH₃) ppm.

**¹³C-NMR** (176 MHz, (CD₃)₂CO, 27°C) *δ*=100.6, 100.5, 79.9, 71.1, 66.0, 61.3, 61.2, 61.2, 30.3, 30.2, 30.1, 20.4, 20.3, 15.9, 15.7 ppm.

### Synthesis of N₃-PEEGE-allyl

In a 100 mL Schlenk flask N₃-PEEGE-OH_5.03kD (1.50 g, 0.299 mmol, 1.0 eq) was dried in vacuo overnight. Next, the polymer was dissolved in 39 mL anhydrous THF and subsequently NaH (60wt%, 72.0 mg, 1.80 mmol, 6.0 eq per OH) and catalytic amounts of 15-crown-5 were added, and the solution was stirred at 50 °C for 1 h. Subsequently allyl bromide (52 µL, 0.60 mmol, 2.0 eq) was added and the reaction was stirred at 50 °C overnight. (reflux condenser under Ar) (cf. Figure 43). The reaction was quenched with MeOH while cooling in an ice bath. The solvent was removed, and the crude was dialyzed against MeOH/CH₂Cl₂ (1:1, 1kDa MWCO, RC tubing) for 2 days. The solvent was removed in vacuo, giving the product as a colorless viscous oil (1.27 g, 0.251 µmol) in 84% yield.

NMR analysis gave the following results:
**¹H-NMR** (600 MHz, (CD₃)₂CO, 25°C) *δ*= 5.92 (ddt, 16.4, 10.5, 5.3 Hz, 1H, RC*H*=CH₂), 5.28 (d, 17.2 Hz, 1H, C=CHH), 5.10 (d, 10.6 Hz, 1H, C=CHH), 4.72 (s, 37H; C*H*CH₃), 4.15 (s, 2H, C*H*₂C=CH₂) 3.78-3.41 (m, 262H; CH₂ and CH), 1.26 (d, *J =* 5.6 Hz, 114H; CHC*H*₃), 1.16 (t, *J* = 7.1 Hz, 111H; CH₃) ppm.

**¹³C-NMR** (151 MHz, (CD₃)₂CO, 25°C) *δ*=136.9, 115.9, 100.5, 100.4, 80.0, 79.9, 71.0, 70.9, 66.0, 61.3, 61.2, 61.2, 52.7, 30.4, 20.3, 20.1, 15.8 ppm.

### Synthesis of DMG-T-PEEGE4500

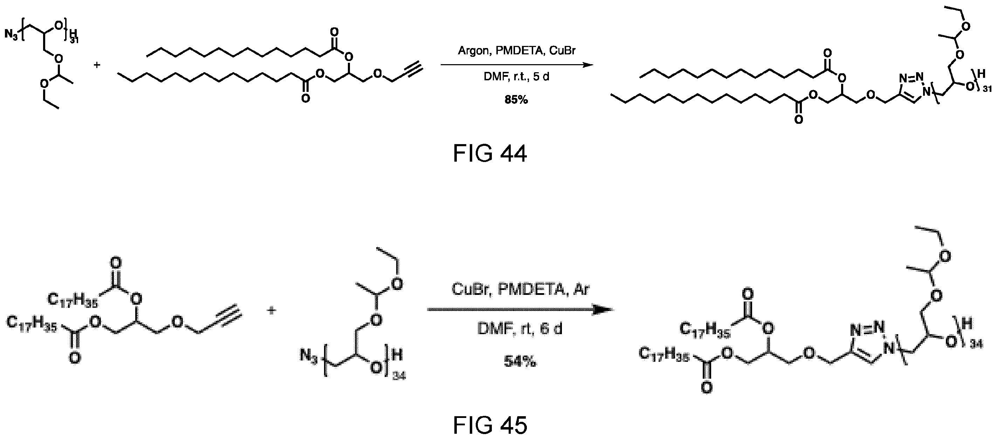

The azido functional polymer N₃-PEEGE-OH (4.5 kDa, 685 mg, 0.169 mmol) was dried under vacuum and subsequently purged with argon three times. The dry polymer was dissolved in 12 mL of anhydrous DMF and purged with argon for 30 minutes. In parallel, lipid DMG-alkyne (0.152 mg, 0.276 mmol, 1.6 eq) was dissolved in 1.5 mL of dry THF and purged with argon for 30 minutes. PMDETA (0.08 mL, 0.40 mmol, 0.2 eq) was added to the polymer solution and the mixture was further purged with argon for 5 minutes before adding CuBr (49 mg, 0.34 mmol, 2.0 eq) and the solution of DMG-alkyne. The reaction mixture was stirred at room temperature for 5 days and then exposed to air (cf. Figure 44). The mixture was diluted with THF, filtered over neutral aluminum oxide, and the solvent was removed under reduced pressure. The crude product was purified by dialyzing against acetone (1 kDa MWCO, RC tubing) for 2 days, and the solvent was removed under reduced pressure to give DMG-T-PEEGE4500 (662 mg, 0.144 mmol, 85%) as a colorless viscous oil.

NMR and chromatographic analyses gave the following results:
**¹H-NMR** (700 MHz, (CD₃)₂CO, 27°C) *δ*=7.99 - 7.95 (m, 1H; H-6), 5.24-5.17 (m, 1H; H-2), 4.73 (d, *J* = 4.7 Hz, 21H, H-11), 4.66-4.62 (m, 3H; H-7, H'-4), 4.54-4.46 (m, 1H; H"-4), 4.34 (dd, *J* = 12.0, 3.6 Hz, 1H; H'-1), 4.17-4.12 (m, 1H; H"-1), 3.75-3.41 (m, 154H; H-3, H-8- H-10, H-12), 2.31 (dt, *J* = 12.2, 7.4 Hz, 4H; H-16), 1.65-1.55 (m, 4H; H-17), 1.30 (s, 40H; H-18 - H-27), 1.26 (d, *J =* 5.3 Hz, 66H; H-13), 1.17 (t, *J =* 7.0 Hz, 66H; H-14), 0.88 (t, *J =* 7.1 Hz, 6H; H-28) ppm.

**GPC** (THF): Mₙ=5.7 kDa; PDI=1.2

### Synthesis of DSG-T-PEEGE5000

In a 100 mL Schlenk flask dried N₃-PEEGE-OH (5.03kDa, 0.415 g, 0.083 mmol, 1.0 eq) was dissolved in 15 mL DMF and 3 mL THF. Subsequently DSG-alkyne (68 mg, 0.10 mmol, 1.2 eq) and PMDETA (35 µL, 0.17 mmol, 2.0 eq) were added and the solution was purged with argon for 20 min. Subsequently CuBr (24 mg, 0.17 mmol, 2.0 eq) was added and the reaction was stirred at room temperature under Argon atmosphere for 6 days (cf. Figure 45). The reaction was exposed to air and diluted with THF. The mixture was then filtered over neutral alumina (height: 12cm, diam.: 2 cm) and subsequently the solvent was removed with a rotary evaporator. The filtered crude product was dialyzed against MeOH/DCM (1:1, 1 kDa MWCO, RC tubing). First exchange after 8 h then dialysis overnight. The solvent was removed in vacuo, giving the product as a slightly yellowish viscous liquid (255 mg, 45.0 µmol) in 54% yield.

NMR analysis gave the following results:
**¹H-NMR** (700 MHz, CDCl₃, 27°C) *δ*=7.67 (s, 1H; H-6), 5.19 (s, 1H; H-2), 4.68 (d, *J* = 5.3 Hz, 32H, H-11), 4.65-4.54 (m, 3H; H-7, H'-4), 4.47-4.39 (m, 1H; H"-4), 4.30 (ddd, J = 11.6, 3.5, 1.3 Hz, 1H; H'-1), 4.11 (ddd, *J =* 11.9, 6.6, 2.9 Hz 1H; H"-1), 3.87 (s, 2H), 3.81-3.29 (m, 267H; H-3, H-8 - H-10, H-12), 2.32-2.24 (m, 4H; H-16), 1.63-1.54 (m, 4H; H-17), 1.31-1.23 (m, 156H; H-13, H-18 - H-31), 1.18 (dd, *J =* 13.1, 7.1 Hz, 99H; H-14), 0.86 (t, *J=* 7.1 Hz, 6H; H-32) ppm.

### Synthesis of DMG-T-PEEGE7500

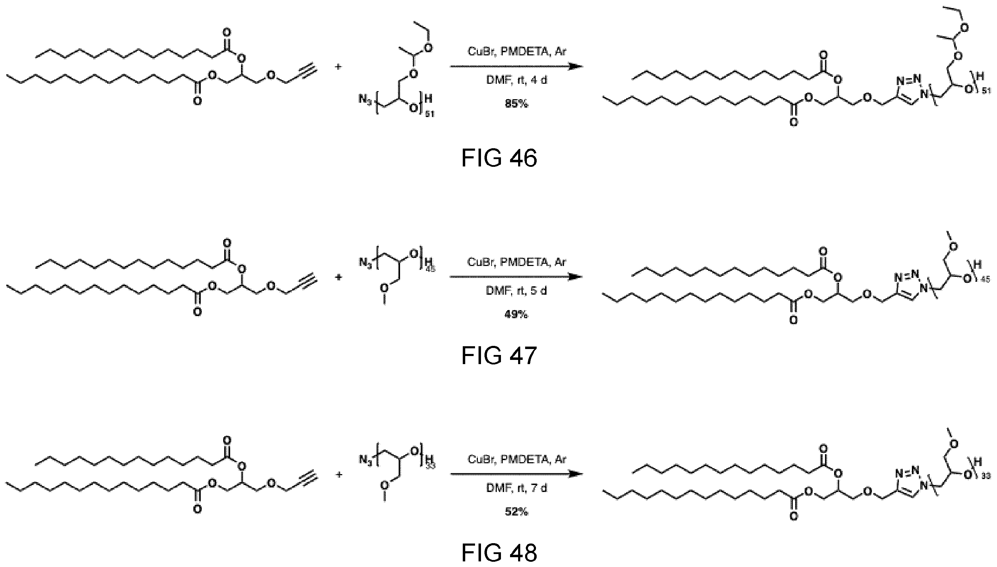

The azido functional polymer N₃-PEEGE-OH (7.5 kDa, 1.50 g, 0.170 mmol) was dried under vacuum and subsequently purged with argon three times. The dry polymer was dissolved in 15 mL of anhydrous DMF and purged with argon for 30 minutes. In parallel, lipid DMG-alkyne (0.170 mg, 0.309 mmol) was dissolved in 1.5 mL of dry THF and purged with argon for 30 minutes. PMDETA (0.07 mL, 0.34 mmol) was added to the polymer solution and the mixture was further purged with argon for 5 minutes before adding CuBr (49 mg, 0.34 mmol) and the solution of DMG-alkyne (cf. Figure 46). The reaction mixture was stirred at room temperature for 4 days and then exposed to air. The mixture was diluted with THF, filtered over neutral aluminum oxide, and the solvent was removed under reduced pressure. The crude product was purified by dialyzing against acetone (1 kDa MWCO, RC tubing) for 2 days, and the solvent was removed under reduced pressure to give DMG-T-PEEGE7500 (1.35 g, 0.144 mmol, 85%) as a colorless viscous oil.

NMR and chromatographic analyses gave the following results:
**¹H-NMR** (600 MHz, (CD₃)₂CO, 25°C) *δ*=8.00-7.95 (m, 1H; H-6), 5.24-5.17 (m, 1H; H-2), 4.76-4.69 (m, 51H; H-11), 4.68-4.57 (m, 3H; H-7, H'-4), 4.54-4.45 (m, 1H; H"-4), 4.33 (dd, *J =* 11.9, 3.6 Hz, 1H; H'-1), 4.17-4.11 (m, 1H; H"-1), 3.75-3.43 (m, 369H; H-3, H-8 - H-10, H-12), 2.30 (dt, *J* = 10.5, 7.5 Hz, 4H; H-16), 1.64 - 1.55 (m, 4H; H-17), 1.30 (s, 40H; H-18 - H-27), 1.26 (d, *J* = 4.9 Hz, 153H; H-13), 1.17 (t, *J* = 7.0 Hz, 153H; H-14), 0.88 (t, *J* = 6.9 Hz, 6H; H-28) ppm.

**¹³C-NMR** (151 MHz, (CD₃)₂CO, 25°C) *δ*=173.4, 173.1 (C=O), 144.9 (C-5), 125.4 (C-6), 100.7, 100.5, 100.4 (C-11), 80.0, 80.0, 79.9, 79.7 (C-9), 78.9, 73.1, 71.0, 70.9, 70.8, 70.6, 69.2, 69.1 (C-2, C-10), 67.5, 66.0, 66.0, 65.9, 65.3 (C-4, C-8), 64.7, 63.3 (C-1, C-3), 61.5, 61.3, 61.2, 61.2 (C-12), 59.4, 51.8 (C-7), 34.7, 34.5 (C-16), 32.7 (C-26), 30.6, 30.4, 30.4, 30.3, 30.3 (C-18 - C-25), 25.7, 25.6 (C-17), 23.3 (C-27), 20.3 (C-13), 15.8, 15.7 (C-14), 14.4 (C-28) ppm.

GPC (THF): Mₙ=9.2 kDa; PDI=1.3

### Synthesis of DMG-T-MeOIPG4000

The azido functional polymer N₃-MeOIPG-OH (4.0 kDa, 399mg, 0.099 mmol) was dried under vacuum and subsequently purged with argon three times. The dry polymer was dissolved in 12 mL of anhydrous DMF and purged with argon for 30 minutes. In parallel, DMG-alkyne (0.120 mg, 0.217 mmol) was dissolved in 1.5 mL of dry THF and purged with Ar for 30 min. PMDETA (0.08 mL, 0.40 mmol) was added to the polymer solution and the mixture was further purged with argon for 5 minutes before adding CuBr (56 mg, 0.395 mmol) and the solution of DMG-alkyne (cf. Figure 47). The reaction mixture was stirred at room temperature for 5 days and then exposed to air. The mixture was diluted with THF, filtered over neutral aluminum oxide, and the solvent was removed under reduced pressure. The crude product was purified by dialyzing against MeOH (1 kDa MWCO, RC tubing). The resulting precipitate was filtered off, and the solvent removed under reduced pressure to afford DMG-T-MeOIPG4000 (223 mg, 0.048 mmol, 49%) as a colorless viscous oil.

NMR and chromatographic analyses gave the following results:
**¹H-NMR** (700 MHz, [D₄]MeOD, 27°C) *δ*=8.02-7.99 (m, 1H; H-6), 5.24-5.19 (m, 1H; H-2), 4.68-4.59 (m, 3H; H-7, H'-4), 4.53-4.45 (m, 1H; H"-4), 4.37 (dd, *J* = 12.0, 3.2 Hz, 1H; H'-1), 4.14 (ddd, *J* = 12.0, 6.7, 1.3 Hz, 1H; H"-1), 3.95-3.89 (m, 1H), 3.87-3.82 (m, 1H), 3.73-3.33 (m, 310H; H-3, H-8 - H-10), 2.31 (dt, *J* = 12.0, 7.4 Hz, 4H;H-13), 1.66-1.55 (m, 4H; H-14), 1.38-1.25 (m, 40H; H-15 - H-24), 0.91 (t, *J* = 7.1 Hz, 6H; H-25) ppm.

**¹³C-NMR** (176 MHz, [D₄]MeOD, 27°C) *δ*=174.8, 174.8, 174.5 (C=O), 145.5, 145.5 (C-5), 126.5, 126.4 (C-6), 80.1, 80.0, 79.9, 79.8, 79.8 (C-9), 79.1, 79.0, 79.0, 76.4, 75.1, 75.1, 73.9, 73.8, 73.8, 73.7, 73.3, 73.3, 73.2, 73.0, 73.0, 73.0, 72.9, 72.7, 72.7 (C-2, C-10), 71.5, 71.4, 71.2, 71.2, 71.2, 71.1, 71.0, 70.9, 70.8, 70.8, 70.7, 69.7, 69.4 (C-1, C-4, C-8), 65.2, 63.9, 63.6, 61.3, 59.7, 59.6, 59.5, 59.5, 59.4 (C-11), 57.7, 56.1, 52.5, 52.4 (C-7), 35.2, 35.1, 35.0, 34.9 (C-13), 33.1, 32.1 (C-23), 30.8, 30.8, 30.7, 30.6, 30.5, 30.5, 30.2, 30.2, 29.5 (C-15 - C-22), 26.1, 26.1 (C-14), 23.8 (C-24), 14.5, 14.5 (C-25) ppm.

GPC (THF): Mₙ=5.2 kDa; PDI=1.1

### Synthesis of DMG-T-MeOIPG2900

The azido functional polymer N₃-MeOIPG-OH (2.9 kDa 400mg, 0.136 mmol) was dried under vacuum and subsequently purged with argon three times. The dry polymer was dissolved in 15 mL of anhydrous DMF and purged with argon for 30 minutes. In parallel, lipid DMG-alkyne (0.287 mg, 0.521 mmol) was dissolved in 1.5 mL of dry THF and purged with argon for 30 minutes. PMDETA (0.08 mL, 0.40 mmol) was added to the polymer solution and the mixture was further purged with argon for 5 minutes before adding CuBr (58 mg, 0.52 mmol) and the solution of DMG-alkyne (cf. Figure 48). The reaction mixture was stirred at room temperature for 7 days and then exposed to air. The mixture was diluted with THF, filtered over neutral aluminum oxide, and the solvent was removed under reduced pressure. The crude product was purified by dialyzing against MeOH (1 kDa MWCO, RC tubing). The precipitate was filtered off, rinsed with MeOH, and the solvent removed under reduced pressure to afford DMG-T-MeOIPG2900 (248 mg, 0.071 mmol, 52%) as a colorless viscous oil.

NMR and chromatographic analyses gave the following results:
**¹H-NMR** (700 MHz, CDCl₃, 27°C) *δ*=7.65 (s, 1H; H-6), 5.24-5.15 (m, 1H; H-2), 4.69-4.61 (m, 2H; H-7), 4.57 (dt, *J* = 14.1, 3.5 Hz, 1H; H'-4), 4.48-4.39 (m, 1H; H"-4), 4.30 (dd, *J* = 11.9, 3.6 Hz, 1H; H'-1), 4.12 (dd, *J* = 11.9, 6.5 Hz, 1H; H"-1), 3.96-3.90 (m, 1H), 3.89-3.83 (m, 1H), 3.66-3.32 (m, 284H; H-3, H-8 - H-10), 2.32-2.25 (m, 4H; H-13), 1.64-1.53 (m, 4H; H-14), 1.33-1.20 (m, 40H; H-15 - H-24), 0.87 (t, *J* = 7.1 Hz, 6H; H-25) ppm.

**¹³C-NMR** (176 MHz, CDCl₃, 27°C) *δ*=173.5, 173.2 (C=O), 144.5 (C-5), 124.4 (C-6), 79.3, 79.1, 78.9, 78.8, 78.7, 78.5 (C-9), 77.9, 77.8, 73.9, 73.8, 73.1, 72.8, 72.8, 72.5, 72.0 (C-2, C-10), 71.3, 70.7, 70.5, 70.1, 69.9, 69.8, 69.6, 68.8 (C-1, C-4, C-8), 64.8, 62.7, 59.5, 59.3 (C-11), 51.3 (C-7), 34.4, 34.2 (C-13), 32.0 (C-23), 29.8, 29.8, 29.6, 29.5, 29.4, 29.3, 29.2 (C-15 - C-22), 25.1, 25.0 (C-14), 22.8 (C-24), 14.2 (C-25) ppm.

**GPC** (THF): Mₙ= 4.15 kDa; PDI=1.2

### Synthesis of DMG-T-IPG2250

DMG-T-IPG2250 was prepared according to the general procedure explained above in the section entitled "Deprotection of poly(ethoxy ethyl glycidyl ether". The educts were used in the following amounts/equivalents: DMG-T-PEEGE5000: 653 mg, 0.127 mmol; oxalic acid: 711 mg, 7.9 mmol, 2 eq per repeating unit n of EEGE, 0.15 g/mL.

The solvent was removed under reduced pressure, affording the CuAAC reaction coupled lipid DMG-T-IPG2250 (368 mg, 0.127 mmol) in quant. yield.

NMR and chromatographic analyses gave the following results:
**¹H-NMR** (700 MHz, [D₄]MeOD, 27°C) *δ*=8.02 (d, *J =* 3.2 Hz, 1H; H-6), 5.26-5.16 (m, 1H; H-2), 4.69-4.61 (m, 3H; 2H of H-7, H'-4), 4.54-4.47 (m, 1H; H"-4), 4.37 (dd, *J* = 12.0, 3.1 Hz, 1H; H'-1), 4.14 (dd, *J =* 12.0, 6.8 Hz, 1H; H"-1), 3.76-3.51 (m, 155H; IPG, H-3), 2.31 (dt, *J =* 13.4, 7.3 Hz, 4H; (C=O)CH₂), 1.66-1.54 (m, 4H; (C=O)CH₂C*H*₂), 1.37-1.25 (m, 40H; H-11-H-19), 0.90 (t, *J* = 7.0 Hz, 6H; CH₃) ppm.

**¹³C-NMR** (176 MHz, [D₄]MeOD, 27°C) *δ*=175.0, 174.7 (C=O), 145.5 (C-5), 126.5 (C-6), 81.6, 81.6, 81.5, 81.4, 80.8, 80.8, 80.8 (IPG), 78.2, 73.3, 72.8, 72.6, 72.5, 71.6, 71.4, 70.8, 70.7, 70.6, 69.8, 69.6, 69.4 (C-2, IPG), 66.6, 65.2, 64.3, 63.9, 63.6, 63.4, 62.7, 62.6, 62.3, 62.0 (C-1, C-4, IPG), 52.2, 52.2 (C-7), 49.5, 35.1, 35.0 (C-9), 33.1 (C-19), 30.8, 30.8, 30.7, 30.5, 30.5, 30.2, 30.2 (C-11 - C-18), 26.1, 26.0 (C-10), 23.7 (C-20), 14.5 (C-21) ppm.

### GPC (H₂O): M_{w}=2.5 kDa; PDI=2.2

### Synthesis of DMG-T-IPG3750

DMG-T-IPG3750 was prepared according to the general procedure explained above in the section entitled "Deprotection of poly(ethoxy ethyl glycidyl ether". The educts were 784 mg, 8.71 mmol, 2 eq per repeating unit n of EEGE, 0.15 g/mL. The solvent was removed under reduced pressure, affording the triazole-linked lipid DMG-T-IPG3750 (366 mg, 72.6 µmol) in quant. yield.

NMR and chromatographic analyses gave the following results:
**¹H-NMR** (700 MHz, [D₄]MeOD, 27°C) *δ*=8.10-8.00 (m, 1H; H-6), 5.22 (s, 1H; H-2), 4.68-4.60 (m, 3H; 2H of H-7, H'-4), 4.55-4.48 (m, 1H; H"-4), 4.37 (dd, J = 12.0, 3.0 Hz, 1H; H'-1), 4.14 (dd, *J =* 12.0, 6.7 Hz, 1H; H"-1), 3.76-3.49 (m, 303H; IPG, H-3), 2.31 (dt, *J =* 13.4, 7.4 Hz, 4H, (C=O)CH₂), 1.64-1.56 (m, 4H; (C=O)CH₂C*H*₂), 1.37-1.25 (m, 40H; (C=O)CH₂CH₂(C*H*₂)₁₀CH₃), 0.90 (t, *J =* 7.0 Hz, 6H; CH₃) ppm.

**¹³C-NMR** (176 MHz, [D₄]MeOD, 27°C) *δ*=175.0, 174.7 (C=O), 145.5(C-5), 126.5 (C-6), 81.6, 81.6, 81.5, 81.4, 80.8, 80.8, 80.8, 80.8 (IPG), 78.2, 73.3, 72.8, 72.6, 72.5, 72.5, 72.2, 71.5, 71.4, 70.8, 70.7, 70.6, 69.6, 69.4 (C-2, IPG), 65.2, 64.3, 63.9, 63.6, 63.4, 62.7, 62.6, 62.6, 62.3, 62.0 (C-1, C-4, IPG), 52.2, 52.2 (C-7), 35.1, 35.0 (C-9), 33.1 (C-19), 30.8, 30.8, 30.7, 30.5, 30.5, 30.2, 30.2 (C-11 - C-18), 26.1, 26.0 (C-10=, 23.7 (C-20), 14.5 (C-21) ppm.

**GPC** (H₂O): M_{w}=6.0 kDa; PDI=1.7

### Synthesis of DMG-T-PEEGE-allyl

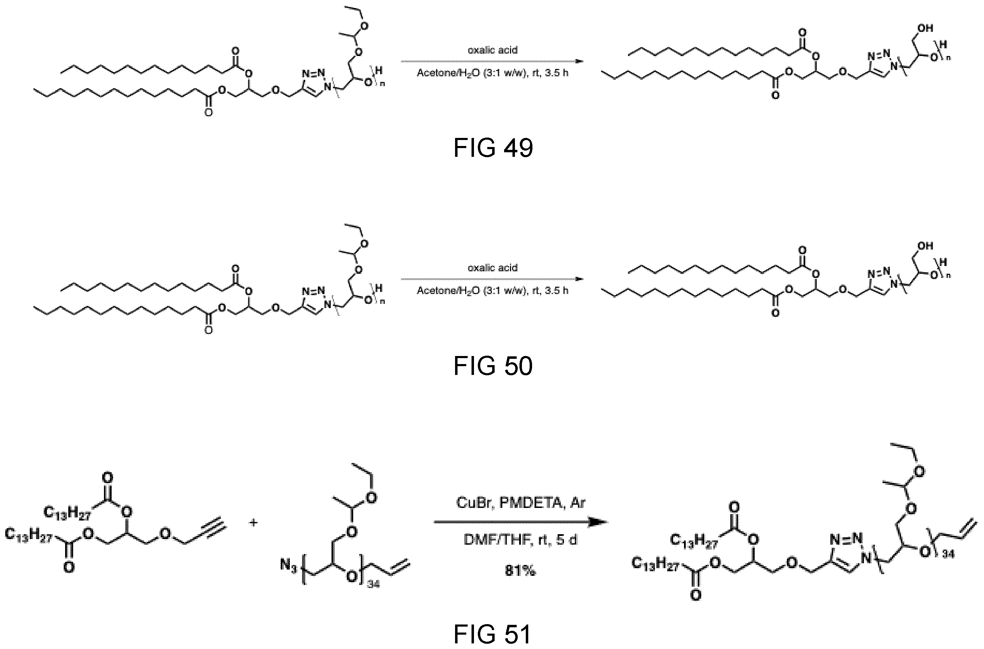

In a 50 mL Schlenk flask N₃-PEEGE-allyl (211 mg, 41.8 µmol, 1.0 eq), DMG-alkyne (28 mg, 50 µmol, 1.2 eq) were dissolved in 4 mL THF and 20 mL DMF, and PMDETA (22 µL, 0.11 mmol, 2.5 eq) was added. The solution was purged with argon for 15 min and then CuBr (12 mg, 84 µmol, 2.0 eq) was added. The reaction was stirred for 5 days at room temperature under argon atmosphere (cf. Figure 51).

The reaction was exposed to air and diluted with THF. The mixture was then filtered over neutral alumina (height: 12cm, diam.: 2 cm) and subsequently the solvent was removed with a rotary evaporator. The filtered crude product was dialyzed against MeOH/DCM (1:1, 1 kDa MWCO, RC tubing). First exchange after 8 h then dialysis overnight. The solvent was removed in vacuo, giving the product as a slightly yellowish viscous liquid (189 mg, 33.7 µmol) in 81% yield.

NMR analysis gave the following results:
**¹H-NMR** (700 MHz, (CD₃)₂CO, 27°C) *δ*=7.99 - 7.95 (m, 1H; H-6), 5.92 (ddt, *J* = 16.2, 10.6, 5.2 Hz, 1H, H-30), 5.28 (d, *J* = 17.2, 1H, H'-31), 5.20 (p, *J* = 5.0 Hz, 1H, H-2), 5.12 (dd, *J =* 10.5, 1.9 Hz, 1H, H"-31), 4.73 (d, *J =* 5.4 Hz, 35 H, H-11), 4.69-4.62 (m, 3H; H-7, H'-4), 4.50 (dq, *J =* 14.2, 6.9 Hz, 1H; H"-4), 4.36 - 4.31 (m, 1H; H'-1), 4.17-4.10 (m, 3H; H"-1, H-29), 3.95 (s, 1H), 3.78 (s, 1H), 3.75-3.42 (m, 257H; H-3, H-8 - H-10, H-12), 2.31 (dt, J = 10.4, 7.4 Hz, 4H; H-16), 1.65-1.55 (m, 4H; H-17), 1.30 (s, 40H; H-18 - H-27), 1.26 (t, *J* = 2.7 Hz, 110H; H-13), 1.17 (t, *J =* 7.0 Hz, 110H; H-14), 0.88 (t, *J* = 6.8 Hz, 6H; H-28) ppm.

### Synthesis of DMG-C-PEEGE4500

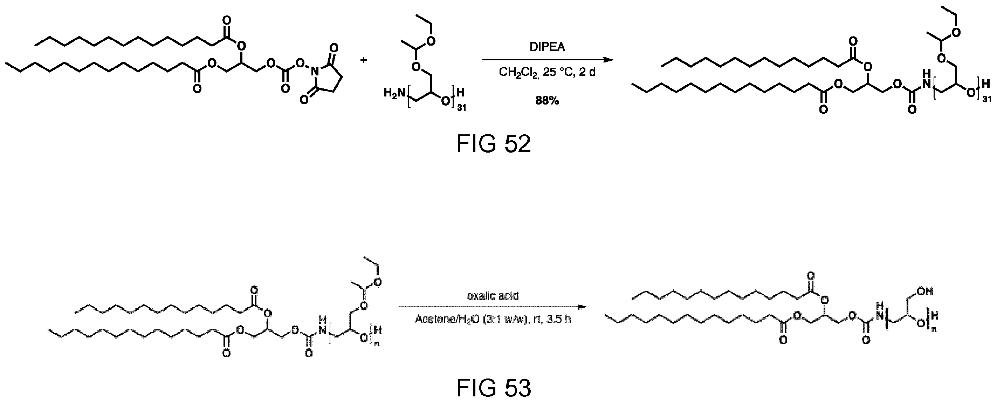

DMG-succinimidyl carbonate (41.6 mg, 0.064 mmol), α-amino-PEEGE (241 mg, 0.053 mmol), and DIPEA (0.11 mL, 0.64 mmol) were dissolved in 8 mL CH₂Cl₂ and stirred at 25°C for 2 days (cf. Figure 52). The solvent was removed under reduced pressure and the crude product was dialyzed against acetone for one day. The solvent was removed under reduced pressure giving DMG-C-PEEGE4500 (237 mg, 0.047 mmol, 88%) as a colorless viscous oil.

NMR analysis gave the following results:
**¹H-NMR** (700 MHz, (CD₃)₂CO, 27°C) *δ*=6.30 (s, 1H; NH), 5.23 (s, 1H; H-2), 4.73 (d, *J* = 4.9 Hz, 31H; H-8), 4.37 - 4.31 (m, 1H; H'-1), 4.31 - 4.23 (m, 1H; H'-3), 4.21 - 4.13 (m, 2H; H"-1, H"-3), 3.80 (s, 2H; H-4), 3.76 - 3.42 (m, 217H; H-5, H-6, H-7, H-10), 2.32 (td, J = 7.4, 2.5 Hz, 4H; (C=O)CH₂), 1.66 - 1.56 (m, 4H; (C=O)CH₂C*H*₂), 1.30 (s, 40H; (C=O)CH₂CH₂(C*H*₂)₁₀CH₃), 1.26 (d, *J* = 4.5 Hz, 93H; H-9), 1.17 (t, *J* = 6.9 Hz, 93H; H-11), 0.89 (t, *J* = 6.9 Hz, 6H; CH₃).

**¹³C-NMR** (176 MHz, (CD₃)₂CO, 27°C) *δ*=173.4 (C=O), 173.1 (C=O), 156.8 (O(C=O)NH), 100.6, 100.5 (C-8), 80.0, 79.9, 79.8, 79.2 (PEEGE backbone), 73.2, 71.0 (C-2), 70.9, 70.8, 70.7, 70.5, 70.4 (PEEGE backbone), 67.5, 66.1, 66.0, 65.6, 63.2, 62.9, 61.3, 61.2, 61.2 (PEEGE backbone), 43.0, 34.7, 34.5 (C-13), 32.7 (C-23), 30.4, 30.3, 30.3 (C-15, C-22), 25.7 (C-14), 23.3 (C-24), 20.4, 20.3 (C-9), 15.8 (C-11), 14.4 (C-25) ppm.

### Synthesis of DMG-C-IPG2250

DMG-C-IPG2250 was prepared according to the general procedure explained above in the section entitled "Deprotection of poly(ethoxy ethyl glycidyl ether". The educts were used in the following amounts/equivalents: DMG-T-PEEGE4500: 199 mg, 39.1 µmol; oxalic acid: 218 mg, 2.43 mmol, 2 eq per repeating unit n of EEGE, 0.15 g/mL. The solvent was removed under reduced pressure, affording the carbamate-linked lipid DMG-C-IPG2250 (14.0 mg, 0.005 mmol) in 12% yield as a colorless viscous liquid.

NMR analysis gave the following results:
**¹H-NMR** (500 MHz, [D₄]MeOD, 25°C) *δ*=5.25 (t, *J* = 29.2 Hz, 1H; H-2), 4.42 - 4.34 (m, 1H; H'-1), 4.27 (dd, *J* = 10.4, 4.1 Hz, 1H; H'-3), 4.16 (dd, *J* = 11.9, 6.2 Hz, 2H; H"-1, H"-3), 3.81 - 3.43 (m, 58H; H-5 - H-7), 2.33 (td, *J* = 7.4, 2.6 Hz, 4H; H-13), 1.61 (s, 4H; H-14), 1.30 (s, 45H, H-15 - H-24), 0.90 (t, *J* = 6.9 Hz, 6H; H-25) ppm.

### Second synthesis approach

According to a second chemical synthesis approach, an anionic ring opening polymerization using 2,3-bis(benzyloxy)propan-1-ol as initiator *via* reaction with the respective oxirane monomer was pursued. This synthesis will be explained in the following in more detail. The four synthesized polymeric lipids were then formulated into LNPs using microfluidic techniques.

### General Procedure for Homopolymer Synthesis by Anionic Ring-Opening Polymerization

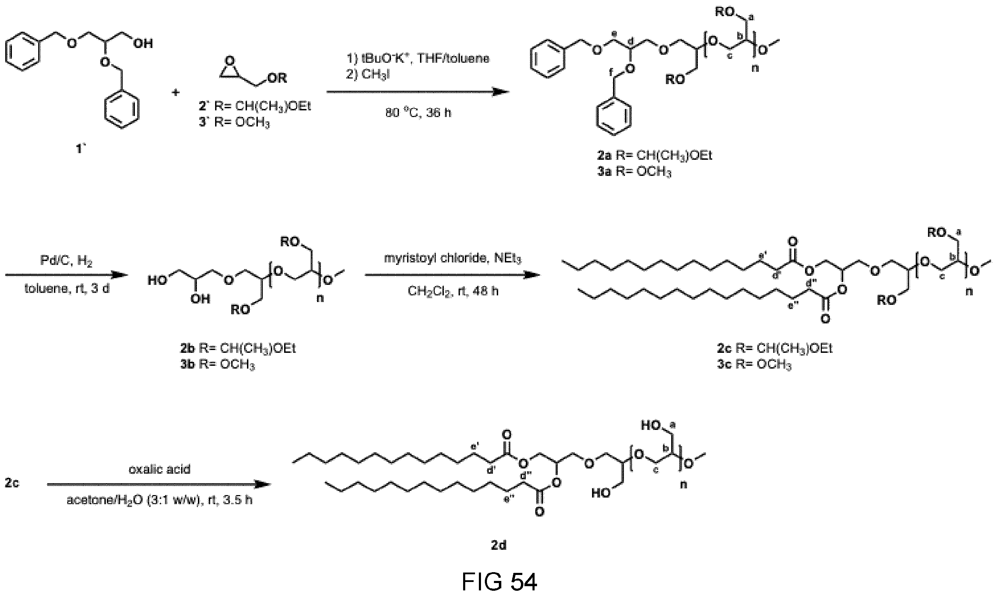

The linear polyglycerols derivatives PEEGE, which is the precursor for IPG, and MeOIPG were prepared by living anionic polymerization with rac-2,3-bis(benzyloxy)propan-1-ol 1' as initiator via reaction with the respective oxirane monomer (cf. Figure 54). Initiator 1' (0.25 g, 0.90 mmol) was dissolved in a solution of KOtBu in THF (2 equiv KOtBu in 5 mL THF) under argon atmosphere and heated to 50 °C for 2 h to deprotonate the alcohol group. The generated t-BuOH and solvent were removed in high vacuum. The remaining alcoholate initiator was completely dried, re-dissolved in dry toluene, and heated to 80 °C under argon atmosphere. Then, freshly distilled monomer (either rac-ethoxyethyl glycidyl ether (EEGE, 2'**,** 3.0 ml, 25.7 mmol) for PEEGE, or rac-glycidyl methyl ether **(3'**, 1.6 mL, 17.8 mmol) for MeOIPG) was added to the alcoholate toluene solution and polymerized for 24 h at 80 °C under argon atmosphere (cf. Figure 54). The reaction mixture was cooled down to RT, followed by the addition of methyl iodide (1 mL) and further stirring at RT for 2 h. After this time, the mixture was concentrated under reduced pressure, and subsequently dried in high vacuum. For purification, the obtained oil was dissolved in Et₂O and centrifuged to separate the insoluble salts. Further purification of the polymer was done by flash chromatography followed by dialysis in acetone (1 kDa MWCO, RC tubing) for three days. The dibenzyl-functionalized PEEGE **2a** and MeOIPG 3a were obtained as oils with 80% yield.

NMR analysis gave the following results:
**Compound 2a** ¹H-NMR: (500 MHz, CDCl₃, TMS): δ (ppm) = 1.19 (t, J = 7.04 Hz 128H, OCHC*H*₃,), 1.28 (d, J = 5.24 Hz, 128H, CHC*H*₃,), 3.74-3.38 (m, 307H, *Hₐ, H_{b}, H_{c},* OC*H*₂CH₃), 4.52 (s, 2H,CH_{f}), 4.72-4.5 (m, 42H, CHₑ, OC*H*C*H*₃ ) 7.34-7.26 (m, 10H, Bn). Mₙ(NMR): 6676 g/mol, n= 42
**Compound 3a** ¹H-NMR: (500 MHz, CDCl₃, TMS): δ (ppm) = 3.33 (s, 82H, OC*H₃*,), 3.75-3.36 (m, 140H, CHC*H₃*,), 4.52 (s, 2H,CH_{f}), 4.66 (s, 2H, CHₑ,) 7.33-7.26 (m, 10H, Bn). Mₙ(NMR): 2920 g/mol, n=27

### General Procedure for Copolymer Synthesis by Anionic Ring-Opening Polymerization

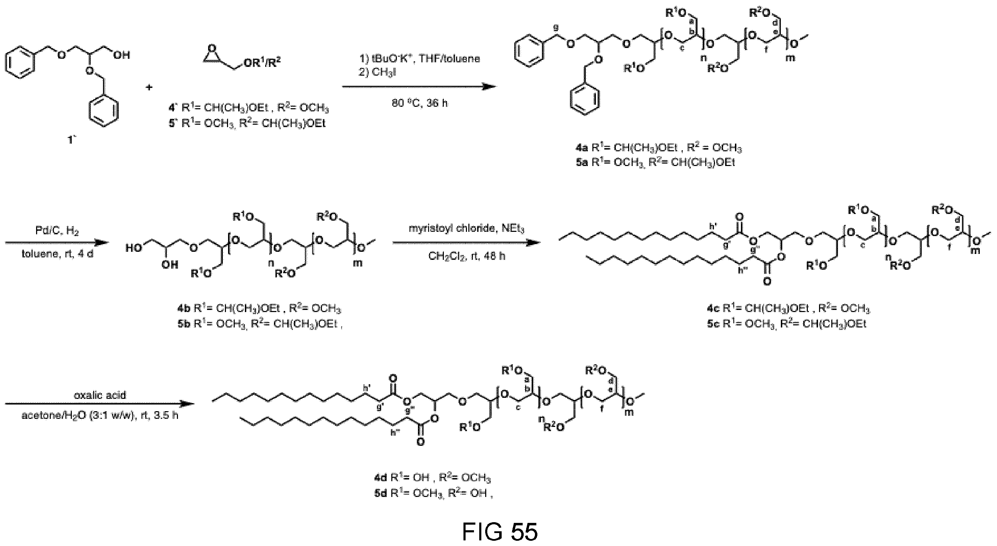

The copolymerized polyglycerols (consisting of IPG and MeOIPG) were prepared by living anionic polymerization with rac-2,3-bis(benzyloxy)propan-1-ol **1'** as initiator *via* reaction with the respective oxirane monomer (cf. Figure 55). Initiator **1'** (0.25 g, 0.9 mmol) was dissolved in 1M KOtBu in THF (8.2 mL, 8.2 mmol) under argon atmosphere and heated to 50 °C for 2 h. The generated t-BuOH and the solvent were removed in high vacuum. The remaining alcoholate initiator was completely dried, re-dissolved in dry toluene, and heated to 80 °C under argon atmosphere. For copolymer **4a** [Bn₂O-PEEGE*co*MeOIPG-OCH₃], freshly distilled monomer rac-ethoxyethyl glycidyl ether **2'** (2.0 mL, 13.9 mmol) was added to the mixture and stirred for 12 h, followed by the addition of rac-glycidyl methyl ether **3'** (1.0 mL, 11.1 mmol) for the second polymer unit of MeOIPG and further stirring for 24 h at 80 °C under argon atmosphere. Similarly, for copolymer **5a** [Bn₂O-MeOIPG*co*PEEGE-OCH₃], *rac*-glycidyl methyl ether **3'** (1.0 mL, 11.1 mmol) was added to the initiator alcoholate and stirred for 12 h, followed by rac-ethoxyethyl glycidyl ether **2'** (2.0 mL, 13.9 mmol) for the second polymer unit of PEEGE to polymerize for a further 24 h at 80 °C under argon atmosphere.

The reaction was quenched by the addition of methyl iodide (1 mL), similarly to the synthesis of homopolymers. For purification, the obtained oil was dissolved in Et₂O and centrifuged to separate the insoluble salts. Further purification of the polymer was done by flash chromatography and dialysis in acetone (1 kDa MWCO, RC tubing) for three days. The dibenzyl-functionalized copolymers IPG (**4a** and **5a**) were obtained as slightly yellow oils.

NMR analysis gave the following results:
**Compound 4a:** 1H-NMR (500 MHz, CDCl₃, TMS): δ (ppm) = 1.19 (t, J = 7.04 Hz, 54H, OR₁(OCHCH₃))), 1.28 (m, 54H, CHC*H*₃), 3.33 (s, 40H, OR₂(OCH₃)), 3.70-3.37 (m, 220H, *Hₐ, H_{b}, H_{c}, H_{d}, Hₑ, H_{f},* OR₁(OC*H*₂CH₃)), 4.52 (s, 2H, CH_{f}), 4.70-4.52 (m, 20H, CH_{g}, OC*H*C*H₃*), 7.34-7.26 (m, 10H, Bn). Mₙ(NMR): 4316, n_{(OEE)} = 18, m_{(OCH3)} = 13.
**Compound 5a:** 1H-NMR (500 MHz,(CD₃)₂CO, TMS): δ (ppm) = 1.19 (t, J = 7.04 Hz, 68H, OCHC*H₃*), 1.28 (m, 68H, CHC*H*₃), 3.33(s, 58H, OR₁(OCH₃)), 3.70-3.37 (m, 271H, *Hₐ, H_{b}, H_{c}, H_{d}, Hₑ, H_{f},* OR₂(OC*H*₂CH₃)), 4.50 (s, 2H,CH_{f}), 4.66-4.50 (m, 20H, CH_{g}, OCHC*H₃*), 7.34-7.26 (m, 10H, Bn). Mₙ(NMR): 5429, n_{(OCH3)} = 19, m_{(OEE)} = 22.

### General Procedure of Hydrogenative Debenzylation

The IPG dibenzyl ethers (either homopolymer **2a/3a,** or copolymer **4a/5a**) were deprotected *via* hydrogenation in Toluene with Pd/C (10% w/w) as catalyst (cf. Figures 54 and 55). Dibenzyl ether polyglycerol was dissolved in toluene and flushed with argon three times. Then hydrogen gas was flushed into the solution and the reaction mixture was left in hydrogen atmosphere at room temperature for 3-4 days. The mixture was filtered through Celite^{®} to remove the catalyst, and the filtrate was concentrated under reduced pressure. The diol-functionalized polymers (Polymer-OCH₂CH(O*H*)CH₂O*H*) were obtained as colorless, viscous oils in quantitative yield after drying in high vacuum (cf. Figures 54 and 55).

NMR analysis gave the following results:
**Compound 2b:** ¹H-NMR: (500 MHz, CDCl₃, TMS) 1.19 (t, 3H, OCHC*H₃*,), 1.28 (d, 3H, CHC*H*₃,), 3.74-3.38 (m, 7H, *Hₐ, H_{b}, H_{c},* OC*H*₂CH₃), 4.72-4.5 (m, 1H, CHₑ, OC*H*C*H₃*)
**Compound 3b:** ¹H-NMR: (500 MHz, CDCl₃, TMS): δ (ppm): 3.32 (s, 3H, OCH₃,) 3.67-3.34(m, 5H, *Hₐ, H_{b}, H_{c}*,)
**Compound 4b** ¹H-NMR: (500 MHz, (CD₃)₂CO, TMS): δ (ppm) 1.19 (m, 3H, OR₁(OCHCH₃)), 1.23 (m, 3H, OR₁(CHC*H₃*), 3.33(s, 3H, OR₂(OCH₃)), 3.70-3.37 (m, 12H,*Hₐ*, *H_{b}, H_{c}, H_{d}, Hₑ, H_{f}* OR₁(OC*H*₂CH₃)), 4.70-4.64 (m, 1H, OC*H*C*H₃*).
**Compound 5b** ¹H-NMR: (500 MHz, CDCl₃, TMS): δ (ppm) 1.19 (m, 3H, OR₂(OCHCH₃)), 1.29 (m, 3H, OR₂(CHC*H*₃), 3.33(s, 3H, OR₂(OCH₃)), 3.74-3.39 (m, 12H,*Hₐ*, *H_{b}, H_{c}, H_{d}, Hₑ, H_{f}* OR₁(OC*H*₂CH₃)), 4.70-4.64 (m, 1H, OC*H*C*H₃*).

### Synthesis of polyglycerol amphiphiles

To install the lipid ester moieties, the diol-functionalized IPG (either homopolymer **2b/3b,** or copolymer **4b/5b**) was dissolved in DCM under argon atmosphere and triethylamine (4 equiv) was added at room temperature. The reaction mixture was stirred for 30 min and then cooled down to 0 °C, upon which myristoyl chloride (2.4 equiv) was added dropwise (cf. Figures 54 and 55). The mixture was stirred for two days at room temperature and then purified by flash column chromatography followed by dialysis in regenerated cellulose membrane with 1 kDa MWCO in acetone to yield the diester products. The methoxylated IPG amphiphile 3c was obtained as colorless oil (80% yield).

NMR analysis gave the following results:
**Compound 2c** ¹H-NMR: (500 MHz, CDCl₃, TMS): δ (ppm) = 0.88-0.86 (t, 6H, C*H₃*), 1.19 (t, m, 127H, OCHC*H₃*), 1.28 (m, 168H, CHC*H₃*, alkyl chain), 1.63-1.55 (m, CHₑ, CH_{e"}), 2.32-2.25 (m, CH_{d}, CH_{d"}), 3.69-3.42 (m, 307H,*Hₐ*, *H_{b}, H_{c},* OC*H*₂CH₃), 4.72-4.5 (m, 42H, OC*H*C*H₃*).
**Compound 3c** ¹H-NMR: (500 MHz, CDCl₃, TMS): δ (ppm) = 0.88-0.86 (t, 6H, C*H₃*), 1.31-1.27 (m, 40H, alkyl chain), 1.72-1.54 (m, CHₑ, CH_{e"}), 2.35-2.25 (m, CH_{d}, CH_{d"}), 3.32 (s, 81H, OCH₃) 3.67-3.34(m, 140H, *Hₐ, H_{b}, H_{c},*)*.* Mₙ(NMR)= 2860 g/mol
**Compound 4c** ¹H-NMR: (500 MHz, (CD₃)₂CO, TMS):δ (ppm) = 0.88-0.86 (t, 6H, C*H₃*), 1.17 (m, 56H, OR₁(OCHC*H*₃)), 1.28 (m, 110H, OR₁(CHC*H₃)* alkyl chain), 1.66-1.56 (m, CH_{g}, CH_{g"}), 2.39-2.24 (m, CHₕ, CH_{h"}), 3.32 (s, 38H, OR₂(OCH₃), 3.67-3.40 (m, 203H, *Hₐ, H_{b}, H_{c}, H_{d}, Hₑ, H_{f}* OR₁(OC*H*₂CH₃)), 4.73-4.69 (m, 18H, OR₁(OC*H*C*H₃)*).
**Compound 5c** ¹H-NMR: (500 MHz, CDCl₃, TMS): δ (ppm) = 0.88-0.86 (t, 6H, C*H₃*), 1.17 (m, 58H, OR₂(OCHC*H₃*)), 1.28 (m, 98H, OR₂(CHC*H₃)* alkyl chain), 1.63-1.52 (m, CH_{g}, CH_{g"}), 2.34-2.23 (m, CHₕ, CH_{h"}), 3.34 (s, 57H, OR₁(OCH₃)), 3.67-3.40 (m, 278H, *Hₐ, H_{b}, H_{c}, H_{d}, Hₑ, H_{f}* OR₂(OC*H₂*CH₃)), 4.72-4.67 (m, 19H, OR₁(OC*H*C*H₃)*).

To obtain the hydroxylated side chain amphiphiles, the EEGE-protected side chains were deprotected (cf. Figures 54 and 55). To a 0.05 mg/mL solution of the PEEGE-based compound (either homopolymer amphiphile **2c,** or copolymer amphiphile **4c/5c**) in acetone, an aqueous solution of oxalic acid (2 equiv. per repeating unit of EEGE, 3:1 acetone:water, 0.15 g oxalic acid/mL water) was added dropwise under fast stirring at room temperature. After 3.5 hours, the reaction was diluted with deionized H₂O and dialyzed against deionized H₂O (1 kDa MWCO, RC tubing) until a pH of 6 to 7 was reached. The solvent was removed under reduced pressure, affording the respective product.

NMR analysis gave the following results:
**Compound 2d** ¹H-NMR: (500 MHz, MeOD, TMS): δ (ppm) = 0.88-0.86 (t, 6H, C*H₃*), 1.28 (m, 40H, alkyl chain), 1.63-1.55 (m, CHₑ, CH_{e"}), 2.32-2.25 (m, CH_{d}, CH_{d"}), 3.69-3.42 (m, 218H, *Hₐ*, *H_{b}, H_{c},* OC*H₂*CH₃). Mₙ(NMR)=3550 g/mol
**Compound 4d** ¹H-NMR: (500 MHz, CD₃OD, TMS): δ (ppm) = 0.95-0.93 (t, 6H,C*H₃*), 1,34-1.26 (m, 40H, alkyl chain), 1.68-1.64 (m, CH_{g}, CH_{g"}), 2.41-2.37 (m, CHₕ, CH_{h"}), 3.39 (s, 38H, OR₂(OCH₃)), 3.78-3.47 (m, 167H, *Hₐ, H_{b}, H_{c}, H_{d}, Hₑ, H_{f},*). Mₙ(NMR)= 3060 g/mol
**Compound 5d** ¹H-NMR: (500 MHz, CD₃OD, TMS): δ (ppm) = 0.89-0.87 (t, 6H,C*H₃*), 1,35-1.28 (m, 40H, alkyl chain), 1.66-1.52 (m, CH_{g}, CH_{g"}), 2.36-2.33 (m, CHₕ, CH_{h"}), 3.34 (s, 55H, OR₁(OCH₃), 3.78-3.47 (m, 178H, *Hₐ, H_{b}, H_{c}, H_{d}, Hₑ, H_{f}*).

### Microfluidic Preparation of LNPs

Aqueous solution for LNP formulation consisted of 1 mg/mL mRNA in sodium citrate (1 mM, pH 6.4), diluted prior to each use with citrate buffer (10 mM, pH 4) to obtain 0.02 mg mRNA/mL. Cell viability, transfection and cryo-EM experiments made use of LNPs prepared with eGFP mRNA, whereas Poly(A) mRNA was used for all remaining measurements.

Ethanol solution for LNP formulation contained four lipids dissolved in ethanol; the ionizable lipid LP-01, cholesterol, DSPC and one polymer lipid (2d, 3c, 4d, or 5d, respectively) were mixed at a 50:39:9:2 molar ratio. A control formulation using DMG-PEG2000 was also produced.

LNPs were prepared by mixing aqueous and ethanol phase using a NanoAssemblr^{™} Ignite^{™} Cartridge with NxGen^{™} mixing technology on a NanoAssemblr^{™} Ignite^{™} system (Precision Nanosystems, Vancouver, BC, Canada). Process flow conditions were 12 mL/min total flowrate at 3:1 aqueous: ethanol solution, and N:P ratio of 6. Buffer-exchange was completed by filling formulations in Amicon^{®} Ultra-4 ultrafiltration centrifuge tubes (MWCO 30 kDa) (Millipore, Burlington, MA, USA). Vials were centrifuged at 5 000 G for 15 minutes, after which subnatant was removed and volume topped up to 4 mL with PBS. Centrifugation was repeated three times, and formulations were stored at 4 °C until use.

### Characterization of LNPs

### DLS for size & stability

Size of LNPs was measured using dynamic light scattering (Zetasizer Ultra, Malvern Panalytical Ltd, Malvern, UK) in 20x PBS, reported as the z-average mean particle diameter. Measurements were repeated at seven day-intervals for stability over time, with samples stored at 4 °C.

### Zeta-Potential

Zeta-potential of 1x LNPs in PBS was measured on a Zetasizer Ultra device using folded capillary zeta cell cuvettes (Malvern Panalytical Ltd.).

### Encapsulation Efficiency

A Quant-iT^{™} Ribogreen^{™} RNA Assay was used to determine mRNA encapsulation efficiency. A protocol as described elsewhere ^{[59]} was followed. Briefly, LNPs were diluted 67-fold in TE-buffer. Subsequently, lipid membranes were lysed by adding 2% v/v Triton X-100 to release encapsulated mRNA. Control samples containing unencapsulated mRNA were diluted with equivalent volumes of TE buffer. Samples were incubated for 10 minutes at 37 °C, before diluting 1:1 with Ribogreen solution. Fluorescence was then quantified by a plate reader (Spark^{®}, TECAN, Männedorf, ZH, Switzerland) (excitation 485 nm; emission 528 nm), and encapsulation efficiency (EE) calculated by equation 1 indicated above.

### Cryo-TEM Imaging

Chloroform-cleaned 300 mesh R1.2/1.3 holey carbon grids (Quantifoil, MicroTools GmbH, Jena, Germany) were hydrophilized by glow discharging in a EMSCOPE SC500 (10 mA, 60 seconds) before applying 4 µL aliquots of eGFP mRNA-LNP solution to the grids.

Samples were vitrified by automatic blotting (blot force -11, blot time 3.5 s, wait time 3 s) and subsequent plunge freezing with a FEI Vitrobot Mark IV (Thermo Fischer Scientifc Inc., Waltham, MA, USA) using liquid ethane as cryogen.

Grids were clipped with copper clip rings and C-clips to produce Autogrids.

Vitrified samples were transferred to the autoloader of an FEI TALOS ARCTICA electron microscope (Thermo Fischer Scientific Inc., Waltham, MA, USA), equipped with a high-brightness field-emission gun (XFEG) operated at an acceleration voltage of 200 kV.

Micrographs were acquired on a FEI Falcon 3 direct electron detector (Thermo Fischer Scientific Inc., Waltham, MA, USA) at normal magnifications of 3,400, 4,300 and 28,000, corresponding to calibrated pixel sizes of 31.3, 24.6 and 3.7 Å/ pixel, respectively.

### Anti-PEG antibody ELISA

Binding of LNPs to anti-PEG antibodies was measured using a competitive ELISA assay following the manufacturer's instructions. Briefly, LNPs were diluted 1:1 with PEG-BSA standard solution, before transferring 50 µL to the provided 96-well plate. The plate was incubated on a plate shaker (400 rpm) for 45 minutes at room temperature. Afterwards, liquid was aspirated out of each well and the plate was washed using wash buffer three times. Subsequently, 100 µL TMB substrate were added before incubating the plate on a plate shaker (400 rpm) in the dark for 15 minutes. Finally, 100 µL stop solution was added and mixed for 1 minute on a plate shaker, before measuring optical density at 450 nm on a Spark^{®} plate reader.

### Cell Culture

HepG2 cells were cultured at 37 °C and 5% CO₂ in high glucose Dulbecco's Modified Eagle Medium with GlutaMAX^{™} supplement containing 10% v/v fetal bovine serum and 1% v/v penicillin-streptomycin. Once cells reached 70% confluency, cells were ready for seeding in experiments.

### Cell Viability

HepG2 cells were cultured at 37 °C and 5% CO₂ in high glucose Dulbecco's Modified Eagle Medium with GlutaMAX^{™} supplement containing 10% v/v fetal bovine serum and 1% v/v penicillin-streptomycin. Once cells reached 70% confluency, 5 000 cells/well were seeded into a 96-well plate and incubated at 37 °C and 5% CO₂ for 24 hours. Following, cell culture media was aspirated out of each well, before adding 100 µL of LNP formulations diluted in fresh media. After returning plates to the incubator for 24 hours, 10% v/v CCK-8 solution was added to each well and incubated for 3 hours, before quantifying absorbance on a plate reader (excitation 450 nm).

### Cell Transfection - Plate Reader

To start, 10 000 cells/well were seeded into a black-bottom 96-well plate and incubated at 37 °C and 5% CO₂. A serial dilution was used to obtain mRNA-LNP solutions containing 100, 50, 25, 12.5 and 6.25 ng mRNA/100 µL. After 24 hours, existing cell medium was aspirated and replaced with 100 µL prepared mRNA-LNP solutions in each well, before incubating for 24 hours. After this time, 1% v/v Triton-X100 was added to each well and briefly placed on a microplate shaker at 300 rpm. Subsequently, eGFP fluorescence was then quantified on a Spark^{®} plate reader (excitation 488 nm, emission 517 nm).

### Cell Transfection - Flow Cytometer

To start, 10 000 cells/well were seeded into a 96-well plate and incubated at 37 °C and 5% CO₂. After 24 hours, mRNA-LNP samples were diluted with cell culture media to achieve 100 ng, 50 ng, 10 ng and 5 ng mRNA per 100 µL. Resulting solutions were added to each well and incubated for 24 hours. After this time, Trypsin was briefly added to wells to obtain cells in suspension, before measuring eGFP fluorescence using Flow Cytometry (Attune NxT Flow Cytometer, Invitrogen, ThermoFischer). Quantification of fluorescence intensity and gene transfection efficiency were determined using FlowJo^{™} v10.10 software (FlowJo LLC, Ashland, OR, USA).

### Statistical Analysis

Statistical analysis of results was performed using Prism 10.2.3 (GraphPad, San Diego, CA, USA). One-way ANOVA was applied to Figure 4. Tukey's multiple comparison test was applied to determine significant differences between groups. Significance levels were designated as *p<0.05.

### Result and Discussion

### Biophysical Characterization

A main constituent of various therapeutic formulations is PEG, providing a stealth effect and limiting immunogenicity upon administration. Methoxy-PEG (mPEG) is a frequently used PEG variation featuring a terminal methoxy group. However, this methoxy group is easily detected by anti-PEG antibodies, which bind to cause an antigenic response in PEG-sensitive patients. The recent drastic increase in anti-PEG antibodies highlights the importance of finding an alternative for PEG in therapeutic applications. In the experiments described in here, amphiphilic polymers were investigated as a PEG-alternative for mRNA-loaded LNP formulations, using different structures of hydrophilic PG grafted to hydrophobic alkyl chains. The incorporation of these polymers into LNPs was examined and compared to DMG-PEG2000, the current gold standard for commercial LNP formulations (e.g. SpikeVax^{®}).

All PG variations were designed to replicate either molecular weight or chain length of PEG2000, and all featured at least one terminal methoxy group. The resulting IPG with a comparable chain length and no additional methoxy groups may be deemed most structurally similar to PEG. Alternately, MeOIPG, IPG-block-MeOIPG and MeOIPG-block-IPG were modified to include methoxy groups along the polymer backbone. MeOIPG was employed to assess LNPs utilizing a simpler polymer synthesis. IPG-block-MeOIPG and MeOIPG-block-IPG were able to provide insights into additional polarity and potential steric hindrance along the polymer backbone. All PG polymers were covalently linked to two 14-C alkyl chains, to replicate DMG-lipid tails. Resulting amphiphilic polymers were then incorporated into LNP formulations. Finally, delivery efficacy of LNPs was quantified by encapsulating eGFP mRNA, to produce eGFP after intracellular release and mRNA translation.

### Synthesis of Polyglycerol lipids

PEEGE, which is the precursor for IPG and MeOIPG, was prepared by living anionic polymerization with 2,3-bis(benzyloxy)propan-1-ol 1' as initiator *via* reaction with the respective oxirane monomer (cf. Figure 54). The monomer skeleton was then selectively deprotected *via* hydrogenation in Toluene under dry condition. The diol functionalized IPGs **2b** and **3b** were ester coupled with myristoyl chloride followed by the final product of the hydroxyl side chain amphiphiles **2d** IPG-(C-14) the acetal-protected side chains of **2c** were deprotected (cf. Figure 54). Similarly, the copolymerized polyglycerols of IPG and MeOIPG, were prepared by living anionic polymerization with 2,3-bis(benzyloxy)propan-1-ol 1' as initiator (cf. Figure 55). The copolymers were prepared by step-wise addition of the different oxirane monomer. All synthesized polymers were well characterized by spectroscopic techniques.

**Table 2: Overview of molecular weight, chain lengths and functional groups of PEG and PG polymers used**

| **Polymer Name** | **Structure #** | **Polymer linked to DMG** | **MW (kDa)** | **# of Repeating Units** | **Functional Groups** |
|---|---|---|---|---|---|
| PEG | - | PEG2000 | 2509.2 | 44 | - |
| IPG | **2d** | IPG-OH | 3119 | 42 | - |
| MeOIPG | **3c** | IPG-OCH₃ | 2553 | 27 | OCH₃ |
| *IPG-block-*MeOIPG | **4d** | IPG-OH-OCH₃ | 3060 | n_{(OH)}= 18; | R₁= OH; |
| | | | | m_{(OCH3)}=13 | R₂ = OCH₃ |
| MeOIPG-*block-*IPG | **5d** | IPG-OCH₃-OH | 3390 | n_{(OCH3)}= 19; | R₁ = OCH₃; |
| | | | | m _{(OH)}=22 | R₂ = OH |

Formulation occurred by mixing aqueous and organic phases via microfluidics to produce LNPs. Negatively charged mRNA in the aqueous phase formed a stabilizing complex with ionizable cationic LP-01 lipids in the organic phase. Remaining lipids in the organic phase self-assembled around these complexes to form mRNA LNP systems. Finally, ultrafiltration centrifugation was used for buffer replacement to storage buffer. Size and stability of these LNPs was then measured using DLS as described. To efficiently pass through blood vessels and facilitate cellular uptake, desirable nanoparticle diameters are in the 100 nm-range. Furthermore, a low PDI is desired as an indicator for a homogeneous population with narrow size distribution. Size of nanoparticles can be decreased by the presence of a stealth lipid, resulting in steric hindrance during production and reducing particle agglomeration.

Microfluidic process flow conditions were optimized to render PEG-LNPs with a mean particle size of 110.0 ± 6.3 nm (PDI 0.045 ± 0.030). With a chain length comparable to PEG2000 (42 repeating units, ru), IPG resulted in the smallest particles (223.1 ± 24.4 nm, PDI 0.105 ± 0.072) of all PG-variations. Similarly, MeOIPG-block-IPG rendered small LNPs (41 ru, 233.7 ± 28.2 nm, PDI 0.113 ± 0.027), whereas MeOIPG (27 ru, 252.3 ± 14.0 nm, PDI 0.169 ± 0.063) and IPG-block-MeOIPG (31 ru, 264.3 ± 66.5 nm, PDI 0.154 ± 0.138) had shorter chain lengths leading to significantly larger particles. Comparing IPG-block-MeOIPG and MeOIPG-block-IPG indicates that LNP size is unrelated to additional functional groups on the PG backbone. However, the comparatively larger size of PG- versus PEG-LNP systems indicates that adjustments of production parameters are required to reduce PG-LNP diameters. By varying microfluidic settings (e.g., microfluidic chip conformation, flow rate/ ratio) or types of aqueous buffer, previous works have shown that a reduction in nanoparticle size is possible ^{[60],[61]}. Increasing molar amounts of stealth lipid may lead to decreased LNP size but comes at the expense of decreased bioavailability and cellular uptake ^{[62] [63]}.

**Table 3: Overview of particle size, polydispersity index, zeta potential and encapsulation efficiency of mRNA once variations of PG were used to formulate LNPs**

| **Polymer to functionalize LNPs** | **Diameter (nm)** | **PDI** | **Zeta Potential (ζ)** | **Encapsulation Efficiency (%)** |
|---|---|---|---|---|
| PEG | 110.0 ± 6.3 | 0.045 ± 0.030 | -3.30 ± 0.55 | 98.3 ± 7.5 |
| IPG | 223.1 ± 24.4 | 0.105 ± 0.072 | -6.05 ± 2.82 | 93.4 ± 8.5 |
| MeOIPG | 252.3 ± 14.0 | 0.169 ± 0.063 | -6.78 ± 2.37 | 61.9 ± 33.2 |
| *IPG-block-*MeOIPG | 264.3 ± 66.5 | 0.154 ± 0.138 | -4.11 ± 2.33 | 79.4 ± 17.0 |
| MeOIPG-*block-IPG* | 233.7 ± 28.2 | 0.113 ± 0.027 | -4.10 ± 2.13 | 78.1 ±23.3 |

Charges in LNP solutions was assessed, as strongly cationic or anionic charges (~ ±30 mV) lead to increased colloidal stability through particle repulsion, but may also increase cellular toxicity ^{[64]}. Zeta potential of all PG-LNP systems ranged between -4.10 (MeOIPG-block-IPG) and -6.78 mV (MeOIPG). Similarly, PEGylated LNPs had a zeta potential of -3.30 mV. As all formulations display a neutral zeta potential, cellular toxicity as a result of excess charges is unlikely to occur ^{[65]}.

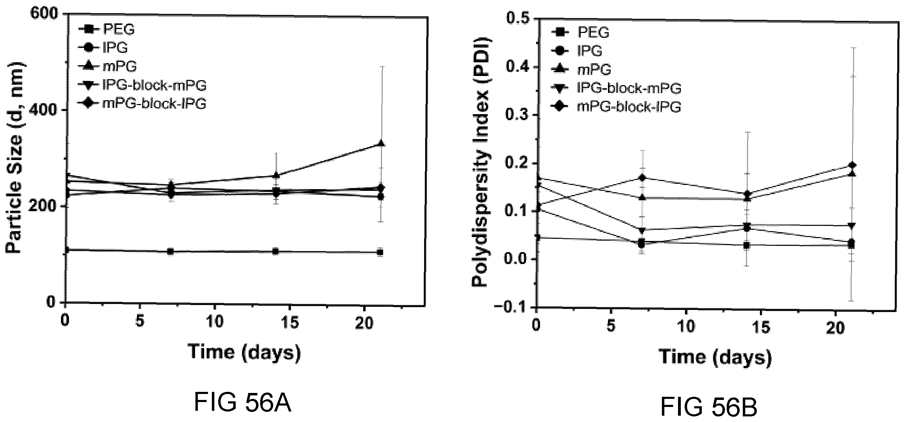

Measuring colloidal storage stability of mRNA LNPs at 4°C demonstrated that PG-variations had negligible changes in size over three weeks, comparable to PEGylated LNPs (cf. Figures 56A and 56B). The sole exception was MeOIPG-LNPs, with a rapid size increase from 252.3 to 336.6 ± 161.8 nm over three weeks. This may indicate that due to its short chain length, MeOIPG has a reduced a stealth effect, leading to particle agglomeration during production and subsequent storage.

Encapsulation efficiency of mRNA was assessed by comparing unencapsulated (free) mRNA with encapsulated mRNA released after lipid membrane lysis with TritonX-100. IPG-LNPs had the highest encapsulation efficiency (93.4%), followed by IPG-block-MeOIPG (79.4%), MeOIPG-block-IPG (78.1%), and finally MeOIPG-LNPs (61.9%). In comparison, PEGylated LNPs had encapsulation efficiencies of 98.3%. Following, IPG may form compact (albeit slightly larger) LNPs with comparable encapsulation efficiencies to PEG-LNPs. While the introduction of some methoxy groups in IPG-block-MeOIPG and MeOIPG-block-IPG led to slightly decreased encapsulation efficiencies alike, MeOIPG has an entirely methoxylated polymer backbone. This feature may contribute to the formation of large, loosely packed MeOIPG-LNPs with mRNA breaching the particle surface, leading to reduced encapsulation efficiencies ^{[64]}.

### Cryo-TEM & Morphology

Cryo-TEM images of eGFP mRNA LNP systems were prepared immediately after microfluidic production (0 days) or after 4 weeks of storage in PBS at 4°C (4 weeks) (data not shown). Subsequent images revealed spherical, unilamellar vesicles in the nanometer range, consistent with prior DLS measurements. LNP morphology was unaltered when replacing PEG with PG-variations. At 0 days, all vesicles contained a lipid monolayer shell with an amorphous electron-dense core. This amorphous "solid core" morphology may indicate an interior oil-phase containing deprotonated ionizable lipids and mRNA ^{[16] [61]}. Less definitive lamellar structures were expected due to the relatively low N/P ratio of 6 ^{[16].} Image acquisition of PG-functionalized LNPs was complicated due to the majority of LNPs being located on hydrophobic carbon film grids rather than more hydrophilic mesh holes. This may indicate a more polar nature of PG, which is maintained when incorporated into LNP shells. In comparison, PEG-LNPs were found on carbon film grids and mesh holes alike, potentially indicating lower polarity.

After four weeks, a slight degradation (thickening of lipid monolayer, expelling of lipids) was apparent in all LNP systems. However, the presence of largely intact PG-LNP systems after several weeks after preparation supports previous stability data and indicates sufficient stability for future therapeutic applications.

### Anti-PEG antibody activation

A major issue of PEGylated therapeutics is the activation of anti-PEG antibodies upon administration, leading to accelerated blood clearance of such compounds and hypersensitivity reactions ^{[33] [66]}. Thus, testing anti-inflammatory activity *in vitro* to observe potential antigenicity and cross-reactivity of PG-LNPs with anti-PEG antibodies was essential.

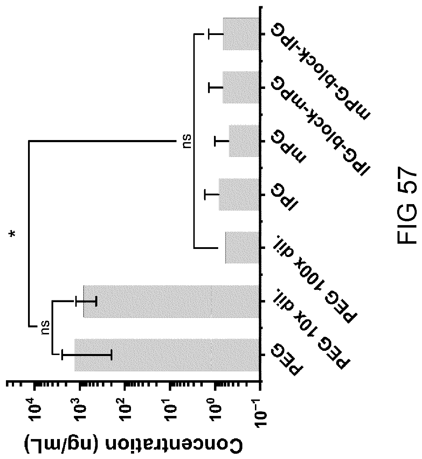

Figure 57 shows the concentration of PEG (ng/mL) based on the anti-PEG antibody affinity as determined via competitive ELISA for a variety of PEG- and PG-functionalized LNPs. The statistical significance was evaluated using one-way ANOVA (* denotes p <0.05; no star denotes lack of significance). The data is representative of three independent experiments, as mean +/- SD.

Rabbit monoclonal anti-PEG antibodies (as used in the competitive ELISA assay) selectively target the terminal methoxy groups of mPEG [abcam protocol] ^{[32]}. Although all PG-variations contained a terminal methoxy group, binding affinity of PG to anti-PEG antibodies was negligible, having binding concentrations of 0.83 (IPG), 0.50 (MeOIPG), 0.68 (IPG*-block-*MeOIPG), and 0.66 ng/mL (MeOIPG-block-IPG), respectively. In comparison, binding affinity was significantly elevated for undiluted PEG-LNPs (1310.55 ng/mL) and remained elevated in 10-fold dilutions (816.81 ng/mL). In fact, 100-fold dilution of PEG-LNPs was required for binding of anti-PEG antibodies to be comparable (0.58 ng/mL, p < 0.05) with that of PG-LNPs.

In comparison, the C-C-O motif in the PEG backbone promotes specificity via hydrophobic interactions with different IgG antibodies, with 3 - 6 antibodies per monomer ^{[67]}.

### Cell Viability

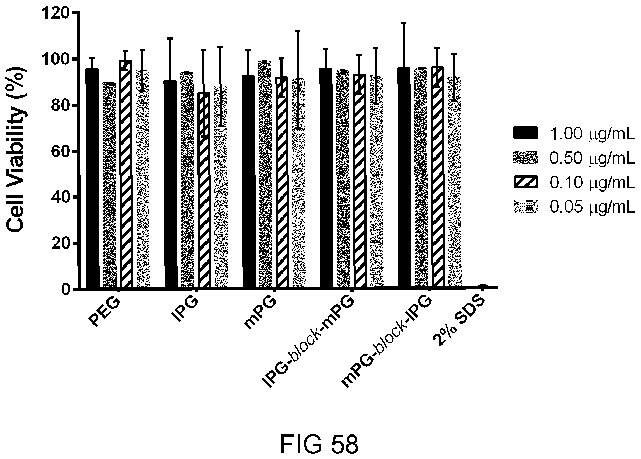

To validate PG-LNPs for potential therapeutic applications, cell viability of HepG2 cells co-incubated with mRNA PG-LNP systems was determined *in vitro* using a CCK8-assay (Figure 58). In this context, Figure 58 illustrates the cell viability as determined via CCK8-assay using HepG2 cells exposed to 1, 0.5, 0.1 and 0.05 µg mRNA/mL encapsulated in LNPs formulated with a variety of amphiphilic polymers derived from polyglycerol. The data shows mean +/- SD of five independent experiments.

Based on zeta-potential of each LNP system, as well as PG being a biocompatible polymer, cell viability should be retained. In the performed experiments, all formulations showed sufficient cell viability over 80% when co-incubated with concentrations up to 1 µg mRNA/mL (equivalent to 100 ng mRNA/well). Specifically, IPG-block-MeOIPG LNPs had highest cell viability at 1 µg/mL (95.4%), comparable to PEG-LNPs (95.6%). The lowest cell viability at 1 µg/mL was 90.4% (IPG). The lowest cell viability was measured for 0.5 µg mRNA/mL in MeOIPG (83.4%). Overall, samples encapsulating 1 µg mRNA/mL had higher cell viability (95.4 (IPG-block-MeOIPG), 95.4 (MeOIPG-block-IPG), 92.5 (MeOIPG) and 90.4% (IPG)) than samples encapsulating 0.05 µg/mL (92.1 (IPG-block-MeOIPG), 91.4 (MeOIPG-block-IPG), 90.6 (MeOIPG) and 87.7% (IPG)). However, none of these results were statistically significant, indicating good biocompatibility of PG-LNPs. Additionally, modification of the polymer backbone, increased polarity or variations in molecular weight seemed inconsequential for cell viability.

### Transfection

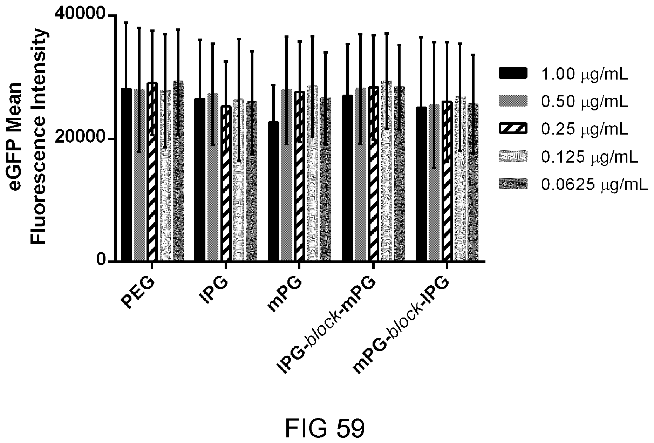

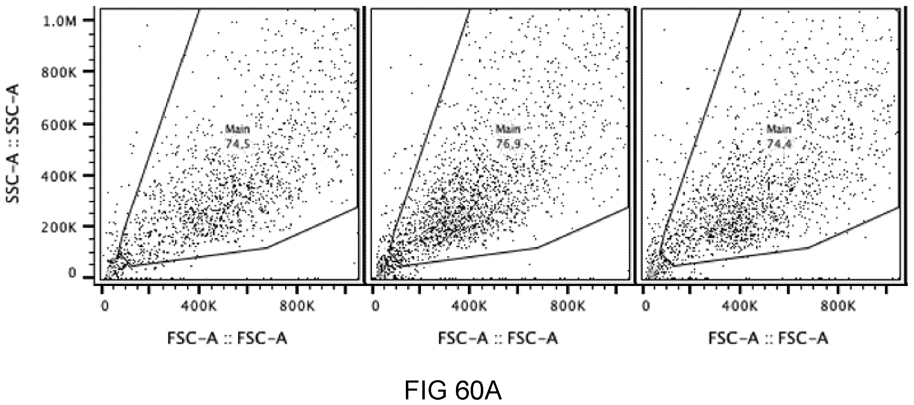

As cellular uptake and transfection efficacy may be dependent on PEG chain length or may decrease with excessive PEGylation ^{[63]}, the ability of PG-LNPs to effectively deliver and release mRNA intracellularly was investigated. In this study, HepG2 cells were transfected with PG-LNPs encapsulating eGFP-encoding mRNA to model mRNA delivery. Subsequently, eGFP expression was measured using fluorescence microscopy and fluorometry (Figure 59), as well as flow cytometry (Figures 60A to 60D).

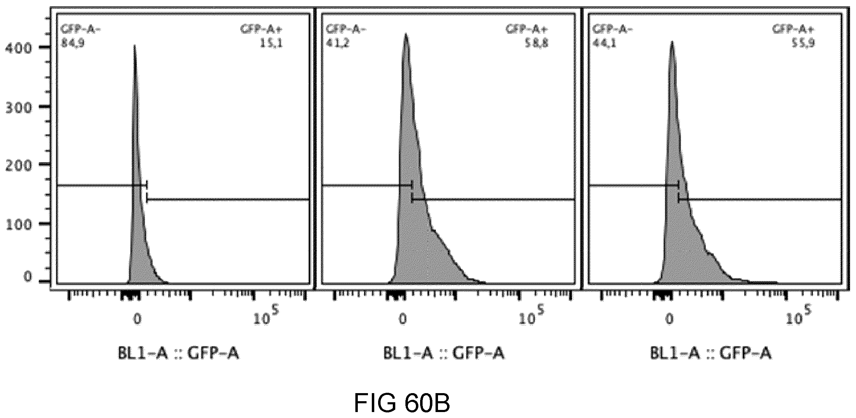

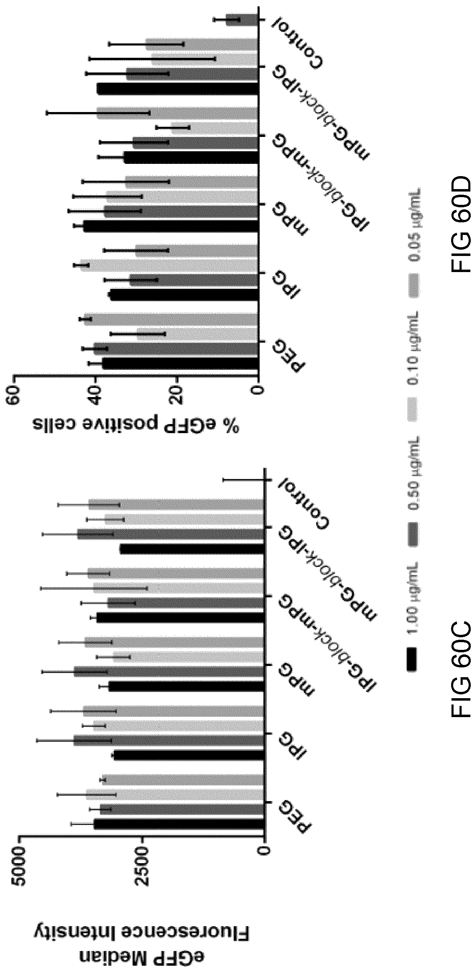

In this context, Figure 59 shows the eGFP fluorescence intensity per well quantified on a fluorometer with respect to HepG2 cells transfected with eGFP mRNA encapsulated in PEG- and IPG-block-MeOIPG-functionalized LNPs after cell lysis using 10% TritonX. The data shows mean +/- SD of three independent experiments. Figure 60A shows forward and side scatter of HepG2 cells transfected with (i) none or eGFP mRNA encapsulated in (ii) PEG- and (iii) PG-LNP systems. Figure 60B shows histograms of eGFP fluorescence in each cell population. Figure 60C illustrates the eGFP fluorescence intensity in individual HepG2 cells after transfection with LNP systems. Figure 60D illustrates the percentage of eGFP positive cells per cell population. The data displays median +/- SD (Figure 60C) and mean +/- SD (Figure 60D) of four independent experiments.

Fluorescence microscopy showed successful cellular uptake and transcription of mRNA to produce eGFP throughout cells (data not shown). After cell lysis using TritonX, eGFP within cellular membranes could be quantified by a fluorometer, measuring fluorescence per well (5000 cells at seeding) (Figure 59). All PG variations demonstrated comparable maximum transfection ability to PEG LNP systems (27017.22 at 0.0625 µg mRNA/mL), having mean fluorescence between 22693.22 (MeOIPG at 1.00 µg/mL) and 29320.89 (IPG-block-MeOIPG at 0.125 µg/mL). Mean fluorescence intensity was comparable for all PG-LNP systems at various mRNA concentrations tested, without apparent trends. This can be explained by the tendency of HepG2 cells to occasionally form clusters rather than even layers across a surface, leading to cellular overlap and blocking of fluorescent proteins by other cells.

Following, flow cytometry enabled the quantification of fluorescence per cell, as well as fraction of eGFP-positive cells able to transcribe mRNA. FlowJo^{®} software was used to select desired cell populations and determine intrinsic fluorescence of cells in positive controls, before applying appropriate gating to remaining samples (Figures 60A and 60B). Resulting median fluorescence per cell (Figure 60C) was comparable for all PG-LNP systems, regardless of polymer length or functionalization. Maximum median fluorescence was 3890.0 for IPG-LNPs (0.5 µg mRNA/mL), while the minimum was 3068.0, also for IPG-LNPs (1.00 µg/mL). In comparison, PEGylated LNPs varied between 3305.3 (0.05 µg/mL) and 3637.7 (0.10 µg/mL). As flow cytometry measured eGFP fluorescence per cell, results indicate effective intracellular delivery and release of mRNA is possible for all PG-variations at concentrations as low as 0.05 µg mRNA/mL.

While all LNP systems successfully delivered mRNA, delivery efficacy varied between formulations and concentrations. IPG-LNPs had the highest fraction of eGFP-expressing cells, with 43.1% (0.10 µg/mL), followed by MeOIPG (42.8%, 1.00 µg/mL), IPG-block-MeOIPG and MeOIPG-*block*-IPG-LNPs (both 39.4%, 0.05 and 1.00 µg/mL), respectively. Lowest expression of eGFP was by IPG-*block*-MeOIPG- (21.1%, 0.05 µg/mL), MeOIPG-*block*-IPG- (27.5%, 0.10 µg/mL), IPG- (30.0%, 0.05 µg/mL) and finally MeOIPG-LNPs (32.5%, 0.05 µg/mL). This indicates that PG-LNPs can effectively deliver mRNA intracellularly for transcription, comparable with the commercial standard of PEGylated LNPs (29.7% (0.10 µg/mL) and 42.5% (0.05 µg/mL)), regardless of polymer functionalization or polarity. A general trend indicates that transfected cells decreased with decreasing mRNA concentration. As mRNA concentration varied between formulations, a comparable volume of PEG- and IPG-LNPs could be added to achieve similar eGFP expression in cells. In contrast, a much larger volume of MeOIPG-LNPs would be needed to achieve similar effects. Such factors need to be considered when designing therapies with alternate polymers.

### Conclusion

Variations of the biocompatible, hydrophilic polymer PG (such as IPG, MeOIPG, *IPG-block-*MeOIPG and MeOIPG-block-IPG) were successfully established that can successfully form an amphiphilic polymer when covalently linked to alkyl chains These compounds represent highly appropriate alternatives to the commercial standard PEG in LNP formulations for mRNA delivery, with comparable transfection efficacy. The most promising candidate as a PEG alternative in LNP formulations was IPG, possessing a similar chain length as PEG2000. While able to provide similar size, stability and transfection efficacy, IPG had negligible binding of anti-PEG antibodies. In comparison, MeOIPG with a significantly shorter chain length and methoxylated polymer backbone resulted in similar antibody binding, but due to low mRNA loading required much larger volumes for similar transfection efficacy.

### List of references cited in the preceding sections or deemed otherwise to be relevant

[1] X. Han, M. J. Mitchell, G. Nie, Matter 2020, 3, 1948-1975.
[2] Y. Weng, C. Li, T. Yang, B. Hu, M. Zhang, S. Guo, H. Xiao, X.-J. Liang, Y. Huang, Biotechnology Advances 2020, 40, 107534.
[3] L. Schoenmaker, D. Witzigmann, J. A. Kulkarni, R. Verbeke, G. Kersten, W. Jiskoot, D. J. A. Crommelin, International Journal of Pharmaceutics 2021, 601, 120586.
[4] N. Chaudhary, D. Weissman, K. A. Whitehead, Nature Reviews Drug Discovery 2021, 20, 817-838.
[5] C. Hald Albertsen, J. A. Kulkarni, D. Witzigmann, M. Lind, K. Petersson, J. B. Simonsen, Advanced Drug Delivery Reviews 2022, 188, 114416.
[6] L. Xu, X. Wang, Y. Liu, G. Yang, R. J. Falconer, C.-X. Zhao, Advanced NanoBiomed Research 2022, 2, 2100109.
[7] Y. Sato, H. Hatakeyama, Y. Sakurai, M. Hyodo, H. Akita, H. Harashima, Journal of Controlled Release 2012, 163, 267-276.
[8] D. C. Litzinger, Journal of Liposome Research 1997, 7, 51-61.
[9] A. M. Reichmuth, M. A. Oberli, A. Jaklenec, R. Langer, D. Blankschtein, Therapeutic Delivery 2016, 7, 319-334.
[10] P. J. C. Lin, Y. Y. C. Tam, I. Hafez, A. Sandhu, S. Chen, M. A. Ciufolini, I. R. Nabi, P. R. Cullis, Nanomedicine: Nanotechnology, Biology and Medicine 2013, 9, 233-246.
[11] S. C. Semple, A. Akinc, J. Chen, A. P. Sandhu, B. L. Mui, C. K. Cho, D. W. Y. Sah, D. Stebbing, E. J. Crosley, E. Yaworski, I. M. Hafez, J. R. Dorkin, J. Qin, K. Lam, K. G. Rajeev, K. F. Wong, L. B. Jeffs, L. Nechev, M. L. Eisenhardt, M. Jayaraman, M. Kazem, M. A. Maier, M. Srinivasulu, M. J. Weinstein, Q. Chen, R. Alvarez, S. A. Barros, S. De, S. K. Klimuk, T. Borland, V. Kosovrasti, W. L. Cantley, Y. K. Tam, M. Manoharan, M. A. Ciufolini, M. A. Tracy, A. de Fougerolles, I. MacLachlan, P. R. Cullis, T. D. Madden, M. J. Hope, Nature Biotechnology 2010, 28, 172-176.
[12] P. Paramasivam, C. Franke, M. Stöter, A. Höijer, S. Bartesaghi, A. Sabirsh, L. Lindfors, M. Y. Arteta, A. Dahlén, A. Bak, S. Andersson, Y. Kalaidzidis, M. Bickle, M. Zerial, Journal of Cell Biology 2021, 221.
[13] D. Pei, M. Buyanova, Bioconjugate Chemistry 2019, 30, 273-283.
[14] M. Qiu, Y. Tang, J. Chen, R. Muriph, Z. Ye, C. Huang, J. Evans, E. P. Henske, Q. Xu, Proceedings of the National Academy of Sciences 2022, 119, e2116271119.
[15] M. J. Mitchell, M. M. Billingsley, R. M. Haley, M. E. Wechsler, N. A. Peppas, R. Langer, Nature Reviews Drug Discovery 2021, 20, 101-124.
[16] J. A. Kulkarni, D. Witzigmann, J. Leung, Y. Y. C. Tam, P. R. Cullis, Nanoscale 2019, 11, 21733-21739.
[17] D. Shi, D. Beasock, A. Fessler, J. Szebeni, J. Y. Ljubimova, K. A. Afonin, M. A. Dobrovolskaia, Advanced Drug Delivery Reviews 2022, 180, 114079.
[18] J. Brady, T. Dürig, P. I. Lee, J. X. Li, in Developing Solid Oral Dosage Forms (Second Edition) (Eds.: Y. Qiu, Y. Chen, G. G. Z. Zhang, L. Yu, R. V. Mantri), Academic Press, Boston, 2017, pp. 181-223.
[19] R. Tenchov, J. M. Sasso, Q. A. Zhou, Bioconjugate Chemistry 2023***.***
[20] N. Maurer, D. B. Fenske, P. R. Cullis, Expert Opinion on Biological Therapy 2001, 1, 923-947.
[21] S.-D. Li, L. Huang, Molecular Pharmaceutics 2008, 5, 496-504.
[22] S. Chen, Y. Y. C. Tam, P. J. C. Lin, M. M. H. Sung, Y. K. Tam, P. R. Cullis, Journal of Controlled Release 2016, 235, 236-244.
[23] K. J. Hassett, J. Higgins, A. Woods, B. Levy, Y. Xia, C. J. Hsiao, E. Acosta, Ö. Almarsson, M. J. Moore, L. A. Brito, Journal of Controlled Release 2021, 335, 237-246.
[24] S. Chen, Y. Y. C. Tam, P. J. C. Lin, A. K. K. Leung, Y. K. Tam, P. R. Cullis, Journal of Controlled Release 2014, 196, 106-112.
[25] J. A. Kulkarni, D. Witzigmann, S. B. Thomson, S. Chen, B. R. Leavitt, P. R. Cullis, R. van der Meel, Nature Nanotechnology 2021, 16, 630-643.
[26] M. J. W. Evers, J. A. Kulkarni, R. van der Meel, P. R. Cullis, P. Vader, R. M. Schiffelers, Small Methods 2018, 2, 1700375.
[27] T. Ishida, M. Ichihara, X. Wang, K. Yamamoto, J. Kimura, E. Majima, H. Kiwada, Journal of Controlled Release 2006, 112, 15-25.
[28] T.-C. Chang, B.-M. Chen, W.-W. Lin, P.-H. Yu, Y.-W. Chiu, Y.-T. Chen, J.-Y. Wu, T.-L. Cheng, D.-Y. Hwang, S. Roffler, Pharmaceutics 2020, 12, 15.
[29] Q. Yang, S. K. Lai, WIREs Nanomedicine and Nanobiotechnology 2015, 7, 655-677.
[30] H. Wang, S. Lin, X. Wu, K. Jiang, H. Lu, C. Zhan, Advanced Science 2023, n/a, 2301777.
[31] Y. Mima, Y. Hashimoto, T. Shimizu, H. Kiwada, T. Ishida, Molecular Pharmaceutics 2015, 12, 2429-2435.
[32] G. T. Kozma, T. Shimizu, T. Ishida, J. Szebeni, Advanced Drug Delivery Reviews 2020, 154-155, 163-175.
[33] B.-M. Chen, T.-L. Cheng, S. R. Roffler, ACS Nano 2021, 15, 14022-14048.
[34] P. Zhang, F. Sun, S. Liu, S. Jiang, Journal of Controlled Release 2016, 244, 184-193.
[35] J. Chen, A. Rizvi, J. P. Patterson, C. J. Hawker, Journal of the American Chemical Society 2022, 144, 19466-19474.
[36] M. C. Castells, E. J. Phillips, New England Journal of Medicine 2020, 384, 643-649.
[37] Y. Ju, W. S. Lee, E. H. Pilkington, H. G. Kelly, S. Li, K. J. Selva, K. M. Wragg, K. Subbarao, T. H. O. Nguyen, L. C. Rowntree, L. F. Allen, K. Bond, D. A. Williamson, N. P. Truong, M. Plebanski, K. Kedzierska, S. Mahanty, A. W. Chung, F. Caruso, A. K. Wheatley, J. A. Juno, S. J. Kent, ACS Nano 2022, 16, 11769-11780.
[38] J. Szebeni, G. Storm, J. Y. Ljubimova, M. Castells, E. J. Phillips, K. Turjeman, Y. Barenholz, D. J. A. Crommelin, M. A. Dobrovolskaia, Nature Nanotechnology 2022, 17, 337-346.
[39] P. Pouyan, M. Cherri, R. Haag, Polymers 2022, 14, 2684.
[40] M. Imran ul-haq, B. F. L. Lai, R. Chapanian, J. N. Kizhakkedathu, Biomaterials 2012, 33, 9135-9147.
[41] A. Thomas, S. S. Müller, H. Frey, Biomacromolecules 2014, 15, 1935-1954.
[42] K. Wagener, M. Worm, S. Pektor, M. Schinnerer, R. Thiermann, M. Miederer, H. Frey, F. Rösch, Biomacromolecules 2018, 19, 2506-2516.
[43] A. S. Abu Lila, K. Nawata, T. Shimizu, T. Ishida, H. Kiwada, International Journal of Pharmaceutics 2013, 456, 235-242.
[44] P. Pouyan, A. Zemella, J. L. Schloßhauer, R. M. Walter, R. Haag, S. Kubick, Scientific Reports 2023, 13, 6394.
[45] M. Tully, N. Hauptstein, K. Licha, L. Meinel, T. Lühmann, R. Haag, Journal of Pharmaceutical Sciences 2022, 111, 1642-1651.
[46] R. Rashmi, H. Hasheminejad, S. Herziger, A. Mirzaalipour, A. K. Singh, R. R. Netz, C. Böttcher, H. Makki, S. K. Sharma, R. Haag, Macromolecular Rapid Communications 2022, 43, 2100914.
[47] F. Reise, J. E. Warias, K. Chatterjee, N. R. Krekiehn, O. Magnussen, B. M. Murphy, T. K. Lindhorst, Chem. - Eur. J. 2018, 24, 17497-17505.
[48] M. Gervais, A. Labbé, S. Carlotti, A. Deffieux, Macromolecules 2009, 42, 2395-2400.
[49] H. Gao, K. Matyjaszewski, Journal of the American Chemical Society 2007, 129, 6633-6639.
[50] A. K. Ghosh, M. Brindisi, Journal of Medicinal Chemistry 2015, 58, 2895-2940.
[51] A. Beloqui, J. Calvo, S. Serna, S. Yan, I. B. H. Wilson, M. Martin-Lomas, N. C. Reichardt, Angewandte Chemie International Edition 2013, 52, 7477-7481.
[52] A. Cioce, M. Thépaut, F. Fieschi, N.-C. Reichardt, Chem. - Eur. J. 2020, 26, 12809-12817.
[53] W. Wang, S. Feng, Z. Ye, H. Gao, J. Lin, D. Ouyang, Acta Pharmaceutica Sinica B 2022, 12, 2950-2962.
[54] C. Rao and C.-F. Liu, Organic & Biomolecular Chemistry 2017, 15, 2491-2496.
[55] M. Tully, M. Dimde, C. Weise, P. Pouyan, K. Licha, M. Schirner, R. Haag, Biomacromolecules 2021, 22, 1406-1416.
[56] A. W. Richter, E. Åkerblom, Int. Arch. Allergy Appl. Immunol. 2009, 74, 36-39.
[57] B.-M. Chen, Y.-C. Su, C.-J. Chang, P.-A. Burnouf, K.-H. Chuang, C.-H. Chen, T.-L. Cheng, Y.-T. Chen, J.-Y. Wu, S. R. Roffler, Anal. Chem. 2016, 88, 10661-10666.
[58] D. Braatz, J. H. Peter, M. Dimde, E. Quaas, K. Ludwig, K. Achazi, M. Schirner, M. Ballauff, R. Haag, J. Mater. Chem. B 2023, 11, 3797-3807.
[59] Ribogreen Assay Training Webinar (https://www.youtube.com/watch?v=1uAyAur_20c), 2020**.**
[60] N. M. Belliveau, J. Huft, P. J. Lin, S. Chen, A. K. Leung, T. J. Leaver, A. W. Wild, J. B. Lee, R. J. Taylor, Y. K. Tam, C. L. Hansen, P. R. Cullis, Mol. Ther. - Nucleic Acids 2012, 1, e37.
[61] M. H. Y. Cheng, J. Leung, Y. Zhang, C. Strong, G. Basha, A. Momeni, Y. Chen, E. Jan, A. Abdolahzadeh, X. Wang, J. A. Kulkarni, D. Witzigmann, P. R. Cullis, Adv. Mater. 2023, 35, 2303370.
[62] B. L. Mui, Y. K. Tam, M. Jayaraman, S. M. Ansell, X. Du, Y. Y. C. Tam, P. J. Lin, S. Chen, J. K. Narayanannair, K. G. Rajeev, M. Manoharan, A. Akinc, M. A. Maier, P. Cullis, T. D. Madden, M. J. Hope, Mol. Ther. Nucleic Acids 2013, 2, e139.
[63] D. Pozzi, V. Colapicchioni, G. Caracciolo, S. Piovesana, A. Laura Capriotti, S. Palchetti, S. D. Grossi, A. Riccioli, H. Amenitsch, A. Laganà, Nanoscale 2014, 6, 2782-2792.
[64] O. F. Khan, Nat. Rev. Methods Primer 2023, 3, 1-2.
[65] K. Syama, Z. J. Jakubek, S. Chen, J. Zaifman, Y. Y. C. Tam, S. Zou, Sci. Rep. 2022, 12, 18071.
[66] M. S. Hershfield, N. J. Ganson, S. J. Kelly, E. L. Scarlett, D. A. Jaggers, J. S. Sundy, Arthritis Res. Ther. 2014, 16, R63.
[67] J. McCallen, J. Prybylski, Q. Yang, S. K. Lai, ACS Biomater. Sci. Eng. 2017, 3, 1605-1615.

## Claims

1. Compound according to general formula (I): 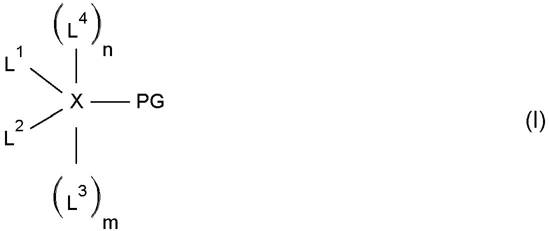 wherein
L¹, L², L³, L⁴ denote independently from each other a lipid moiety,
n is 0 or 1,
m is 0 or 1,
X is a linker moiety, and
PG is a polyglycerol moiety.

2. Compound according to claim 1, **characterized in that** L¹, L², L³, L⁴ are independently from each other a lipid moiety chosen from the group consisting of natural linear fatty acid residues, natural branched fatty acid residues, synthetic linear fatty acid residues, synthetic branched fatty acid residues, sterol lipids, and prenol lipids.

3. Compound according to claim 1 or 2, **characterized in that** L¹ and L² both are a myristic acid residue.

4. Compound according to any of the preceding claims, **characterized in that** the linker moiety corresponds to general formula (IV) or (V): 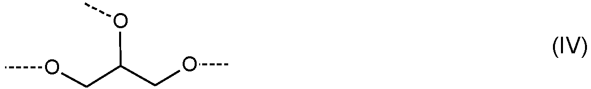 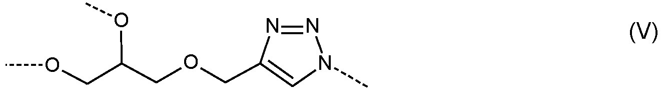 wherein the dashed lines in general formulae (IV) and (V) indicate a covalent bond to the lipid moiety or the polyglycerol moiety bound to the linker moiety.

5. Compound according to any of the preceding claims, **characterized in that** the polyglycerol moiety is a substituted linear polyglycerol, wherein i) first polyglycerol units carrying a first substituent and ii) second polyglycerol units carrying a second substituent or being non-substituted form a) a statistical copolymer in which the first polyglycerol units and the second polyglycerol units are statistically distributed or b) a block copolymer in which the first polyglycerol units and the second polyglycerol units are block-wise arranged.

6. Compound according to any of the preceding claims, **characterized in that** the compound corresponds to any of general formulae (VIa), (VIb), (VIc), or (VId) : 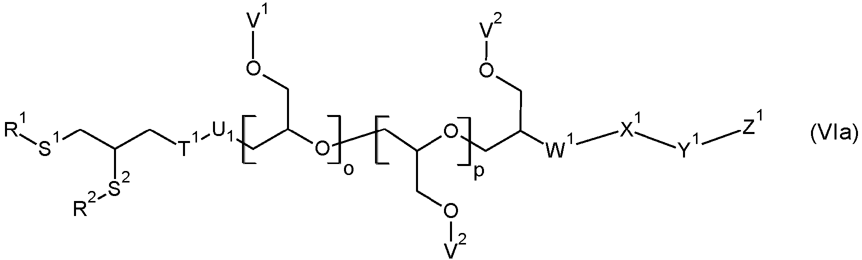 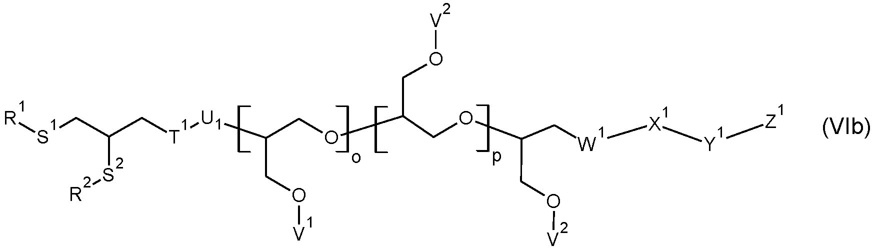 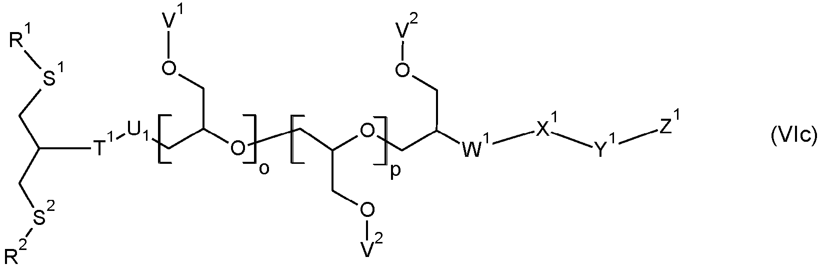 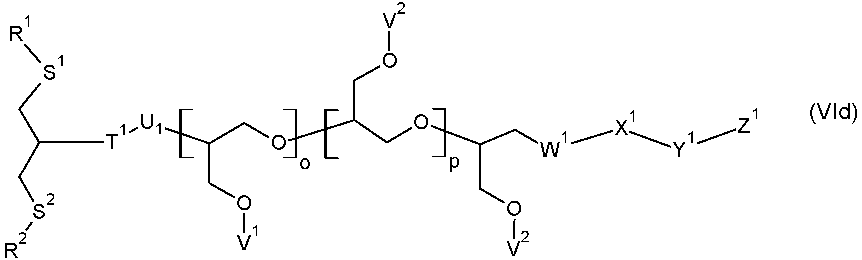 wherein
R¹, R² denote independently from each other a C8-C22 linear or branched alkyl or alkenyl residue, a sterol lipid residue, or a prenol lipid residue, wherein the C8-C22 linear or branched alkyl or alkenyl residue, the sterol lipid residue, and/or the prenol lipid residue optionally carry a functional group chosen from the group consisting of amines, ethers, esters, amides, aryl substituents, and heteroaryl substituents,
S¹, S² denote independently from each other O, S, C(O)O, C(O)NH, C(O)NCH₃, NCH₃,
T¹ denotes OCH₂, OC₂H₄, OC₃H₆, SCH₂, SC₂H₄, SC₃H₆, NHCH₂, OC(O), NHC(O), or is absent,
U¹ denotes NH, O, or a triazole residue,
V¹, V² denote independently from each other H, CH₃, CH(CH₃)OCH₂CH₃, or a statistical mixture of at least two of H, CH₃, and CH(CH₃)OCH₂CH₃, wherein a percentage of an individual of H, CH₃, and CH(CH₃)OCH₂CH₃ in the statistical mixture, if present in the statistical mixture, lies within a range from 0.1 % to 99.9 %,
o, p are independently from each other any number from 0 to 100, with the proviso that a sum of o and p is a number from 10 to 200,
W¹ denotes O, S or NH,
X¹ denotes H, CH₃, or a C1-C4 alkylidene chain, with the proviso that Y' and Z¹ are absent if X¹ denotes H or CH₃,
Y¹ is absent or denotes CHO, COOH, CH=CH₂, N₃, a triazole residue, O, S, NH, or NCH₃, with the proviso that Z¹ is absent if Y' denotes CHO, COOH, CH=CH₂, or N₃, and
Z¹ is absent or denotes CH₂COOH, CH₂CHO, C2-C5alkyl-NH, C2-C5alkyl-NCH₃, CH₂C(O)NH, CH₂C(O)NCH₃, a biologically active targeting moiety, or H.

7. Compound according to claim 6, **characterized in that** X¹ denotes H, CH₃, or a C1-C4 alkylidene chain and **in that** Y' and Z¹ are absent.

8. Compound according to claim 6 or 7, **characterized in that** the compound corresponds to general formula (Vla) or (Vlb).

9. Compound according to claim 6 and 7, **characterized in that** the compound corresponds to general formula (Vla) or (Vlb).

10. Compound according to any of claims 6 to 9, **characterized in that** S¹ and S² denote independently from each other O or C(O)O.

11. Compound according to claims 6 and 7, **characterized in that** S¹ and S² denote independently from each other O or C(O)O.

12. Compound according to claims 6 and 8, **characterized in that** S¹ and S² denote independently from each other O or C(O)O.

13. Compound according to claim 9, **characterized in that** S¹ and S² denote independently from each other O or C(O)O.

14. Compound according to any of the preceding claims for use in administering a pharmaceutically active therapeutic substance, a therapeutic oligonucleotide, or a therapeutic polynucleotide to a human or animal patient.

15. Use of a compound according to any of claims 1 to 13 as carrier for a substance in an in-vitro method for delivering the substance to an intended site of action.
